# EUROPEAN PATENT APPLICATION

(11) **EP 1 586 325 A1**
(43) Date of publication of application: **19.10.2005**
(21) Application number: 03815452.2
(22) Date of filing: 21.08.2003
(51) Int. Cl.: A61K 38/17, A61K 39/395, A61K 45/00, A61K 48/00, A61P 5/38, A61P 29/00, A61P 35/00, A61P 37/02, A61P 37/06, A61P 37/08, A61P 43/00

(54) **DRUGS CONTAINING GALECTIN 9**

(30) Priority: 24.01.2003 JP 2003016076
(71) Applicant: Galpharma Co., Ltd., Takamatsu-shi, Kagawa 761-0301 (JP)
(72) Inventor: HIRASHIMA, Mitsuomi Idai Shukusha B-404, Takamatsu-shi, Kagawa 761-0322 (JP); NISHI, Nozomu, Kita-gun, Kagawa 761-0703 (JP); YAMAUCHI, Akira, Takamatsu-shi, Kagawa 761-0113 (JP); YOSHIDA, Naoko, Takamatsu-shi, Kagawa 760-0080 (JP); SEKI, Masako, Kumamoto-shi, Kumamoto 862-0959 (JP)
(74) Representative: von Kreisler, Alek
(86) International application number: PCT/JP2003/010587
(87) International publication number: WO 2004/064857

(57) **Abstract**

Galectin 9 exerts various functions depending on its localizations. On the other hand, galectin 9 is expected as participating in various biological functions. Thus, it has been required to clarify the detailed biological activities of galectin 9 and develop galectin 9-related techniques including development of drugs. Human galectin 9 shows a cytotoxic activity and an apoptosis-inducing activity on tumor cells but shows neither cytotoxic activity nor apoptosis-inducing activity on normal cells. Therefore, it is possible to employ galectin 9 proteins, galectin 9 agonists, galectin 9 antagonists, anti-galectin 9 binding protein antibodies, anti-galectin 9 binding sugar chain antibodies, galectin 9-producing, releasing or inducing substances, etc. as antitumor, antiallergic, immunosuppressive agents, drugs for autoimmune diseases, anti-inflammatory agents and active ingredients for adrenocortical steroid hormone alternatives.

## Description

### FIELD OF THE INVENTION

The present invention relates to techniques utilizing cytotoxic actions on malignant tumor cells, malignant tumor cell-apoptosis-inducing actions, anti-tumor (antineoplastic) actions on malignant tumor cells, and actions of inducing activated T cell-apoptosis (especially, CD4-positive T cell-apoptosis), as well as immunosuppressive, anti-inflammatory, and anti-allergic actions, owned by galectin 9, especially human galectin 9 (Gal-9). The present invention relates to anti-tumor (antineoplastic), anti-allergic, immunosuppressive agents, drugs for auto-immune diseases, anti-inflammatory agents, and adrenocortical steroid hormone alternatives, to which galectin 9 and related techniques are applied.

### BACKGROUND OF THE INVENTION

It has been reported that galectin-9 raises apoptosis in mouse thymocytes. It has been also reported that, although galectin-9 induces T cell apoptosis in rat spleen cells, it does specifically in CD8+ T cells, but not in CD4+ T cells. Also, other galectins such as galectin-1 have been reported to induce apoptosis in activated T cells. Meanwhile, inhibition of apoptosis is known to be observed by galectin-3 gene transfer (Non-Patent Documents 1 to 4).

The inventor and et al. have succeeded in the cloning of a human T cell-derived eosinophilic chemotactic factor and found (Non-Patent Document 6) that it was ecalectin, a variant of human galectin-9 reported by Tureci et al. (Non-Patent Document 5). In addition, they have demonstrated that ecalectin and galectin-9 are identical substances and that there are 3 types for human galectin-9, i.e., short, medium, and long types, depending on the length of the link peptide (Non-Patent Document 7).

Allergy and autoimmune diseases are induced by exaggerated immune responses of CD4+ T lymphocytes. Steroid hormones or immunosuppressive agents are used for the treatment of refractory allergies or autoimmune diseases. However, steroids cause many problems because of their severe side effects, as well as some existing immunosuppressive agents.

[Non-Patent Document 1] Wada J. et al., J Clin Invest., 1997, 99: 2452-61
[Non-Patent Document 2] Tsuchiyama Y. et al., Kidney Int., 2000, 58: 1941-52
[Non-Patent Document 3] Perillo NL et al., Nature, Dec. 14, 1995, 378(6558): 736-9
[Non-Patent Document 4] Matarrese P. et al., Int J Cancer., Feb. 15, 2000, 85(4): 545-54
[Non-Patent Document 5] Tureci O. et al., J Biol Chem., Mar. 7, 1997, 272(10):6416-22
[Non-Patent Document 6] Matsumoto R. et al., J Biol Chem., 1998, 273:16976-84
[Non-Patent Document 7] Matsushita N. et al., J Biol Chem., 2000, 275:8355-60

Many physiologically active substances have been found within the living body, however, most of them cannot necessarily be put into practical use for pharmaceuticals with ease, even if some of their activities are demonstrated, since, for example, they work on normal cells in the same way as tumor cells.

Galectin-9 is one of the galectins, namely β-galactoside-binding lectins. Galectins exhibit a wide variety of biological activities, including development and differentiation of organisms, cell proliferation, apoptosis, intercellular adhesion, adhesion between cells and extracellular matrix, and migration. Among galectins, galectin-9 is a substance whose production and release can be induced by various stimuli, wherein its production is dissociated with its release. In addition, different functions of galectin-9 are predicted, depending on their localizations such as cytoplasm, cell membrane, and extracellular sites.

Therefore, technological development associated with galectin-9 is required, starting with the elucidation of detailed biological activities of galectin-9 and drug development based on findings.

### SUMMARY OF THE INVENTION

Galectin-9 induces apoptosis in activated T lymphocytes. In addition, galectin-9 induces apoptosis in activated CD4+ and CD8+ T lymphocytes, but not in non-activated T lymphocytes. The CD4+ lymphocytes are more sensitive to the apoptosis induced by galectin-9. Also, it has been successfully demonstrated that apoptosis by galectin-9 can be induced via pathways including calcium, calpain, caspase-1, caspase-3, or caspase-7. Because of the same pathway as corticosteroid hormones (glucocorticoid) to induce apoptosis, expression of galectin-9 is expected to exert the same biological activities as corticosteroid hormones, which are widely used as antiinflammatory agents, antiallergic agents, or immunosuppressive agents, and this expectation has led to the present invention.

In addition, the inventors have discovered the following phenomena, which have led to the present invention: Galectin-9 exhibits cytotoxicity in tumor cells, but not in normal cells. Galectin-9 brings favorable results by exhibiting characteristic effects (e.g. metastasis inhibition) on tumor cells, especially on malignant tumor cells and metastatic tumor cells. Galectin-9 induces apoptosis in tumor cells, but not in normal cells.

The present invention provides the following:
[1] A pharmaceutical or veterinary drug which comprises
   (i) an effective amount of at least one or more members selected from the group consisting of
      (a) galectin 9 and analogs thereof;
      (b) polynucleotides each coding for galectin 9 or a polypeptide having a biological activity substantially equivalent to that owned by galectin 9;
      (c) inducing factors for production and/or release of galectin 9;
      (d) anti-galectin 9-receptor antibodies; and
      (e) antibodies against a galectin 9-binding saccharide,
   (ii) wherein said drug is selected from the group consisting of anti-tumor (antineoplastic) agents, anti-allergic agents, immunosuppressants, drugs for auto-immune diseases, anti-inflammatory agents, and active components for adrenocortical steroid hormone alternatives.
[2] An antineoplastic agent comprising an effective amount of at least one or more members selected from the group consisting of galectin 9 and peptide analogs thereof.
[3] An anti-allergic agent comprising an effective amount of at least one or more members selected from the group consisting of galectin 9 and peptide analogs thereof.
[4] An immunosuppressant comprising an effective amount of at least one or more members selected from the group consisting of galectin 9 and peptide analogs thereof.
[5] A drug for auto-immune diseases, comprising an effective amount of at least one or more members selected from the group consisting of galectin 9 and peptide analogs thereof.
[6] An anti-inflammatory agent comprising an effective amount of at least one or more members selected from the group consisting of galectin 9 and peptide analogs thereof.
[7] An adrenocortical steroid hormone alternative comprising an effective amount of at least one or more members selected from the group consisting of galectin 9 and peptide analogs thereof.
[8] A tumor cytotoxic therapeutic agent for malignant cells which comprises an effective amount of at least one or more members selected from the group consisting of (a) galectin 9 and Gal-9 analogs, and (b) polynucleotides each coding for galectin 9 or a polypeptide having a biological activity substantially equivalent to that owned by galectin9.
(9) An antimetastatic agent for cancer cells, which comprises an effective amount of at least one or more members selected from the group consisting of (a) galectin 9 and Gal-9 analogs, and (b) polynucleotides each coding for galectin 9 or a polypeptide having a biological activity substantially equivalent to that owned by galectin9.
[10] A cytotoxic drug for tumor cells which comprises an effective amount of at least one or more members selected from the group consisting of
   (a) galectin 9 and analogs thereof;
   (b) polynucleotides each coding for galectin 9 or a polypeptide having a biological activity substantially equivalent to that owned by galectin 9;
   (c) inducing factors for production and/or release of galectin 9;
   (d) anti-galectin 9-receptor antibodies; and
   (e) antibodies against a galectin 9-binding saccharide.
[11] An apoptosis-inducing drug for tumor cells, which comprises an effective amount of at least one or more members selected from the group consisting of
   (a) galectin 9 and analogs thereof;
   (b) polynucleotides each coding for galectin 9 or a polypeptide having a biological activity substantially equivalent to that owned by galectin 9;
   (c) inducing factors for production and/or release of galectin 9;
   (d) anti-galectin 9-receptor antibodies; and
   (e) antibodies against a galectin 9-binding saccharide.
[12] An apoptosis-inducing drug for immune cells, including especially activated T cells, which comprises an effective amount of at least one or more members selected from the group consisting of
   (a) galectin 9 and analogs thereof;
   (b) polynucleotides each coding for galectin 9 or a polypeptide having a biological activity substantially equivalent to that owned by galectin 9;
   (c) inducing factors for production and/or release of galectin 9;
   (d) anti-galectin 9-receptor antibodies; and
   (e) antibodies against a galectin 9-binding saccharide.
[13] A prophylactic and/or therapeutic agent for diseases or pathological conditions, caused by activated T cells, which comprises an effective amount of at least one or more members selected from the group consisting of
   (a) galectin 9 and analogs thereof;
   (b) polynucleotides each coding for galectin 9 or a polypeptide having a biological activity substantially equivalent to that owned by galectin 9;
   (c) inducing factors for production and/or release of galectin 9;
   (d) anti-galectin 9-receptor antibodies; and
   (e) antibodies against a galectin 9-binding saccharide.
[14] The prophylactic and/or therapeutic agent according to the above (13) wherein the disease caused by activated T cells is selected from the group consisting of allergy, autoimmune diseases, graft rejection in transplantation, and inflammation.
[15] A galectin 9-binding factor which is selected from the group consisting of
   4F2 heavy chain antigen (177216);
   ATPase, Na⁺/K⁺ transporting, alpha 1 polypeptide (21361181);
   sodium-dependent neutral amino acid transporter type 2 truncated isoform (15004317);
   stromal cell derived factor receptor 1 isoform a (9257240);
   stromal cell derived factor receptor 1 isoform b;
   heat shock 90kDa protein 1, beta (20149594);
   heat shock 90kDa protein 1, alpha;
   heat shock 70kDa protein 5 (glucose-regulated protein, 78kDa) (16507237);
   heat shock 70kDa protein 8 isoform 2 (24234686);
   heat shock 70kDa protein 9B precursor (24234688);
   fatty-acid-Coenzyme A ligase, long-chain 3 (27469830);
   NADH dehydrogenase (ubiquinone) Fe-S protein 1, 75kDa (NADH-coenzyme Q reductase) (4826856);
   S-adenosylhomocysteine hydrolase-like 1 (21361647); programmed cell death 8 isoform 1 (4757732);
   60 kDa heat shock protein, mitochondrial precursor (129379);
   ATP synthase, H⁺ transporting, mitochondrial F1 complex, alpha subunit, isoform 1, cardiac muscle (4757810);
   ribophorin II precursor (88567);
   farnesyl-diphosphate farnesyltransferase 1 (4758350);
   Ubiquinol-cytochrome C reductase complex core protein 2, mitochondrial precursor (21903482);
   dolichyl-diphosphooligosaccharide-protein glycosyltransferase (21104416);
   calcium-binding transporter (6841066);
   NADH dehydrogenase-ubiquinone Fe-S protein 2 precursor (3540239);
   actin, beta (14250401);
   translation elongation factor EF-Tu-like protein P43 precursor, mitochondrial (7443384);
   metaxin 1 (4505281); sideroflexin 1 (23618867);
   TCR beta chain (2982508); Hnrnp A1 (2194069);
   phosphate carrier precursor isoform 1b (4505775);
   ATP synthase, H⁺ transporting, mitochondrial F1 complex, gamma polypeptide 1 (4885079);
   voltage-dependent anion channel 1 (4507879);
   hyaluronan-binding protein precursor (8699626);
   androgen-regulated short-chain dehydrogenase/reductase 1 (20070798);
   solute carrier family 25 (mitochondrial carrier;
   oxoglutarate carrier), member 11 (21361114);
   3-hydroxybutyrate dehydrogenase precursor (17738292);
   B-cell receptor associated protein (1673514);
   ATP synthase, H⁺ transporting, mitochondrial F1 complex, O subunit (4502303);
   ATP synthase, H⁺ transporting, mitochondrial F0 complex, subunit d (5453559);
   ATP synthase, H⁺ transporting, mitochondrial F0 complex, subunit b, isoform 1 (21361565);
   small GTP-binding protein (13569962);
   NADH dehydrogenase (ubiquinone) Fe-S protein 8, 23kDa (NADH-coenzyme Q reductase) (4505371);
   vesicle trafficking protein sec22b (4759086);
   mitochondrial import receptor Tom22 (9910382);
   signal sequence receptor, delta (5454090);
   ATP synthase, alpha chain (114517 or P25705);
   ATP synthase, beta chain (114549 or P06576);
   Sodium/potassium-transporting ATPase beta-3 chain (1703470 or P54709);
   ADP, ATP carrier protein (113463, P12236, 113459, P05141, 113455 or P12235);
   ubiquinol-cytochrome C reductase complex core protein 1 (731047 or P31930), and
   Cytochrome c oxidase polypeptide II (117020 or P00403)
   wherein each number in parentheses indicates a "GenInfo Identifier" sequence identification number assigned to each specific protein in databases where protein information and/or nucleotide sequence information (including data of DNA coding for the protein) is acquired by the entry of said number at the NCBI internet home page (http://www.ncbi.nlm.nih.gov/).
(16) A technique for controlling the activity of galectin 9 which comprises utilizing any of interactions between the galectin 9-binding factor according to Claim 15 and galectin 9.

The above objectives and other objectives, features, advantages, and aspects of the present invention are readily apparent to those skilled in the art from the following disclosures. It should be understood, however, that the description of the specification including the following best modes of carrying out the invention, examples, etc. is illustrating preferred embodiments of the present invention and given only for explanation thereof. It will become apparent to the skilled in the art that a great number of variations and/or alterations (or modifications) of this invention may be made based on knowledge from the disclosure in the following parts and other parts of the specification without departing from the spirit and scope thereof as disclosed herein. All of the patent publications and reference documents cited herein for illustrative purposes are hereby incorporated by reference into the present disclosure.

### BRIEF DESCRIPTION OF THE DRAWINGS

Fig. 1: (A) shows flow cytometry results wherein the apoptosis inducing efficacy of rGal-9 was examined by the PI method. (B) shows flow cytometry results wherein the apoptosis inducing efficacy of rGal-9 was examined by the Annexin V analysis. Data is a representative example of 4 experiments.
Fig. 2 shows assay results wherein the apoptosis inducing efficacy of rGal-9 was measured in the presence or absence of lactose or sucrose. Data is mean ± SEM of 4 experiments.
Fig. 3 shows flow cytometry results wherein the apoptosis inducing efficacy of rGal-9 was examined in various cell lines by the PI method. Data is mean ± SEM of 3 experiments.
Fig. 4 shows flow cytometry results wherein the apoptosis inducing efficacy of rGal-9 was examined in peripheral blood T cells by the PI method. Data is mean ± SEM of 3 experiments.
Fig. 5 shows experimental results of inhibitory effects on Gal-9-induced apoptosis in the presence of various caspase inhibitors. Data is mean ± SEM of 3 experiments.
Fig. 6 shows experimental results of inhibitory effects on Gal-9-induced apoptosis in the presence of pan-caspase or caspase-1 inhibitor at different concentrations. Data is mean ± SEM of 3 experiments.
Fig. 7 shows experimental results of inhibitory effects on Gal-9-induced apoptosis in the presence of calpain inhibitor at different concentrations. Data is mean ± SEM of 3 experiments.
Fig. 8 shows experimental results wherein the efficacy of Gal-9 on calcium influx was examined using cells that underwent Gal-9-induced apoptosis. Data is mean ± SEM of 3 experiments.
Fig. 9 shows experimental results wherein the efficacy or action of Gal-9 was examined on apoptosis induced by DEX, anti-Fas Ab, or etoposide. Data is mean ± SEM of 4 experiments.
Fig. 10 shows flow cytometry results wherein the apoptosis-inducing efficacy of Gal-9 was examined on a malignant melanoma cell line, MM-RU, by PI method.
Fig. 11 shows electrophoresis profiles (photos) illustrating analysis results wherein the amount of galectin DNA was assayed using RT-PCR after extracting mRNA from human melanoma cell lines MM-BP and MM-RU.
Fig. 12 is a set of photos illustrating morphologic configurations of colony-forming breast cancer cell line MCF-7, and MCF-7 K-10 without any obvious colony formation (novel cell line established from MCF-7).
Fig. 13 shows electrophoresis profiles (photo) illustrating the western blot results of galectin-9 in colony-forming cell line MCF-7 K-4, and MCF-7 K-10 without any obvious colony formation (novel established cell line).
Fig. 14 is a set of photos illustrating the resultant morphologic configurations of melanoma cell line MM-RU, wherein the efficacy of galectin-9 was examined by adding recombinant galectin-9 to MM-RU cells exogenously.
Fig. 15 is a set of photos illustrating the resultant morphologic configurations of MCF-7 K-10, wherein the efficacy of galectin-9 gene transfer was examined into MCF-7 K-10 cells established from the breast cancer cell line.
Fig. 16 shows electrophoresis profiles (photos) illustrating galectin-9CT column-adsorbed MOLT4-derived protein patterns.

### BEST MODES OF CARRYING OUT THE INVENTION

The present invention is based on the findings that human galectin-9 shows cytotoxic activity against tumor cells but does not show cytotoxic activity against normal cells, and that galectin-9 induces apoptosis in tumor cells but does not induce apoptosis in normal cells. These findings suggest that galectin-9 proteins, galectin-9 agonists, galectin-9-antagonist antagonists, anti-galectin-9 binding protein antibodies, anti-galectin-9-binding saccharide chain antibodies, and galectin-9 production/release-inducing substances can be used as antineoplastic agents (anti-tumor agents) which induce apoptosis in tumor cells and are cytotoxic to tumor cells, but do not affect normal cells. Furthermore, the present invention provides methods for inhibiting cancer metastasis by utilizing the fact that human galectin-9 influences metastatic malignant cells to induce aggregation and has metastasis-inhibiting activity against malignant tumors such as cancers. Such methods for inhibiting cancer metastasis are performed by administration of galectin-9 protein or its derivatives, gene transfer of galectin-9, or inducing production and release of galectin-9. The present invention provides reagents, kits, and systems (including qualitative analysis and quantitative analysis) used for the methods.

The present invention is based on the fact that human galectin-9 does not induce apoptosis in resting T cells, particularly, in CD4-positive T cells (helper T cells) and slightly induce apoptosis in resting CD8-positive T cells (suppressor T cells and cytotoxic T cells), but human galectin-9 markedly induce apoptosis in CD4-positive T cells (helper T cells) activated by CD3 and also induce apoptosis in activated CD8-positive T cells (suppressor T cells and cytotoxic T cells) higher than that galectin-1 or galectin-3 does.

The addition of human galectin-3 induces apoptosis in activated CD4-positive T cells (helper T cells) and in activated CD8-positive T cells (suppressor T cells and cytotoxic T cells). It is observed that apoptosis induction by galectin-9 is concentration-dependent and time-dependent. It is known that CD4-positive T cells have a very important function for inducing an immune reaction. It is assumed that various autoimmune diseases and allergies are induced by an overresponse of CD4-positive T cells.

As mentioned above, particularly, galectin-9 markedly induces apoptosis in activated CD4-positive T cells (helper T cells). Therefore, it is assumed that galectin, specifically, galectin-9 protein, gene transfer of galectin-9, or the inducing of production and release of galectin-9 induces apoptosis in CD4-positive T cells to show immune suppression and anti-inflammatory effects. With these technologies, galectin-9 can be used as a pharmaceutical drug for autoimmune diseases, allergies, and further as an anti-inflammatory agent. Additionally, since the above-mentioned T cells are immune cells, galectin-9 has an activity to induce apoptosis in immune cells.

### [Revelation of galectin-9-induced apoptosis pathways]

Apoptosis is induced by various substances such as glucocorticoids which serve as immunosuppressive agents, Fas ligands, and anti-Fas antibodies. In the present invention, it is elucidated how apoptosis is induced by galectin-9. Namely, it was revealed by the propidium iodide (PI) and Annexin V methods using Jurkat cells (T-cell line) or MOLT4 cells, instead of human peripheral blood T cells, that galectin-9 induces apoptosis in these cells. Furthermore, the apoptosis induced by galectin-9 is inhibited by lactose but not by sucrose. Therefore, the β-galactoside-binding activity of galectin-9 is necessary for induction of apoptosis.

According to the present invention, it has been investigated whether caspase is responsible to apoptosis induced by galectin-9. A pan-caspase inhibitor concentration-dependently and time-dependently inhibits apoptosis induced by galectin-9, as well as apoptosis induced by dexamethasone, anti-Fas Ab, TNF-α, or C2-ceramide. Furthermore, inhibitors, which inhibit caspase-1, 8, 9, and 10 in upstream, have been investigated by using a caspase-1 inhibitor, caspase-8 inhibitor, caspase-9 inhibitor, and caspase-10 inhibitor. As a result, galectin-9-induced apoptosis is inhibited by a caspase-1-specific inhibitor but not by other caspase-specific inhibitors. Similarly, dexamethasone-induced apoptosis is inhibited by the caspase-1 inhibitor. Apoptosis induced by anti-Fas Ab or TNF-α is inhibited by the caspase-8 inhibitor and the caspase-10 inhibitor, and apoptosis induced by C2-ceramide is inhibited by the caspase-9 inhibitor. With these results, it is assumed that galectin-9 induces apoptosis via a pathway similar to that of dexamethasone.

It is known that activation of calpain precedes activation of caspase-1. Consequently, effects of calpain inhibitors on galectin-9-induced apoptosis have been studied. As a result, it was observed that the calpain inhibitors concentration-dependently inhibit apoptosis induced by galectin-9. Since calcium influx into cells is observed before the activation of calpain, it has been studied whether calcium influx is stimulated by galectin-9. It has been recognized that galectin-9 obviously induces calcium influx into cells. The influx is inhibited by lactose but not by sucrose. The results show that β-galactoside-binding activity is required for calcium influx by galectin-9. With these results, it is assumed that galectin-9 induces apoptosis through a pathway similar to that in glucocorticoid (dexamethasone), i.e. from a receptor → calcium influx → calpain → caspase-1 → caspase-3 and caspase-7.

Effects of galectin-9 against apoptosis induced by dexamethasone, anti-Fas Ab, or etoposide have been examined to confirm the fact that galectin-9 has an additive effect on apoptosis induced by the anti-Fas antibody and etoposide, but does not show an additive effect on apoptosis induced by dexamethasone. In contrary, galectin-9 inhibits apoptosis induced by dexamethasone in eosinophilic leukocytes. Thus, it is strongly suggested that galectin-9 and glucocorticoid induce apoptosis through pathways similar to each other. As mentioned above, according to the present invention, galectin-9 has high possibility to serve as an anti-inflammatory agent, anti-allergy agent, or immunosuppressive agent. Galectin-9 proteins, galectin-9 gene transfers, or induction of galectin-9, can serve as an immunosuppressive agents having a low side effect. Furthermore, a combination of galectin-9 with glucocorticoid can reduce the dose levels of glucocorticoids, resulting in a decrease of side effects.

At present, glucocorticoids are broadly used as anti-inflammatory agents, anti-allergy agents, and immunosuppressive agents; and immunosuppressants such as FK506 are used for refractory allergies. However, these drugs cause severe side effects. The above-mentioned results suggest that galectin-9 can be used as an alternative drug for glucocorticoid to exhibit biological activities equivalent to or better than those of glucocorticoid. Therefore, antineoplastic agents, anti-allergy agents, immunosuppressive agents, anti-autoimmune disease agents, anti-inflammatory agents, and active substitutes for adrenocortical steroid hormones can be provided by using galectin-9 and its analogues, or by controlling concentration or expression of galectin-9 in vivo. Furthermore, the use of galectin-9 can be applied to a field in which pharmacological effects and biological activities of glucocorticoid are utilized.

Allergies and auto-immune diseases are induced by an excessive immune reaction of CD4-positive T lymphocytes, and steroids and immunosuppressive agents are used for treating refractory allergies and auto-immune diseases. Therefore, it is obvious that galectin-9 has an immunosuppressive activity, anti-inflammatory activity, and anti-allergy activity. Thus, galectin-9 proteins, gene transfers of galectin-9, galectin-9 production/release-inducing factors, anti-galectin-9 receptor antibodies, and antibodies against galectin-9-binding saccharide chain can be utilized as antineoplastic agents, anti-allergy agents, anti-autoimmune disease agents, anti-inflammatory agents, and substitutes for adrenocortical steroid hormones, instead of steroids and immunosuppressants.

In the present invention, utilization of "gene recombination techniques" enables not only acquisition, isolation, and sequencing of targeted nucleic acids, peptides and fragments thereof, but also construction and production of recombinants thereof. Gene recombination techniques (including recombinant DNA techniques) as can be used herein include those known in the art, and can be carried out by the methods described in, for example, J. Sambrook, E. F. Fritsch & T. Maniatis, "Molecular Cloning: A Laboratory Manual (2nd edition)", Cold Spring Harbor Laboratory Press, Cold Spring Harbor, New York (1989); D. M. Glover et al. ed., "DNA Cloning", 2nd ed., Vol. 1 to 4, (The Practical Approach Series), IRL Press, Oxford University Press (1995); The Japanese Biochemical Society (JBS) ed., "Zoku-Seikagaku Jikken Koza 1, Idenshi Kenkyu-Hou II", Tokyo Kagaku Dozin Co. Ltd., Japan, (1986); JBS ed., "Shin-Seikagaku Jikken Koza 2, Kakusan III (Recombinant DNA technique)", Tokyo Kagaku Dozin Co. Ltd., Japan, (1992); R. Wu ed., "Methods in Enzymology", Vol. 68 (Recombinant DNA), Academic Press, New York (1980); R. Wu et al. ed., "Methods in Enzymology", Vol. 100 (Recombinant DNA, Part B) & 101 (Recombinant DNA, Part C), Academic Press, New York (1983); R. Wu et al. ed., "Methods in Enzymology", Vol. 153 (Recombinant DNA, Part D), 154 (Recombinant DNA, Part E) & 155 (Recombinant DNA, Part F), Academic Press, New York (1987); J. H. Miller ed., "Methods in Enzymology", Vol. 204, Academic Press, New York (1991); R. Wu et al. ed., "Methods in Enzymology", Vol. 218, Academic Press, New York (1993); S. Weissman (ed.), "Methods in Enzymology", Vol. 303, Academic Press, New York (1999); J. C. Glorioso et al. (ed.), "Methods in Enzymology", Vol. 306, Academic Press, New York (1999) etc., or by methods described in the references quoted therein or methods substantially equivalent thereto or modified methods thereof, the disclosures of which are incorporated herein by reference (hereinafter, all such techniques or methods shall be referred to as "gene recombination techniques").

As used herein, the term "homology" or "homologous" means the quantity (or number), in terms of identity, which can be obtained by determining that corresponding amino acid residues or corresponding nucleotide bases are matched each other between two chains in polypeptide sequences (or amino acid sequences) or polynucleotide sequences (or base sequences) when amino acid residues or nucleotide bases constituting the chain are compared one another between the two chains and it also means the level of sequence correlation in terms of similarity between two polypeptide sequences or two polynucleotide sequences. The homology can be easily calculated. Various methods for measuring the homology between two polynucleotide sequences or polypeptide sequences have been known and the term "homology" (sometimes called "identity") has been well known to the persons skilled in the art (for example, Lesk, A. M. (Ed.), Computational Molecular Biology, Oxford University Press, New York, (1988); Smith, D. W. (Ed.), Biocomputing: Informatics and Genome Projects, Academic Press, New York, (1993); Grifin, A. M. & Grifin, H. G. (Ed.), Computer Analysis of Sequence Data: Part I, Human Press, New Jersey, (1994); von Heinje, G., Sequence Analysis in Molecular Biology, Academic Press, New York, (1987); Gribskov, M. & Devereux, J. (Ed.), Sequence Analysis Primer, M-Stockton Press, New York, (1991), etc.). General techniques for determining the homology between two strands include those disclosed in Martin, J. Bishop (Ed.), Guide to Huge Computers, Academic Press, San Diego, (1994); Carillo, H. & Lipman, D., SIAM J. Applied Math., 48: 1073 (1988), etc., but are not limited to. Preferred methods for measuring the homology include those which are designed so as to obtain the part of the highest fitting relation between the two sequences tested. An example of such methods is a technique which is constructed as a computer program. Preferred computer programming methods include a GCG program package (Devereux, J. et al., Nucleic Acids Research, 12(1): 387 (1984)), BLASTP, BLASTN, FASTA (Atschul, S. F. et al., J. Mol. Biol., 215: 403 (1990)), etc., but are not limited to. For such methods, those known in the art may be employed.

The term "polypeptide(s)" used herein may indicate any polypeptide as described herein below. The basic structure of polypeptides is well known, and described in a great number of reference books and other documents in this technology field. In view of this fact, the term "polypeptide(s)" used herein encompasses any of peptides or proteins composed of two or more amino acids which are linked together to each other by peptide bonds or modified peptide bonds. The term "polypeptide(s)" used herein may ordinarily cover both of short chain ones such as called peptides, oligopeptides or peptide oligomers, and long-chain ones such as in general called proteins, many types or forms of which are known. The polypeptide may often contain amino acids other than the naturally occurring type amino acids (natural amino acids: or gene-encoded amino acids). It is to be understood that the polypeptides may be altered (modified) by either natural processes, such as posttranslational processing and other alterations (or modifications), or by chemical modification techniques which are well known in the art, wherein such modifications can occur anywhere in the given polypeptide, including the peptide backbone, the amino acid side-chains and the amino or carboxyl termini. It is known that a given polypeptide may contain many types of modifications (or alterations). Such modifications are well described in basic texts and in more detailed monographs, as well as in a voluminous research literature, and well known to artisans in the art. Some examples of customary alterations and modifications are alkylation, acylation, esterification, amidation, glycosylation, covalent attachment of a lipid or lipid derivative, sulfation, phosphorylation, γ-carboxylation of a glutamic acid residue, hydroxylation, and ADP-ribosylation, and others; see for instance, T. E. Creighton, Proteins-Structure and Molecular Properties, Second Edition, W. H. Freeman and Company, New York, (1993); B.C.Johnson (Ed.), Posttranslational Covalent Modification of Proteins, Academic Press, New York, (1983) (Wold, F., "Posttranslational Protein Modifications: Perspective and Prospects", pp.1-12); Seifter et al., "Analysis for Protein Modifications and nonprotein cofactors", Methods in Enzymology, 182: 626-646 (1990); Rattan et al., "Protein Synthesis: Posttranslational Modification and Aging", Ann. N. Y. Acad. Sci., 663: p.48-62 (1992), etc.

As used herein, the term "galectin-9" shall refer inclusively to naturally-occurring type (native) galectin 9 species. Long type galectin-9 (Gal-9L), medium type galectin-9 (Gal-9M), and short type galectin-9 (Gal-9S) have been reported at present in connection with the naturally-occurring type galectin 9 species. It is understood that long type galectin-9 (Gal-9L) is a molecule where the N-terminal domain (N-CRD) is linked to the C-terminal domain (C-CRD) via the putative link peptide of SEQ ID NO: 4 as set forth in the Sequence Listing of WO 02/37114 A1, medium type galectin-9 (Gal-9M) a molecule where the N-terminal domain (N-CRD) is linked to the C-terminal domain (C-CRD) via the putative link peptide of SEQ ID NO: 5 as set forth in the Sequence Listing of WO 02/37114 A1, and short type galectin-9 (Gal-9S) a molecule where the N-terminal domain (N-CRD) is linked to the C-terminal domain (C-CRD) via the putative link peptide of SEQ ID NO: 6 as set forth in the Sequence Listing of WO 02/37114 A1, respectively. Gal-9M is an amino acid deletion form wherein the constituent amino acid residues of the SEQ ID NO: 7 amino acid sequence as set forth in WO 02/37114 A1 are deleted from said link peptide region in Gal-9L, thereby being discriminated from Gal-9L. Gal-9S is an amino acid deletion form wherein the constituent amino acid residues of the SEQ ID NO: 8 amino acid sequence as set forth in WO 02/37114 A1 are deleted from said link peptide region in Gal-9M, thereby being discriminated from Gal-9M. Thus, Gal-9S is an amino acid deletion form wherein the constituent amino acid residues of the SEQ ID NO: 9 amino acid sequence as set forth in WO 02/37114 A1 are deleted from said link peptide region in Gal-9L, thereby being discriminated from Gal-9L.

Herein, the galectin 9 (Gal-9) may encompass the abovementioned Gal-9L, Gal-9M, Gal-9S, other naturally-occurring variants of the galectin-9 family, compounds obtained by incorporating artificial mutations (that is, deletions, additions, modifications, insertions with respect to one or more amino acid residues) into such galectin-9 peptides, and those containing a partial domain or partial peptide fragment thereof.

Representatives of the galectin 9 proteins according to the present invention include those having any amino acid sequence of SEQ ID NO:1 to 3 in the Sequence Listing as set forth in WO 02/37114 A1, or a substantially equivalent amino acid sequence thereof. Examples of the galectin 9 protein are those having not only at least 5 to 311, 5 to 323 or 5 to 355 contiguous amino acid residues contained in any amino acid sequence of SEQ ID NO:1, 2 and 3, but also a substantially equivalent biological activity such as antigenicity; those having not only any of their characteristic properties, but also at least 50% or higher homology, at least 60% or higher homology, at least 70% or higher homology, at least 80% or higher homology, at least 90% or higher homology, at least 95% or higher homology, or at least 98% or higher homology, to any of the respective domains contained in SEQ ID NO:1, 2 and 3 as set forth in WO 02/37114 A1; and others.

The human galectin 9 polypeptides include those having a sequence with contiguous amino acid residues which comprise all or part of any amino acid sequence of SEQ ID NO:1 to 3 in the Sequence Listing disclosed in WO 02/37114 A1, or those having a sequence with 5 or more contiguous amino acid residues, preferably 10 or more, still preferably 20 or more, yet preferably 30 or more, further preferably 40 or more, more preferably 50 or more, still more preferably 60 or more, further preferably 70 or more, yet more preferably 80 or more, still further more preferably 90 or more, yet most preferably 100 or more, and still most preferably 110 or more amino acid residues, contained in any amino acid sequence of SEQ ID NO:1 to 3 in the Sequence Listing disclosed in WO 02/37114. The human galectin 9-related polypeptides according to the present invention may contain all or part of an amino acid sequence selected from the aforementioned SEQ ID NO:1 to 3 (Met may lack which corresponds to the initiation codon). All of those having such a sequence are encompassed by these.

The nucleic acids coding for galectin 9, its constituent domain or fragment may be any as long as they contain a nucleotide sequence with the same efficacy as galectin 9, i.e., one of those encoding a peptide with substantially equivalent biological activity such as equal antigenicity, to that exerted by galectin 9. The galectin 9-coding nucleic acids are single- and double-stranded DNA, RNA, DNA:RNA hybrids, synthetic DNA, and others. They may be any of human genome DNA, human genomic DNA libraries, human tissue/cell-derived cDNA, and synthetic DNA. The galectin 9-coding nucleotide sequences can be modified (by addition, deletion, substitution, etc.), and those thus modified may be encompassed herein. Amino acid mutations may also occur naturally. Further, the nucleic acids may include those encoding any of galectin-9L, -9M, and -9S peptides and fragments thereof, and are preferably DNA. The terms "nucleotide sequence with the same efficacy", "equivalently effective nucleotide sequence" and "equivalent nucleotide sequence" refer to those which hybridize with a nucleotide sequence with 5 or more contiguous nucleotide residues, preferably 10 or more contiguous nucleotide residues, more preferably 15 or more contiguous nucleotide residues, and still more preferably 20 or more contiguous nucleotide residues, in the nucleotide sequence encoding the amino acid sequence of SEQ ID NO: 1 in the Sequence Listing disclosed in WO 02/37114 A1, for example, under stringent conditions, and encode a substantially equivalent amino acid sequence to human galectin 9, their complementary strands, etc.

The galectin 9-coding nucleic acids are any as long as they are typically those comprising a nucleotide sequence coding for a peptide represented by any of SEQ ID NO:1 to 3 in the Sequence Listing of WO 02/37114 A1 or a consecutive amino acid sequence selected from part of said peptides (including those coding for the respective characteristic domains exclusively), those in which an initiation codon (codon coding for Met) and a termination codon (stop codon) are added to the coding sequence, and those coding for a peptide having not only an amino acid sequence with at least 50% homology to the protein encoded by said nucleotide sequence wherein said amino acid sequence contains at least contiguous unique amino acid residues contained in any amino acid sequence of said SEQ ID NO:1 to 3, but also a substantially equivalent biological activity such as an equivalent antigenicity, that is, those containing a nucleotide sequence with the same efficacy.

The term "polymerase chain reaction" or "PCR" used herein usually refers to techniques described in U.S. Pat. No. 4,683,195 and other documents. For example, the PCR is an in vitro method for the enzymatic amplification of desired specific nucleotide sequences. In general, the PCR includes repetitive series of cycles wherein a primer elongation synthesis is constructed using two oligonucleotide primers capable of preferentially hybridizing with a template nucleic acid. Typically, the primers used in PCR may include those which are complementary to the internal nucleotide sequence of interest in the template. For example, preferable primer pairs as used herein may be those which are complementary to both ends of said nucleotide sequence to be amplified, or flanking regions adjacent to said nucleotide sequence. It is preferable to select a 5'-terminal primer such that at least an initiation codon is contained or the amplification can be performed including the initiation codon, and to select a 3'-terminal primer such that at least a stop codon is contained or the amplification can be performed including the stop codon. The primers include oligonucleotides made up of preferably 5 or more nucleotide bases, more preferably 10 or more nucleotide bases, and still preferably 18 to 25 nucleotide bases.

The PCR reactions can be carried out by methods known in the art or methods substantially equivalent thereto and modified methods thereof. For example, the PCR can be performed according to methods described in R. Saiki, et al., Science, 230: 1350, 1985; R. Saiki, et al., Science, 239: 487, 1988 ; H. A. Erlich ed., PCR Technology, Stockton Press, 1989 ; D. M. Glover et al. ed., "DNA Cloning", 2nd ed., Vol. 1, (The Practical Approach Series), IRL Press, Oxford University Press (1995) ; M. A. Innis et al. ed., "PCR Protocols: a guide to methods and applications", Academic Press, New York (1990)); M. J. McPherson, P. Quirke and G. R. Taylor (Ed.), PCR: a practical approach, IRL Press, Oxford (1991); M. A. Frohman et al., Proc. Natl. Acad. Sci. USA, 85, 8998-9002 (1988), and modified methods or variants thereof. The PCR methods can also be performed using commercially available kits suitable therefor, and can also be carried out according to protocols disclosed by manufacturers or distributors of the kits.

For the PCR, in a representative case, for example, a template (e.g., DNA synthesized using mRNA as a template; 1st strand DNA) and primers synthesized according to designs on said gene are mixed with a 10×reaction buffer (attached with a Taq DNA polymerase kit), dNTPs (deoxyribonucleoside triphosphates; dATP, dGTP, dCTP and dTTP mix), Taq DNA polymerase and deionized distilled water. The mixture is subjected to 25 to 60 cycles of amplification using an automated thermal cycler such as GeneAmp 2400 PCR system (Perkin-Elmer/Cetus) under general PCR cycle conditions. The number of amplification cycles can be suitably set to an appropriate value depending on purposes. The PCR cycle includes, for example, denaturation at 90 to 95°C for 5 to 100 sec, annealing at 40 to 60°C for 5 to 150 sec and extension at 65 to 75°C for 30 to 300 sec, and preferably denaturation at 94°C for 15 sec, annealing at 58°C for 15 sec and extension at 72°C for 45 sec. For the annealing temperature and reaction time, an appropriate value is suitably selected by experimentation. For the denaturation and extension time, an appropriate value suitably varies according to the strand length of expected PCR products. In general, the annealing reaction time preferably varies depending on the Tm value of primer-template DNA hybrids. The time period of extension is usually set with the aim of getting about 1 min per 1000 bp in strand length, but it may be possible to select a shorter time period in some cases.

The term "oligonucleotide(s)" used herein refers to a relatively short single-stranded polynucleotide or double-stranded polynucleotides, or preferably polydeoxynucleotide(s). They can be chemically synthesized by known methods as described in Angew. Chem. Int. Ed. Engl., Vol. 28, pp.716-734 (1989), including phosphotriester, phosphodiester, phosphite, phosphoamidite, phosphonate methods, and the like. It has been typically known that the synthesis can be conveniently carried out on modified solid supports. For example, the synthesis can be carried out using an automated synthesizer and such a synthesizer is commercially available. The oligonucleotide may contain one or more modified nucleotide bases and, for example, it may contain a nucleotide base which does not naturally occur, such as inosine, or a tritylated nucleotide base. In some cases, they may contain one or more nucleotide bases tagged with a marker.

Identification of the target nucleic acids (polynucleotides) can be conducted by adaptations of hybridization techniques. The hybridization may be carried out according to methods as disclosed in documents mentioned in the aforementioned "gene recombination techniques", or substantially equivalent methods and modifications thereof. For instance, the hybridization is achieved by transferring a sample containing a nucleic acid such as DNA onto carriers including membranes such as nylon filters, as required, optionally followed by denaturation, fixation, washing, etc., and then reacting the transfers on the carrier (e.g., membrane), with labeled DNA probe fragments which are, as required, optionally denatured in a hybridization buffer. The hybridization operations can be ordinarily conducted at about 35 to about 80°C, more preferably about 50 to about 65°C, for about 15 min to about 36 hours, more preferably about 1 to about 24 hours, but optimal hybridization conditions may be suitably selected. For example, the hybridization is carried out at about 55°C for about 18 hours. The hybridization buffers can be selected from those customarily used in the art. Examples of the hybridization buffers are Rapid hybridization buffer (Amersham), etc. The denaturation of carriers (e.g., membranes) with transfers includes techniques using an alkali denaturing solution. It is preferable to treat the carrier with a neutralizing solution and a buffer solution after the denaturation. The carrier fixation ((e.g., membrane fixation) is usually achieved by baking at about 40° to about 100°C, more preferably about 70° to about 90°C, for about 15 min to about 24 hours, more preferably about 1 to about 4 hours, but desired fixation conditions may be suitably selected. For example, the fixation is carried out by baking at about 80°C for about 2 hours. The washing of carriers (e.g., membranes) with transfers can be performed with washing solutions customarily used in the art, such as 50mM Tris-HCl buffer, pH8.0, containing 1M NaCl, 1mM EDTA and 0.1% sodium dodecyl sulfate (SDS). The carriers including membranes such as nylon filters can be selected from those customarily used in the art.

The alkaline denaturing solution, neutralizing solution and buffer solution can be selected from those conventionally used in the art. The alkaline denaturing solution may include, for example, solutions containing 0.5M NaOH and 1.5M NaCl, etc. The neutralizing solution may include, for example, 0.5M Tris-HCl buffers (pH8.0) containing 1.5M NaCl, etc. The buffer solution may include, for example, 2×SSPE (0.36M NaCl, 20mM NaH₂PO₄ and 2mM EDTA), etc. As required, prior to hybridization, it is desired to optionally prehybridize carriers (e.g., membranes) containing transferred DNA, etc., to prevent non-specific hybridization. For the prehybridization, the sample is dipped, for example, in a solution for prehybridization (50% formamide, 5× Denhardt's solution (0.2% bovine serum albumin and 0.2% polyvinylpyrrolidone), 5×SSPE, 0.1% SDS, and 100 µg/ml thermally denatured salmon sperm DNA), etc., and reacted at about 35 to 50°C, preferably about 42°C, for about 4 to 24 hours, preferably about 6 to 8 hours. These conditions can be determined by those of skill in the art with suitably repeated experiments and preferred conditions would be selected. Labeled probe DNA fragments used in hybridization can be denatured, for example, under heating conditions at about 70 to 100°C, preferably about 100°C, for about 1 to 60 minutes, preferably about 5 minutes, etc. The hybridization is carried out by well known techniques per se in the art or according to methods analogous thereto. As used herein, the stringent conditions refer to, for example, those equivalent to hybridization in about 15 to 50 mM, preferably about 19 to 40 mM, and more preferably about 19 to 20 mM, with regard to Na ion concentration, at about 35 to 85°C, preferably about 50 to 70°C, and more preferably about 60 to 65°C with regard to temperature.

After the hybridization is completed, the carriers (such as filters) are washed extensively to remove labeled probes other than the labeled probe DNA fragments which specifically hybridize. The filter washing process may be performed by a method suitably selected from techniques used in the art. For example, the washing is carried out in 0.5 × SSC solution (×SSC = 0.15M NaCl, 15mM citric acid) containing 0.1% SDS.

The hybridized nucleic acids can be detected representatively by autoradiography, but the detection may be performed by a method suitably selected from techniques used in the art. The nucleic acid bands corresponding to the detected signal are suspended in a suitable buffer solution such as SM solution (50mM Tris-HCl buffer, pH7.5, containing 100mM NaCl and 10mM MgSO₄). After the nucleic acid suspension is diluted to a suitable level, target nucleic acids can be isolated and purified. Further, the nucleic acids can be subjected to amplification. The term "high homology" as used herein may refer to, though it depends on the sequence length of the targets, for example, 50% or higher, further 60% or higher, preferably 70% or higher, still preferably 80% or higher, in a particular case 95% or higher and most preferably 97% or higher homology. The "nucleotide sequence with the same efficacy" or "equivalently effective nucleotide sequence" includes, for example, those which hybridize with any of those containing the sequence of concern under stringent conditions. Examples of such nucleotide sequences are those which hybridize with a nucleotide sequence with 5 or more contiguous nucleotides, preferably 10or more contiguous nucleotides, more preferably 15 or more contiguous nucleotides, or further preferably 20 or more contiguous nucleotides, selected from said nucleotide sequence, and code for a substantially equivalent amino acid sequence to said polypeptide. The nucleic acids may also be chemically synthesized. In such cases, fragments may be chemically synthesized and coupled together with enzymes.

Screening treatments can be repeated plural times with hybridization techniques for target nucleic acids from nucleic acid samples including gene libraries, cDNA libraries, and others. Utilizable cDNA libraries are cloned human-derived ones including, for example, cDNA libraries of various human-derived tissues, cultured human cells, or human cell lines (in particular, human tissues and cells such as kidney, brain, corpus peale, posterior pituitary gland, nerve cells, retina, retinal blood vessel cells, retinal nerve cells, thymus, blood vessel, endothelial cells, vascular smooth muscle cells, blood cells, macrophages, lymphocytes, testis, ovary, uterus, intestine, heart, liver, pancreas, small intestine, large intestine (including colon), gingiva-related cells, skin-related cells, glomerular cells, renal tubular cells, and connective tissue cells; various tumor tissues, and cancer cells; and other sources). Further, the cDNA library used as a template may be directly selected from commercially available cDNA libraries derived from a variety of tissues. Examples of the commercially available cDNA libraries are those commercially distributed or delivered by Stratagene (US), Invitrogen (US), Clontech (US), and other distributors. In typical embodiments, the utilizable products include gene libraries generated from human tissues and cells, such as human P1 artificial chromosome genomic libraries (Human Genome Mapping Resource Center, US), and human tissue cDNA libraries (e.g., available from Clontech, US). The screening can be done using as probes human genomic DNA libraries or human-derived cDNA libraries constructed from various human tissues or culture cell lines and other resources. The probe, etc. may be labeled by a radioactive isotope using a commercially available labeling kit, such as the Random Prime DNA Labeling Kit (Boehringer Mannheim), etc. For example, a random priming kit (Pharmacia LKB, Uppsala), etc. may be used to label the probe DNA with [α-³²P]dCTP (Amersham), etc. and thus provide a probe with radioactivity.

Phage particles, recombinant plasmids, recombinant vectors and others, containing the target nucleic acids, can be isolated and purified by customary techniques used in the art. For instance, they are obtained by glycerol gradient ultracentrifugation (Molecular Cloning, a laboratory manual, ed. T. Maniatis, Cold Spring Harbor Laboratory, 2nd ed. 78, 1989), electrophoresis and other techniques. DNA can be isolated and purified from phage particles and the like by customary techniques used in the art. For instance, the resulting phages are suspended in TM solution (50mM Tris-HCl buffer, pH7.8, containing 10mM MgSO₄), etc., and treated with DNase I and RNase A, etc. followed by addition of a Proteinase K mixture solution (20mM EDTA, 50 µg/ml Proteinase K and 0.5% SDS). The resultant mixture is incubated at about 65°C for 1 hr., subjected to phenol extraction and then to diethyl ether extraction, followed by precipitation with ethanol to form DNA precipitates. Next, the resultant DNA is washed with 70% ethanol, dried and dissolved in TE solution (10mM Tris-HCl buffer, pH8.0, containing 10mM EDTA). In addition, a large amount of target DNA can be obtained by subcloning, etc. For example, the subcloning can be performed with plasmid vectors, etc. in host E. coli, etc. The DNA thus subcloned can also be isolated and purified by techniques including phenol extraction, ethanol precipitation, etc. in the same manner as aforementioned.

The resultant nucleic acids (including DNA) such as PCR products are typically herein subjected to electrophoresis on 1 to 2% agarose gels. Specific bands are cut out from the gel, and DNA is extracted with a commercially available kit, e.g., Gene clean kit (Bio 101) and the like. The extracted DNA is cleaved with appropriate restriction enzymes and purified if necessary. Further, the 5'-end is, if necessary, phosphorylated with T4 polynucleotide kinase, etc. and subsequently the DNA is ligated into an appropriate plasmid vector including a pUC vector such as pUC18, and transformed into suitable competent cells. The cDNA library can also be constructed based on the produced DNA fragments using phage vectors, plasmid vectors etc. The cloned PCR products are sequenced and analyzed. Commercially available plasmid vectors such as p-Direct (Clontech), pCR-Script™ SK(+) (Stratagene), pGEM-T (Promega), and pAmp™ (Gibco-BRL) are useful for cloning of the PCR products. Transformation of host cells can be carried out by methods known in the art such as the calcium method, the rubidium/calcium method, the calcium/manganese method, the TFB high efficiency method, the FSB frozen competent cell method, the rapid colony method, electroporation and methods substantially equivalent thereto (D. Hanahan, J. Mol. Biol., 166: 557, 1983, etc.). Reverse transcription PCR (polymerase chain reaction coupled reverse transcription; RT-PCR) and RACE (rapid amplification of cDNA ends) can be applied to isolate the target DNA. RACE can be carried out according to the methods, for example, described in M. A. Innis et al. ed., "PCR Protocols" (M. A. Frohman, "a guide to methods and applications"), pp.28-38, Academic Press, New York (1990), etc. DNA can be cloned depending on necessity. Suitable vectors for cloning DNA include plasmids, λ phages, cosmids, P1 phage, F element, YAC and others, and are preferably vectors derived from λ phages, such as Charon 4A, Charon 21A, λ gt10, λ gt11, λ DASXII, λ FIXII, λ EMBL3, and λ ZAPII™ (Stratagene). The resultant DNA fragments can be incorporated into an appropriate vector such as plasmid pEX, pMAMneo, pKG5, and pET3a (Stratagene), as described in detail below, and can be expressed in appropriate host cells, e.g., E. coli, yeast, CHO cells, COS cells and others as described in detail below. The DNA fragments can be introduced into animal cells as intact molecules or appropriate control sequence-added DNA fragments or after incorporated into an appropriate vector. Thus, transgenic animals which express the given gene can be produced. The animals include mammalian animals, and include, for example, mice, rats, rabbits, guinea pigs, cattle etc. Preferably, the transgenic animal can be produced by introducing the DNA fragments into fertilized eggs of an animal such as a mouse. Targeted gene products are verified using suitable animal cells, such as 293T cells and COS-1 cells, transfected with said foreign gene.

The methods for introducing foreign genes into mammal animal cells may be practicable ones known in the art or substantially similar thereto, The method may include, for example, the calcium phosphate method (e.g., F. L. Graham et al., Virology, 52: 456, 1973, etc.), the DEAE-dextran method (e.g., D. Warden et al., J. Gen. Virol., 3: 371, 1968, etc.), electroporation (e.g., E. Neumann et al., EMBO J, 1: 841, 1982, etc.), microinjection, the liposome method, virus infection, the phage particle method and the like. The gene products produced by the animal cells transfected with the given gene in such ways can also be analyzed.

Any plasmid into which the target gene and others (DNA obtainable in the present invention and the like) are incorporated may be used as long as said DNA can be expressed in host cells conventionally used in genetic engineering techniques (such as prokaryotic host cells including Escherichia coli, Bacillus subtilis, etc. and eukaryotic host cells including yeasts, CHO cells, COS cells, and insect host cells such as Sf21. It goes without saying that it is possible to use those selected from attachments in commercially available kits and reagents. In such plasmid sequences, it is possible, for example, to contain modified codons suitable for expressing the cloned DNA in selected host cells or to construct restriction enzyme sites. It is also possible to contain control sequences, enhancer sequences, and other sequences for facilitating the expression of the target gene; linkers, adaptors and others, useful for ligating the target gene; effective sequences useful in controlling resistance to antibiotics or in controlling metabolism or in selection (including those coding for hybrid proteins and fusion proteins); and the like. Preferably, suitable promoters may be used. For example, such promoters may include tryptophan promoter (trp), lactose promoter (lac), tryptophan-lactose promoter (tac), lipoprotein promoter (lpp), λ phage P_{L} promoter, etc. in the case of plasmids where hosts are E. coli; SV40 late promoter, MMTV LTR promoter, RSV LTR promoter, CMV promoter, SRα promoter, etc. in the case of plasmids where hosts are animal cells; and GAL1, GAL10 promoters, etc. in the case of plasmids where hosts are yeast. It is also possible to use regulation systems such as CYCI, HIS3, ADH1, PGK, PHO5, GAPDH, ADC1, TRP1, URA3, LEU2, EN0, TP1, and AOX1.

An enhancer can be inserted into the vector to facilitate the transcription of DNA encoding the desired polypeptide. Such enhancers include elements of approximately 10 to 100 bp, acting on the promoter to facilitate the transcription and typically having a cis action. Many enhancers have been known in mammalian genes such as globin, elastase, albumin, α-fetoprotein, insulin genes and the like. Preferably useful representatives of the enhancers are those obtained from eukaryotic infectious viruses, including, for example, an SV40 enhancer (100-270 bp) located at the late region of the replication origin, a cytomegalovirus enhancer for the early promoter, a polyoma enhancer located at the late region of the replication origin, an adenovirus enhancer and the like. A signal sequence fitting for the host can be added if necessary. Such signal sequences which can be used herein are well known by those skilled in the art.

The plasmids for E. coli hosts include, for example, pBR322, pUC18, pUC19, pUC118, pUC119, pSP64, pSP65, pTZ-18R/-18U, pTZ-19R/-19U, pGEM-3, pGEM-4, pGEM-3Z, pGEM-4Z, pGEM-5Zf(-), pBluescript KS™ (Stratagene) and the like. The plasmid vectors suitable for the expression in E. coli also include pAS, pKK223 (Pharmacia), pMC1403, pMC931, pKC30, pRSET-B (Invitrogen) and the like. The plasmids of which hosts are animal cells include the SV40 vector, polyoma viral vector, vaccinia viral vector, retroviral vector and the like, and include, for example, pcD, pcD-SRα, CDM8, pCEV4, pME18S, pBC12BI, pSG5 (Stratagene) and the like. The plasmids of which hosts are yeast include YIp, YEp, YRp, YCp type vectors and the like, and, for example, pGPD-2 and the like. The host cells which are E. coli include those derived from the E. coli K12 strain and, for example, NM533, XL1-Blue, C600, DH1, DH5, DH11S, DH12S, DH5α, DH10B, HB101, MC1061, JM109, STBL2, etc., and for those derived from the E. coli B834 strain, BL21(DE3)/pLYsS and the like. The host cells which are yeast include, for example, Saccharomyces cerevisiae, Schizosaccharomyces prombe, Pichia pastoris, Kluyveromyces cells, Candida, Trichoderma reesia and the other yeast cells.

The host cells which are animal cells include, for example, African grivet fibroblast-derived COS-7 cells, COS-1 cells, CV-1 cells, human renal cell-derived 293 cells, human epidermal cell-derived A431 cells, human colon cell-derived 205 cells, murine fibroblast-derived COP cells, MOP cells, WOP cells, Chinese hamster cell-derived CHO cells, CHO DHFR cells, human HeLa cells, murine cell-derived C127 cells, murine cell-derived NIH 3T3 cells, murine L cells, 9BHK, HL60, U937, HaK and Jurkat cells, other cell lines obtained by transformation, normal diploid cells, cell lines induced from primary cultured tissue in vitro, and the like. Insect cells include cells using Spodoptera frugiperda (caterpillar), Aedes aegypti (mosquito), Aedes albopictus (mosquito), Drosophila melanogaster (fruitfly), silk worm larva or cultured cells (e.g., BM-N cells) with vectors, silk worm (Bombyx mori) nuclear polyhedrosis virus, those derived therefrom or other suitable ones (for example, Luckow et al., Bio/Technology, 6, 47-55 (1988); Setlow, J. K. et al. (eds.), Genetic Engineering, Vol. B, pp.277-279, Plenum Publishing, 1986; Maeda et al., Nature, 315, pp.592-594 (1985)). With utilizing Agrobacterium tumefaciens etc., it is possible to use plant cells as the host cells, which have been widely known along with vectors suitable therefor in the art. In the gene engineering techniques of the present invention, it is possible to use restriction enzymes, reverse transcriptases known and widely used in the art, DNA-modifying enzymes, DNase, DNA polymerases, terminal nucleotidyltransferases, DNA ligases and the like to modify or convert DNA into a structure suitable for cloning the DNA fragments. For example, restriction enzymes include those described in, for example, R. J. Roberts, Nucleic Acids Res., 13: r165, 1985; S. Linn et al. ed. Nucleases, p. 109, Cold Spring Harbor Lab., Cold Spring Harbor, New York, 1982; R. J. Roberts, D. Macelis, Nucleic Acids Res., 19: Suppl. 2077, 1991, etc.

In accordance with the present invention, if necessary, appropriate selection markers are used to select host cells transformed with the expression vector containing the target polypeptide (protein)-coding polynucleotide. Cloning can be repeated to obtain stable cell clones with high expression levels. For instance, when a dhfr gene is utilized as a selection marker in the transformed host animal cells (transformants or transfectants), cell clones with higher expression levels can be obtained by culturing with a gradual increase in methotrexate (MTX) concentration to amplify the target polypeptide-coding DNA and selecting resistant cells. The transformants or transfectants can be cultured, under conditions wherein the target polypeptide-coding nucleic acids are expressible, to produce and accumulate target products. The transformants (transfectants) can be cultured in media conventionally used in the art. For example, cultivation of the transformant (transfectant) in which the host is a prokaryotic cell such as Escherichia coli and Bacillus subtilis, yeast or the like can be carried out suitably in a liquid culture medium. The culture medium may contain carbon sources, nitrogen sources, minerals, and others, necessary for growing the transformant. The carbon source may include glucose, dextrin, soluble starch, sucrose, etc. The nitrogen source may include organic or inorganic substances such as ammonium salts, nitrates, corn steep liquor, peptone, casein, meat extracts, malt extracts, bean-cakes, potato extracts, etc. Examples of the minerals may include calcium chloride, sodium dihydrogen phosphate, magnesium chloride, calcium carbonate, etc. It may also be supplemented with yeast extracts, vitamins, casamino acids, growth-promoting factors, etc. Depending on necessity, the medium may be supplemented with drugs such as 3β-indolyl acrylic acid in order to improve efficiency of the promoter. It is desired that the pH for culture medium is from about 5 to about 8.

In the case of the Escherichia hosts, the cultivation is carried out usually at about 15 to 45°c for about 3 to 75 hours. As required, aeration and stirring may be applied. In case of the transformants in which the hosts are animal cells, the culture medium used may include MEM medium, RPMI 1640 medium, DMEM medium, and others, which are containing, for example, fetal calf serum at about 5 to 20%. It is preferable that the pH is from about 6 to about 8. The cultivation is usually carried out at about 30 to 40°C for about 15 to 72 hours. As required, aeration and stirring may be optionally applied. Although target gene product-expressing transformants can be used without any isolation/purification, they may be utilized in the form of cell homogenates. The target gene products may be isolated for use. To extract the products from the cultured microorganisms or cells, the microorganisms or cells are collected by known methods after the cultivation, next suspended in a suitable buffer solution, disrupted by sonication, lysozyme digestion and/or freeze-thawing, and other treatments, and crude extracts are then obtained by centrifugation or filtration. Other conventional extraction or isolation methods can be applied. The buffer solution may contain a protein-denaturing agent such as urea or guanidine hydrochloride or a detergent such as Triton X-100 (trade name) and Tween-80 (trade name). In the case where the target products are secreted into culture media, supernatants are separated from the microorganisms or cells with widely known methods after the cultivation is finished and the resulting supernatants are collected.

The culture supernatants thus obtained and target products contained in extracts can be purified by suitable combinations of widely known techniques per se for separation, isolation and purification. Such widely known techniques per se are, for example, salting out such as ammonium sulfate precipitation, etc.; gel filtration on Sephadex™, etc.; ion exchange chromatography using carriers having, for example, a diethylaminoethyl or carboxymethyl group, etc.; hydrophobic chromatography using carriers having, for example, a hydrophobic group such as butyl, octyl, or phenyl, etc.; pigment (or chromophore) gel chromatography; electrophoresis; dialysis; ultrafiltration; affinity chromatography; high performance liquid chromatography; etc. Preferably, the target products can be isolated, separated and purified by polyacrylamide gel electrophoresis, affinity chromatography in which ligands are immobilized. Examples of such techniques also include gelatin-agarose affinity chromatography, heparin-agarose chromatography, etc.

In the polypeptides (proteins) of the present invention, amino acid residues contained therein can be modified by chemical techniques. Also, they can be modified and partially decomposed to make derivatives thereof using enzymes such as peptidases, e.g., pepsin, chymotrypsin, papain, bromelain, endopeptidase, exopeptidase and the like. In the polypeptides of the present invention, the C-terminal end is typically a carboxyl group (-COOH) or a carboxylate (-COO⁻), but the C-terminal end may be an amide form (-CONH₂) or an ester form (-COOR). For said ester, R includes C₁ to C₆ alkyl groups such as methyl, ethyl, n-propyl, isopropyl and n-butyl, C₃ to C₈ cycloalkyl groups such as cyclopentyl and cyclohexyl, C₆ to C₁₂ aryl groups such as phenyl and α-naphthyl, phenyl-C₁ to C₂ alkyl groups such as benzyl and phenethyl, C₇ to C₁₄ aralkyl groups including α-naphthyl-C₁ to C₂ alkyl groups such as α-naphthylmethyl, as well as a pivaloyloxymethyl group widely used as an ester in oral use. When the proteins of the present invention have a carboxyl group (or carboxylate) at the site other than the C-terminal end, those in which the carboxyl group can be amidated or esterified are included in the proteins of the present invention. As the ester in this case, for example, the C-terminal ester and the like described above are used.

The polypeptides (proteins) of the present invention may be those having a methionine residue at N-terminus in the above proteins, and further include those in which an amino group of the methionine residue is protected with a protecting group (for example, C₁ to C₆ acyl groups including C₁ to C₅ alkyl-carbonyl groups such as formyl and acetyl), those in which the N-terminus is cleaved in vivo and the resultant glutamyl group is pyroglutamylated, those in which substituents (for example, -OH, -COOH, amino, imidazole, indole, guanidino groups and the like) on side chains of the intramolecular amino acids are protected with appropriate protecting groups (for example, C₁ to C₆ acyl groups such as formyl and acetyl groups), or conjugated proteins (such as so-called glycoproteins) in which saccharide chains are linked.

Further, by relying on the gene nucleotide sequences associated with the present invention, equivalent polypeptides or derivatives thereof wherein each amino acid sequence of the target polypeptides is altered may be produced with conventional genetic engineering techniques. Such alterations include substitution (replacement), deletion, insertion, transfer or addition of one or more amino acid residues, etc. For example, such mutations, conversions and modifications are those described in The Japanese Biochemical Society (JBS) ed., "Zoku-Seikagaku Jikken Koza 1, Idenshi Kenkyu-Hou II", p. 105 (Susumu Hirose), Tokyo Kagaku Dozin Co. Ltd., Japan, (1986); JBS ed., "Shin-Seikagaku Jikken Koza 2, Kakusan III (Recombinant DNA technique)", p. 233 (Susumu Hirose), Tokyo Kagaku Dozin Co. Ltd., Japan, (1992); R. Wu, L. Grossman, ed., "Methods in Enzymology", Vol. 154, p. 350 & p. 367, Academic Press, New York (1987); R. Wu, L. Grossman, ed., "Methods in Enzymology", Vol. 100, p. 457 & p. 468, Academic Press, New York (1983); J. A. Wells et al., Gene, 34: 315, 1985; T. Grundstroem et al., Nucleic Acids Res., 13: 3305, 1985; J. Taylor et al., Nucleic Acids Res., 13: 8765, 1985; R. Wu ed., "Methods in Enzymology", Vol. 155, p. 568, Academic Press, New York (1987); A. R. Oliphant et al., Gene, 44: 177, 1986, etc. For example, included are methods such as the site-directed mutagenesis (site specific mutagenesis) utilizing synthetic oligonucleotides or others (Zoller et al., Nucl. Acids Res., 10: 6487, 1987; Carter et al., Nucl. Acids Res., 13: 4331, 1986), the cassette mutagenesis (Wells et al., Gene, 34: 315, 1985), restriction selection mutagenesis (Wells et al., Philos. Trans. R. Soc. London Ser A, 317: 415, 1986), the alanine scanning (Cunningham & Wells, Science, 244: 1081-1085, 1989), PCR mutagenesis, Kunkel method, dNTP[ αS] method (Eckstein), the region directed mutagenesis using sulfurous acid and nitrous acid and the like.

And, the polypeptides (proteins) may be expressed as fusion polypeptides (fusion proteins) when produced by gene recombination techniques, and may be converted or processed into those having substantially equivalent biological activity as compared to those which naturally occur in vivo or in vitro. The fusion polypeptide expression system usually used in gene engineering can be applied. Such fusion polypeptides can be purified by an affinity chromatography and the like, taking advantage of their fusion moieties. Such fusion polypeptides include those fused to a histidine tag, or those fused to the amino acid sequence of β-galactosidase (β-gal), maltose-binding protein (MBP), glutathione S-transferase (GST), thioredoxin (TRX), or Cre Recombinase. Similarly, the polypeptide can be added with a tag of heterogeneous epitope, and can be isolated/purified by an immunoaffinity chromatography using an antibody specifically binding to the epitope. In more suitable embodiments, the representatives include poly histidine (poly-His) and polyhistidine-glycine (poly-His-Gly) tags, and epitope tags such as AU5, c-Myc, CruzTag 09, CruzTag 22, CruzTag 41, Glu-Glu, HA, Ha.11, KT3, FLAG (registered trademark, Sigma-Aldrich), Omni-probe, S-probe, T7, Lex A, V5, VP16, GAL4, and VSV-G (Field et al., Molecular and Cellular Biology, 8: pp.2159-2165 (1988); Evan et al., Molecular and Cellular Biology, 5: pp.3610-3616 (1985); Paborsky et al., Protein Engineering, 3(6): pp.547-553 (1990); Hopp et al., BioTechnology, 6: pp.1204-1210 (1988); Martin et al., Science, 255: pp.192-194 (1992); Skinner et al., J. Biol. Chem., 266: pp.15163-15166 (1991); Lutz-Freyermuth et al., Proc. Natl. Acad. Sci. USA, 87: pp.6393-6397 (1990), etc.). Yeast two-hybrid systems are also utilizable.

Besides, the fusion polypeptides can be those tagged with a marker such that they become detectable proteins. In more suitable embodiments, the detectable markers may be Biotin-Avi Tag which is a biotin/streptavidin system, and fluorescent substances. The fluorescent substances include green fluorescent proteins (GFP) derived from luminescent jelly fish such as Aequorea victorea and the like, modified variants thereof (GFP variants) such as EGFP (enhanced-humanized GFP) and rsGFP (red-shift GFP), yellow fluorescent proteins (YFP), green fluorescent proteins (GFP), cyan fluorescent proteins (CFP), blue fluorescent proteins (BFP), GFP derived from Renilla reniformis, and the like (Atsushi Miyawaki ed., Jikken Igaku (Experimental Medicine), Besatsu (suppl.), Postgenome Jidai no Jikken Kouza 3 (GFP and Bioimaging), Yodosha Co., Ltd., 2000). Also, detection can be carried out using antibodies (including monoclonal antibodies and fragments thereof) which specifically recognize the above fusion tag. The expression and purification of such fusion polypeptides can be performed using commercially available kits suitable for these techniques, and can also be carried out according to protocols as instructed by manufacturers or distributors of the kits. The resultant proteins (which may include peptides and polypeptides) can be coupled with suitable carrier or solid phases by techniques known in the enzyme immunoassay and others to form solid phased products. Solid-phased proteins and solid-phased peptides are conveniently useful in binding assays and screenings for substances.

Modifications and alterations of the polypeptide or protein structures can be performed in reference to, for example, The Japanese Biochemical Society (JBS) ed., "Shin-Seikagaku Jikken Koza 1, Protein VII, Protein Engineering" Tokyo Kagaku Dozin Co. Ltd., Japan, 1993) using the methods described therein or the methods described in the references quoted therein, and, further, the methods substantially equivalent thereto. Their biological activities as described herein below may include an immunological activity, for example, an antigenicity. The modification and alteration may be deamination, hydroxylation, carboxylation, phosphorylation, sulfation, alkylation such as methylation, acylation such as acetylation, esterification, amidation, ring-opening, cyclization, glycosylation, alteration of contained saccharide chains to different types, increasing or decreasing the number of contained saccharide chains, lipid-binding, substitution to D-amino acid residues, etc. Those methods are known in the art (for example, T. E. Creighton, Proteins: Structure and Molecular Properties, pp.79-86, W.H. Freeman & Co., San Francisco, USA (1983), etc.).

The human-derived peptides or polypeptides (or proteins) may be those of which one or more amino acid residues are different in terms of identity from those which naturally occur, and those of which sites of one or more amino acid residues are different from those which naturally occur. The human-derived peptides include deletion analogs with amino acid deletions of one or more (for example, from 1 to 80, preferably from 1 to 60, still preferably from 1 to 40, more preferably from 1 to 20, and especially from 1 to 10) amino acid residues characteristic of human galectin 9 proteins (including Gal-9L, -9M, and -9S), substitution analogs where one or more (for example, from 1 to 80, preferably from 1 to 60, still preferably from 1 to 40, more preferably from 1 to 20, and especially from 1 to 10) amino acid residues characteristic of said human galectin 9 are substituted with other residues, and addition analogs with amino acid additions (or insertions) of one or more (for example, from 1 to 80, preferably from 1 to 60, still preferably from 1 to 40, more preferably from 1 to 20, and especially from 1 to 10) amino acid residues.

The mutants as aforementioned are all included in the present invention as long as they retain the domain structure or active carbohydrate binding structure characteristic of native human galectin 9. Also, it is thought that the peptides or polypeptides may include those having all or part of substantially equivalent primary structure conformations to those of native galectin 9 proteins. Furthermore, it is also thought that the peptides or polypeptides may include those having substantially equivalent biological activity as compared to said native galectin 9 proteins. Moreover, they can be one of the mutants which naturally occur. The human-derived proteins (or peptides or polypeptides) include, for example, those having an amino acid sequence with more than 60%, and in some cases more than 70% homology for an amino acid sequence selected from SEQ ID NOs: 1 to 3 in the Sequence Listing of WO 02/37114 A1, and more preferably those having an 80 or 90% or more homologous amino acid sequence to any amino acid sequence of said SEQ ID NOs: 1 to 3. The peptide fragments (partial peptides) derived from the human-derived protein may be any as long as they are part of said human-derived proteins (that is, partial peptides or fragmented peptides of said proteins) and have substantially equivalent activity to the galectin 9 protein. For example, the partial peptides (or peptide fragments) of the protein of the present invention include peptides having a sequence with at least 5 or more, preferably 20 or more, still preferably 50 or more, more preferably 70 or more, still more preferably 100 or more, and, in some cases, 200 or more amino acid residues contained in the galectin 9-constituent amino acid sequence, preferably wherein said amino acid residues are contiguous. Preferable examples of them are those having the same homology as aforementioned, with respect to homology to the region corresponding to any amino acid sequence of SEQ ID NOs:1 to 3 in the Sequence Listing of WO 02/37114 A1.

The term "substantially equivalent" used herein means that the activity, for example, the cytotoxic, apoptosis-inducing, anti-inflammatory, anti-allergic, immunosuppressive, saccharide chain-binding, physiological or biological activity owned by proteins of interest is substantially equivalent. Further, the meanings of that term may include a case having the substantially same quality of activity. The substantially same quality of activity can include, for example, a binding activity, a cytotoxity, an apoptosis-inducing activity, etc. The substantially same quality of activity indicates that these activities are qualitatively homogenous, and, for example, that they are physiologically, pharmacologically or biologically homogenous. For instance, it is preferred that the activities such as the binding activity, the cytotoxity and the apoptosis-inducing activity are equivalent (for example, from about 0.001 to 1000 fold, preferably from about 0.01 to 100 fold, more preferably from about 0.1 to 20 fold, and still preferably from about 0.5 to 2 fold), but quantitative elements such as the extents of these activities, molecular weights of the proteins etc. may be different. Next, the substitution, deletion or insertion (addition) of amino acids does not often cause a great alteration in physiological or chemical properties of a polypeptide. In some cases, a desirable modification will be provided. In such a case, a polypeptide with substitution, deletion or insertion will be considered to be substantially identical to a polypeptide without such substitution, deletion or insertion. Substantially identical substituents of amino acids in the amino acid sequence can be selected from other amino acids in the class to which the amino acid belongs. For instance, non-polar (hydrophobic) amino acids include alanine, phenylalanine, leucine, isoleucine, valine, proline, tryptophan, methionine and the like; polar (neutral) amino acids include glycine, serine, threonine, cysteine, tyrosine, asparagine, glutamine and the like; amino acids having a positive charge (basic amino acids) include arginine, lysine, histidine and the like; and amino acids having a negative charge (acidic amino acids) include aspartic acid, glutamic acid and the like.

The methods known in the peptide synthetic art, for example, chemical synthetic methods such as liquid phase synthetic methods, and solid phase synthetic methods can be used for the synthesis of the proteins and peptide fragments thereof according to the present invention. In such methods, using a resin for synthesis of proteins or peptides, an appropriately protected amino acid is sequentially attached to the desired amino acid sequence on the resin by various condensation methods as known in the art. Various activating reagents as known in the art are preferably used for the condensation reactions. For example, carbodiimides such as dicyclohexylcarbodiimide can be preferably used as such reagents. A target reagent can be obtained by suitably removing a protecting group when a product has the protecting group.

In cases where the peptide (or polypeptide) is in a free form, the free peptide (or polypeptide) can be converted into a salt thereof by known methods per se or methods analogous thereto. In case where the peptide (or polypeptide) is in a salt form vice versa, the peptide salt (or the polypeptide salt) can be converted into a free form or into any other salt thereof by known methods per se or methods analogous thereto. The salts of said peptide and polypeptide according to the present invention preferably include physiologically or pharmaceutically acceptable salts but are not limited to. Examples of such salts are salts thereof with inorganic acids (e.g. hydrochloric acid, hydrobromic acid, sulfuric acid, nitric acid, phosphoric acid, etc.), salts thereof with organic acids (e.g. acetic acid, formic acid, maleic acid, fumaric acid, succinic acid, citric acid, tartaric acid, malic acid, benzoic acid, methanesulfonic acid, p-toluenesulfonic acid, benzenesulfonic acid, etc.), etc. In addition, examples of such salts are ammonium salts thereof, salts thereof with organic bases such as ethylamine, dimethylamine, trimethylamine and hydroxyethylamine.

Phenomena associated with the disclosed biological activities exerted by galectin 9 are detectable via detecting/measuring the galectin 9-expressing genes (including DNA such as cDNA and RNA such as mRNA) according to the aforementioned "gene recombination techniques", by the known techniques for detecting/measuring the expression of a specific gene in the art, such as in situ hybridization, Northern blotting, dot blotting, RNase protection assay, RT-PCR, Real-Time PCR (Journal of Molecular Endocrinology, 25, 169-193(2000) and reference documents quoted therein), and DNA array analysis ((Mark Shena (Ed.), "Microarray Biochip Technology", Eaton Publishing (March, 2000)). Galectin 9 expressing gene assay systems, and reagents, methods or processes for their applications, utilizing these techniques are all encompassed in the present inventive techniques and application systems therefor. The in situ hybridization may include, for example, non-RI in situ hybridization, and may also include, for example, direct and indirect methods. The direct method is based on, for example, direct labels where a detectable molecule (reporter) is directly bound to a nucleic acid probe, whereas the indirect method is based on, for example, indirect ones where a signal is amplified using an antibody against a reporter molecule. Functional groups (e.g., primary aliphatic amino groups, SH groups, etc.) are incorporated into oligonucleotides in the nucleic acid probe, and may be coupled with haptens, fluorescent dyes, enzymes and the like. Representatives of labels for the nucleic acid probes include digoxigenin (DIG), biotin, fluorescein and the like. The labels as used herein can be suitably selected from those described in connection with antibodies as disclosed herein below. Multiple labeling can also be utilized. Further labeled antibodies can also be utilized. Applicable methods of preparing labeled nucleic acid probes are suitably selected from those techniques known in the art, but include, for example, random prime labeling, nick translation, , PCR-mediated DNA amplification, labeling/tailing, in vitro transcription, etc. The treated samples can be observed using techniques suitably selected from those known in the art. Examples of such techniques may include dark-field microscopy, phase-contrast microscopy, reflection-contrast microscopy, fluorescent microscopy, digital imaging microscopy, electron microscopy and the like. Furthermore, flow cytometry can be used.

The malignant cell as used herein may encompass metastatic tumor cells. A tumor that may metastasize to several sites is a malignant neoplasm, and the term "malignant neoplasm" is generally referred to as being epithelial or non-epithelial and may be distinguished as being cancer, sarcoma, or leukemia, etc. Among the general public, when the neoplasm or tumor is simply called "cancer", it refers to a malignant neoplasm or tumor. As used herein, the term "cancer" is employed in the broadest sense and should not be interpreted as being just an epithelial malignant neoplasm. The term "cancer" used herein may cover epithelial malignant tumors and non-epithelial malignant tumors (including those that are tumorigenic and non-tumorigenic), such as skin cancers (which may include melanomas), mammary cancers, ovarian cancers, uterine cancers, malignant testicular tumors, prostatic cancers, urinary bladder cancers, renal cancers, thyroid cancers, pharyngeal/larynx cancers, lingual cancers, maxillary cancers, esophageal cancers, stomach cancers, colon/rectal cancers, lung/bronchial cancers, liver cancers (including liver cell cancers and intrahepatic bile duct cancers), extrahepatic bile duct/gall bladder cancers, pancreatic cancers, leukemia, malignant lymphoma, plasmacytoma, osteosarcoma, chondrosarcoma, leiomyosarcoma, rhabdomyosarcoma, liposarcoma, fibrosarcoma, malignant hemangioma, malignant hemangioendothelioma, brain tumors (including meningioma, glyoma, astrocytoma), etc., but is not restricted to these. It shall be understood as not being restricted to particular species as long as they become targets disclosed or uncovered herein when the activities owned by galectin 9 are utilized or applied.

When functions executed by Gal-9 are utilized, screening can be done for compounds, or salts thereof, which promote (agonists) or inhibit (antagonists) the interesting Gal 9-mediated functions such as biological actions (e.g., cytotoxic actions, apoptosis-inducible actions, glucocorticoid-like actions, malignant cell metastasis-inhibiting actions and the like). This means that screening kits and reagents are contemplated herein. Thus, the present invention provides methods of screening for either (1) a promoting compound (agonist), or a salt thereof, which promotes the determined functions exerted by any of galectin 9 proteins (including human galectin 9), peptide fragments thereof, and salts thereof, etc., wherein the function may include Gal 9-mediated biological actions as identified or verified herein, or (2) an inhibitory compound (antagonist), or a salt thereof, which inhibits the same function, which comprises using a disclosed or identified action or activity mediated or owned by a member selected from the group consisting of said galectin 9 proteins (including human galectin 9), peptide fragments thereof, and salts thereof, in connection with a variety of substances.

For example, the screening comprises
(i) contacting galectin-9 protein, a peptide fragment thereof, a salt thereof or the like (wherein a transformant or transfectant which expresses said protein may be included; it has hereinafter the same meaning) with a suitable test sample, thereby obtaining a first assay;
(ii) incubating the protein of the present invention, a peptide fragment thereof, a salt thereof, or the like, without the test sample of interest, thereby obtaining a second assay; and
(iii) comparing said first assay and said second assay.

In an embodiment of the screening, said biological activities (e.g., activities associated with interactions between each galectin 9 protein and biological components, etc.) are measure and compared.

The screening systems may contain suitable detection substrates for the convenience of assays. The substrates may be any as long as they are effectively utilizable in assays. For instance, they can be selected from those known to be conventional substrates and preferably include synthesized compounds and other materials. The substrate can be employed without any modification, or preferably after labeling with fluorochromes such as fluorescein, enzymes or radioactive substances.

The test samples include, for example, proteins, peptides, nonpeptide compounds, synthetic compounds, fermented products, plant extracts, tissue extracts such as animal tissue extracts, cell extracts, etc. Examples of test compounds as used for the test samples may include preferably anti-galectin antibodies, enzyme inhibitors, cytokines, a variety of compounds having inhibitor activity, in particular synthetic compounds, etc. These compounds can be novel or known to the public. The screening is conducted according to conventional techniques for measuring binding activities or enzyme activities, for example, by referring to known methods in the art. It can also be performed by using various labels, buffers and suitable other reagents, etc. and according to the operations, etc., as described herein for the assays. In the screening, it is possible to treat the proteins used and the like with activators, and to convert their precursors or latent forms into active forms thereof prior to the assay. The assay can usually be performed in buffer without any adverse effect on the reaction, including Tris-HCl buffer, phosphate buffer, etc., for example, at pH about 4 to 10, preferably at pH about 6 to 8. For each of these screenings, by giving technical consideration ordinarily owned by persons skilled in the art to customary conditions and operations for each method, suitable assay systems may be constructed in connection with each of galectin 9 proteins and polypeptides or peptides having substantially equivalent activity thereto, according to the present invention. With details of those conventional techniques, a variety of reviews, reference books, etc. may be referred to (e.g., Methods in Enzymology, Academic Press, New York, USA). For apoptosis assays, it is possible to refer to Sei-ichi Tamuma (Ed.), "Saiboukogaku Bessatsu: Jikken Protocol Series, Apoptosis Jikken Protocol" (1st Edition, 2nd Print), Shujunsha Co., Ltd., January 20, 1995 and others, and to use commercially available assay kits.

The compounds or salts thereof identified or obtained by the screening method or kit according to the present invention are those selected from the aforementioned test compounds, including peptides, proteins, nonpeptide compounds, synthetic compounds, fermented products, cell extracts, plant extracts, animal tissue extracts, etc. Such compounds are those which enhance (or promote) or inhibit (or suppress) the functions of the proteins and other species according to the present invention. Salts of said compounds are, for example, pharmaceutically acceptable salts thereof, etc. Examples of such salts are those of inorganic bases, of organic bases, of inorganic acids, of organic acids, of basic or acidic amino acids, etc. Preferred examples of the inorganic base salts are alkaline metal salts such as sodium salts, and potassium salts; alkaline earth metal salts such as calcium salts and magnesium salts; aluminum salts, ammonium salts; etc. Preferred examples of the organic base salts are salts with trimethylamine, triethylamine, pyridine, picoline, 2,6-lutidine, ethanolamine, diethanolamine, triethanolamine, cyclohexylamine, dicyclohexylamine, N,N-dibenzylethylene-diamine, etc. Preferred examples of the inorganic acid salts are salts with hydrochloric acid, hydrobromic acid, sulfuric acid, phosphoric acid, etc. Preferred examples of the organic acid salts are salts with formic acid, acetic acid, propionic acid, fumaric acid, oxalic acid, tartaric acid, maleic acid, citric acid, succinic acid, malic acid, methanesulfonic acid, benzenesulfonic acid, benzoic acid, etc. Preferred examples of the basic amino acid salts are those of arginine, lysine, ornithine, etc. Preferred examples of the acidic amino acid salts are those of aspartic acid, glutamic acid, etc.

Herein, the anti-galectin-9 antibody (Ab) may be obtained as polyclonal or monoclonal Ab.

As used herein, the term "antibody" can be used in the broadest sense and may cover a single species of desirable monoclonal antibodies against galectin-9 proteins, galectin 9-constituent polypeptides and related peptide fragments; antibody compositions (or mixtures) having a specificity to various epitopes thereof; further monovalent or polyvalent antibodies and polyclonal and monoclonal antibodies, and also those which are intact molecules or fragments and derivatives thereof, including F(ab')₂, Fab' and Fab fragments; and also chimeric antibodies, hybrid antibodies each having at least two antigen or epitope binding sites, or bispecific recombinant antibodies (e.g., quadromes, triomes, etc.), interspecies hybrid antibodies, anti-idiotypic antibodies and those which have been chemically modified or processed and must be regarded as derivatives of these antibodies and further which may be produced either by adopting cell fusion or hybridoma techniques or antibody engineering or by using synthetical or semisynthetical techniques in a known manner, which may be prepared either by the known conventional methods in view of antibody production or by recombinant DNA techniques, and which have neutralizing or binding properties with respect to the target antigen substances or target epitopes described and defined herein. Preferably the inventive antibodies are especially those capable of specifically recognizing a polypeptide selected from the group consisting of intact (naturally-occurring type) galectin-9 medium isoform (or medium type galectin-9: Gal-9M) polypeptides and intact (naturally-occurring type) galectin-9 long isoform (or long type galectin-9: Gal-9L) polypeptides. Examples of such antibodies are capable of distinguishing Gal-9M or -9L polypeptides from galectin-9 short isoform (or short type galectin-9: Gal-9S) polypeptides.

In order to obtain a polyclonal anti-galectin-9 antibody, a mammal, bird, etc. is immunized with an immunogen, galectin-9, or a fragment thereof, that is, a peptide that is part of the galectin-9 sequence, and an antiserum is collected from the immunized mammal, bird, etc. The polyclonal antibodies contained in this antiserum may then be used.

Though a mammal or bird for immunization with the sensitizing antigen, galectin-9, is not restricted to particular species, but includes, in general, rodents, such as mouse, rat, hamster, etc.; rabbit, lamb, goat, cattle, horse, pig, dog, cat; monkey or other primates; and birds, such as chicken. In some cases, it is preferable to select the animal in consideration of the compatibility with the parent cell to be used for cell fusion.

The immunization of animals with the sensitizing antigen is carried out according to known methods. For example, as a general method, the sensitizing antigen is injected peritoneally or subcutaneously into a mammal, etc. A suitable carrier may also be used for immunization with the sensitizing antigen.

The immunized animal is kept for a predetermined period and the antiserum containing the polyclonal antibody may then be prepared from blood collected from the animal. The antiserum thus obtained is used as an active target component according to the present invention after checking that the antiserum specifically recognizes galectin-9.

First, galectin-9 used as the sensitizing antigen for acquiring the antibodies may be obtained by expression of a known galectin-9 gene/galectin-9 amino acid sequence (the amino acid sequences for galectin 9 are disclosed in WO 02/37114 A1). Briefly, after a gene sequence encoding galectin-9 or a partial domain thereof, a partial protein or peptide fragment of galectin-9, or a peptide having a partial amino acid sequence corresponding to the amino acid sequence of galectin-9, is inserted into a known expression vector with which a suitable host cell is transformed or transfected, and targeted galectin-9 proteins or a partial domain protein thereof, partial protein or polypeptide fragment of galectin-9, or a peptide having the partial amino acid sequence corresponding to the amino acid sequence of galectin-9 is isolated and/or purified from the transformed or transfected host cell or its culture medium with known methods.

As used herein, the anti-galectin-9 antibody may include mammal-derived monoclonal antibodies.

Monoclonal antibodies prepared against antigenic substances are produced by any method capable of providing production of antibody molecules by a series of cell lines in culture. The modifier "monoclonal" indicates the character of the antibody as being obtained from a substantially homogeneous population of antibodies, and is not to be construed as requiring production of the antibody by any particular method. The individual antibodies are those containing a population of identical antibodies except for possible naturally occurring mutations that may be present in minor amounts. Monoclonal antibodies are highly specific, being directed against a single antigenic site. In contrast to conventional (polyclonal) antibody preparations which typically include different antibodies directed against different determinants (epitopes), each monoclonal antibody is directed against a single determinant on the antigen. In addition to their specificity, the monoclonal antibodies are advantageous in that they are synthesized by the hybridoma culture, uncontaminated by other immunoglobulins. The monoclonal antibodies include hybrid and recombinant antibodies. They are obtainable by substituting a constant domain of an antibody for a variable domain (e.g., humanized antibodies), or a heavy chain for a light chain, by substituting a chain from one species with a chain from another species, or by fusing to heterogeneous proteins, regardless of species of origin or immunoglobulin class or subclass designation, so long as they exhibit the desired biological activity (e.g., U.S. Pat. No. 4,816,567; Monoclonal Antibody Production Techniques and Applications, pp.79-97, Marcel Dekker, Inc., New York, 1987; etc.).

Preferable techniques for producing monoclonal antibodies include, for example, the methods using hybridoma cells (G. Kohler and C. Milstein, Nature, 256, pp.495-497 (1975)); the methods using human B cell hybridomas (Kozbor et al., Immunology Today, 4, pp.72-79 (1983); Kozbor, J. Immunol., 133, pp.3001 (1984); Brodeur et al., Monoclonal Antibody Production Techniques and Applications, pp.51-63, Marcel Dekker, Inc., New York (1987) ; triome methods; EBV-hybridoma methods (Cole et al., Monoclonal Antibodies and Cancer Therapy, Alan R. Liss, Inc., pp.77-96 (1985))(techniques for production of human monoclonal antibodies); U.S. Pat. No. 4,946,778 (techniques for production of single-chain antibodies), as well as the following documents in connection with antibodies: S. Biocca et al., EMBO J, 9, pp.101-108 (1990); R.E. Bird et al., Science, 242, pp.423-426 (1988); M.A. Boss et al., Nucl. Acids Res., 12, pp.3791-3806 (1984); J. Bukovsky et al., Hybridoma, 6, pp.219-228 (1987); M. DAINO et al., Anal. Biochem., 166, pp.223-229 (1987); J.S. Huston et al., Proc. Natl. Acad. Sci. USA, 85, pp.5879-5883 (1988); P.T. Jones et al., Nature, 321, pp.522-525 (1986); J.J. Langone et al. (ed.), "Methods in Enzymology", Vol. 121 (Immunochemical Techniques, Part I: Hybridoma Technology and Monoclonal Antibodies), Academic Press, New York (1986); S. Morrison et al., Proc. Natl. Acad. Sci. USA, 81, pp.6851-6855 (1984); V.T. Oi et al., BioTechniques, 4, pp.214-221 (1986); L. Riechmann et al., Nature, 332, pp.323-327 (1988); A. Tramontano et al., Proc. Natl. Acad. Sci. USA, 83, pp.6736-6740 (1986); C. Wood et al., Nature, 314, pp.446-449 (1985); Nature, 314, pp.452-454 (1985) or documents quoted therein (the disclosures of which are incorporated herein by reference).

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they have the desirable biological activity (U.S. Pat. No. 4,816,567; Morrison et al., Proc. Natl. Acad. Sci. USA, 81, pp. 6851-6855 (1984)).

The monoclonal antibodies according to the present invention may be produced from mammalian origin-derived hybridomas or may be produced from hosts that have been transformed with expression vectors containing an antibody gene according to genetic engineering techniques.

Monoclonal antibody-producing hybridomas which produce anti-galectin 9 Abs can be produced with cell fusion techniques utilizing myeloma cells according to the following steps:

Briefly, such hybridomas may be prepared by performing immunization using galectin-9 or a fragment thereof as the sensitizing agent in accordance with a conventional immunization, fusing the resultant immune cells with known parent cells in accordance with a conventional cell fusion, and screening for monoclonal antibody producing cells by a conventional screening method.

The method of preparing galectin-9 or fragments thereof, the method of immunizing mammals, etc. may be carried out in accordance with the above-described techniques for preparing antisera containing polyclonal antibodies. In particular in this case, after immunization of mammals, immune cells are taken out from mammals wherein an increased serum level of desired antibodies has been confirmed and subjected to cell fusion. Particularly preferable immune cells are spleen cells.

Mammal myeloma cells are used as the other parent cells to be fused with the abovementioned immune cells. Any of various known cell lines may be used for the myeloma cells. The cell fusion between the immune cells and myeloma cells may basically be carried out in accordance with known methods, such as Kohler and Milstein's method (Kohler, G. and Milstein, C., Methods Enzymol. (1981) 73, 3-46), etc.

Described herein below is the production of antibodies, including embodiments of monoclonal antibodies.

The monoclonal antibody according to the present invention may be a product obtained by utilizing cell fusion techniques with myeloma cells. The monoclonal antibody can be produced, for example, by the following processes:
(1) preparation of immunogenic antigens; (2) immunization of animals with immunogenic antigens; (3) preparation of myeloma cells; (4) cell fusion between antibody-producing cells and myeloma cells; (5) selection and cloning of hybridomas (hybrid cells), and (6) production of monoclonal antibodies.

### (1) The preparation of immunogenic antigens may be carried out as follows:

The antigen as used herein includes, as disclosed above, isolated native galectin-9 polypeptides or fragments derived therefrom (which include part of such proteins, i.e., domain polypeptides, link polypeptides, fragments, partial peptides, and synthetic polypeptides). The antigen also includes a suitable oligopeptide chemically synthesized based on information on the determined amino acid sequences of galectin-9. Representative examples thereof are peptides having at least five continuous amino acids selected from the amino acid residues existing in a region selected from the group consisting of (1) amino acid sequences of SEQ ID NO: 1 and 2 in the Sequence Listing of WO 02/37114 A1 or amino acid sequences of constituent domains thereof (part of such sequences); (2) amino acid sequences of constituent domains for SEQ ID NO: 3 in WO 02/37114 Al; (3) amino acid sequences of SEQ ID NO: 4, 5, 7, 8, and 9 in the Sequence Listing of WO 02/37114 Al; (4) among SEQ ID NO: 1 in the Sequence Listing of WO 02/37114 A1, constituent amino acid sequences for the C-terminal domain following the amino acid sequence corresponding to SEQ ID NO: 4 or partial fragments thereof; and (5) among SEQ ID NO: 1 in the Sequence Listing of WO 02/37114 A1, constituent amino acid sequences for the N-terminal domain preceding the amino acid sequence corresponding to SEQ ID NO: 4 or partial fragments thereof.

Although the antigen may be used to immunize animals after being mixed with a suitable adjuvant without any modifications, it can be used after formation of immunogenic conjugates. For instance, the antigen for such an immunogen may be selected from fragmented molecules derived from galectin-9 proteins, synthetic polypeptide fragments which are prepared via selecting characteristic sequence areas based on the Gal-9 amino acid sequences followed by design and chemical synthesis. The fragments may be coupled with various carrier proteins via suitable coupling agents to form immunogenic conjugates such as hapten-proteins. The immunogenic conjugates can be used to design monoclonal antibodies that can react with (or recognize) specific sequences exclusively. A cysteine residue or others can be added to the polypeptide thus designed so as to prepare an immunogenic conjugate easily. To couple with a carrier protein or the like, the carrier protein is first activated. This activation may include incorporation of an activated binding group thereinto, etc. The activated binding groups include (1) active ester or active carboxyl groups such as a nitrophenyl ester group, a pentafluorophenyl ester group, a 1-benzotriazol ester group, and an N-succinimido ester group; (2) active dithio groups such as a 2-pyridyldithio group, etc. The carrier proteins include keyhole limpet haemocyanin (KLH), bovine serum albumin (BSA), ovalbumin, globulin, polypeptides such as polylysine, and bacterial components such as BCG.

### (2) The immunization of animals with immunogenic antigens may be carried out as follows:

Animals can be immunized by methods known to those skilled in the art and according to techniques as described in, for example, Shigeru Muramatsu et al. ed., "Jikken-seibutsu-gaku-koza 14, Men-eki-seibutsu-gaku", Maruzen Co. Ltd., Japan, (1985); The Japanese Biochemical Society (Ed.), "Zoku-seikagaku-jikken-kouza 5, Men-ekiseikagaku-kenkyuho", Tokyo Kagaku Dojin Co. Ltd., Japan (1986); The Japanese Biochemical Society (Ed.), "Shin-seikagaku-jikken-kouza 12, Bunshi-men-eki-gaku III (Kougenkoutai-hotai)", Tokyo Kagaku Dojin Co. Ltd., Japan (1992); etc., the disclosures of which are hereby incorporated by reference. Immunization can be performed in a mammal, for example, by one or more injections of an immunizing agent (and, if desired, together with an adjuvant). Typically, the immunizing agent and/or adjuvant will be injected in the mammal by multiple subcutaneous or intraperitoneal injections. The immunizing agent may include the aforementioned antigen peptides or related peptide fragments. It may be useful to conjugate the immunizing agent to a protein known to be immunogenic in the mammal being immunized. Examples of such immunogenic proteins which may be employed include the aforementioned carrier proteins. The adjuvant to be used with the antigen includes Freund's complete adjuvant, Ribi adjuvant, pertussis vaccine, BCG, lipid A, liposome, aluminum hydroxide, silica, etc. The Immunization is carried out with suitable animals, including mice such as BALB/c, hamsters, and others. The antigen dose is, for example, about 1 to 400 µg/animal for mice. Generally, the antigen is injected intraperitoneally or subcutaneously into a host animal, followed by additional immunization by repeated courses wherein intraperitoneal, subcutaneous, intravenous or intramuscular administrations are carried out approximately 2 to 10 times at 1- to 4-week intervals, preferably 1- to 2-week intervals. For immunization, BALB/c mice, as well as F1 mice between BALB/c mice and other mice, etc. can be used. As required, the levels of animal immunization can be assessed by constructing an antibody titer measuring system and measuring the titer of an antibody. The antibody of the present invention may include those obtainable from such immunized animals, for example, anti-serum, polyclonal antibodies, etc.

### (3) The preparation of myeloma cells may be carried out as follows:

Immortal cell lines (tumor cell lines) to be used for cell fusion can be selected from non-immunoglobulin-producing cell lines. The cell lines to be used for cell fusion may include, for example, P3-NS-1-Ag4-1 (NS-1, Eur. J. Immunol., 6: 511-519, 1976), SP-2/0-Ag14 (SP-2, Nature, 276: 269 to 270, 1978), mouse myeloma MOPC-21 cell line-derived P3-X63-Ag8-U1 (P3U1, Curr. topics Microbiol. Immunol., 81: 1-7, 1978), P3-X63-Ag8 (X63, Nature, 256: 495-497, 1975), P3-X63-Ag8-653 (653, J. Immunol., 123: 1548-1550, 1979), etc. 8-Azaguanine resistant mouse myeloma cell lines can be sub-cultured in a cell culture medium, such as Dulbecco's modified Eagle's medium (DMEM) and RPMI-1640, supplemented with antibiotics such as penicillin and amikacin, fatal calf serum (FCS) or others and 8-azaguanine (for example, 5 to 45µg/ml). The specified number of cell lines can be prepared by passage the normal medium two to five days prior to cell fusion. The frozen and preserved cell lines to be used may be completely thawed at about 37°C, and washed on the normal medium such as RPMI-1640 three or more times, followed by culturing on the normal medium to give the specified number of cell lines.

### (4) The cell fusion between antibody-producing cells and myeloma cells may be carried out as follows:

After animals such as mice are immunized according to the above step (2), their spleens are taken out in two to five days from final immunization, and the spleen cell suspension is obtained. Besides the spleen cells, lymph node cells at various sites of organisms can be obtained and used for cell fusion. To be more specific, the cell fusion is carried out in a normal nutrient culture medium, for example, in the presence of a cell fusion accelerator. The culture medium used for the aforementioned cell fusion include, for example, RPMI 1640 medium and MEM, favorable for the growth of the aforementioned myeloma cell lines, and other normal cell media used in this cell culture. Furthermore, serum supplements, such as fetal calf serum (FCS), may be used in combination. The spleen cell suspension thus obtained and the myeloma cell lines obtained by the above step (3) are placed in a medium such as minimum essential medium (MEM), DMEM and RPMI-1640 medium, followed by addition of a fusogen, such as polyethylene glycol (PEG). A widely-known fusogen can be used, including inactivated HVJ (Hemagglutinating virus of Japan, "Sendai virus") and the like. Preferably, 0.5 to 2 ml of 30 to 60% PEG can be added. PEG with molecular weights from 1,000 to 8,000 can be employed, more preferably, PEG with molecular weights from 1,000 to 4,000. The preferred concentration of PEG in the fusion medium is from 30 to 60%. As required, a small amount of dimethyl sulfoxide or the like is added to promote fusion efficiency. The ratio of immune cells to myeloma cells (spleen cell or lymphocyte : myeloma cell line ratio) to be used for fusion may be set as suited, and is, for example, preferably 1:1 to 20:1, and more preferably falls within 4:1 to 10:1.

For cell fusion, predetermined amounts of immune cells and myeloma cells are mixed well in a culture medium, into which a solution of PEG (for example, with an average molecular weight of approximately 1,000 to 6,000) preheated to approximately 37°C is added normally at a concentration of 30 to 60% (w/v). The resultant cell mixture was mixed to form targeted fused cells (hybridomas). Thereafter, the cell fusion agent and others, unfavorable for hybridoma growth, are removed by repeated operations of successively adding suitable culture media, centrifuging, and separating the supernatant. The fusion reaction is conducted for 1 to 10 min, prior to the addition of a cell culture medium such as RPMI-1640 medium. Fusion reaction can be done several times. After fusion reaction, cells are separated by centrifugation and the like, and then transferred to a selection medium.

### (5) The selection and cloning of hybridomas (hybrid cells) may be carried out as follows:

The selection media include conventionally known "HAT medium", i.e., FCS-containing MEM, RPMI-1640 medium, 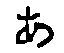 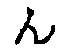 and other media, which are supplemented with hypoxanthine, aminopterin, and thymidine. Culturing in this HAT medium is continued for a period (normally a few days to several weeks) adequate for the dying out of cells (non-fused cells), exclusive of targeted hybridomas. The replacement method for the selection medium is to replenish a volume equivalent to the capacity dispensed to the medium plate on the following day, after which the medium is replaced by half an amount with HAT medium every one to three days. The replacement can be modified depending on situations. Eight to sixteen days after fusion, the medium may be exchanged every 1 to 4 days with conventionally known "HT medium" wherein aminopterin is excluded. As a feeder cell, for example, mouse thymocyte can be used, which is sometimes effective.

The supernatant of a culture well with vigorously growing hybridomas is screened with an assay system such as radioimmunoassay (RIA), enzyme-linked immunosorbent assay (ELISA), fluoroimmunoassay (FIA), luminescence immunoassay (LIA), and Western blotting, or with a fluorescence activated cell sorter (FACS), etc. using a predetermined peptide fragment as an antigen or a labeled anti-mouse antibody to measure target antibodies. The target antibody-producing hybridoma is then cloned. Cloning is carried out by picking up colonies in an agar medium or by the limiting dilution. The limiting dilution is preferred. Cloning should be performed preferably several times. The monoclonal antibody-producing hybridomas thus prepared may be subcultured in a normal culture medium or be subjected to long-term preservation in liquid nitrogen.

### (6) The production of monoclonal antibodies may be carried out as follows:

For acquisition of monoclonal antibodies from the hybridoma, the methods employed may include culturing the hybridoma according to usual methods to give the antibody in the form of culture supernatants, implanting and growing the hybridoma in a compatible mammal to harvest the antibody in the form of ascites, etc. The former method is suited for obtaining a highly pure antibody while the latter method is suited for antibody mass production.

The resultant hybridoma cells thus obtained are cultured in a suitable growth medium such as FCS-containing MEM, RPMI-1640 medium or others, and a desired monoclonal antibody can be obtained from the culture supernatant. Large amounts of monoclonal antibodies can be produced by propagating hybridomas as ascites tumors, etc. In this case, each hybridoma is implanted intraperitoneally and propagated in a histocompatible animal isogenic to an animal from which the myeloma cell is derived. Alternatively, each hybridoma can be inoculated, for example, in nude mice or others, and propagated to produce the monoclonal antibody in the ascites of the animals. The produced monoclonal antibody can be collected from the ascetic fluid and isolated. Prior to implantation of hybridomas, the animal is pretreated intraperitoneally with mineral oils such as Pristane (2,6,10,14-tetramethylpentadecane). After the pretreatment, the hybridoma can be propagated therein and the ascitic fluid can be harvested. The ascitic fluid can be used as a monoclonal antibody without purification or after purification by conventionally known methods, including salting out such as precipitation with ammonium sulfate, gel filtration with Sephadex and the like, ion exchange chromatography, electrophoresis, dialysis, ultrafiltration, affinity chromatography, high-performance liquid chromatography, etc. The isolated or purified products can be employed as monoclonal antibodies. Preferably, the monoclonal antibody-containing ascitic fluid is fractionated with ammonium sulfate, separated and purified by treatments with anion exchange gel such as DEAE-Sepharose, an affinity column such as protein A column, etc. More preferably, it is treated with affinity chromatography using immobilized antigens or antigen fragments (for example, synthetic peptides, recombinant antigen proteins or peptides, portions which the antibody can specifically recognize, etc.); affinity chromatography with immobilized protein A; hydroxyapatite chromatography; etc.

In addition, it is possible to use transgenic mice and other organisms (including other transgenic mammals) for expressing antibodies such as humanized antibodies against the immunogenic polypeptide products of the present invention. It is also possible to produce antibodies with recombinant DNA techniques wherein the antibody thus obtained in a large amount is sequenced and/or a nucleotide sequence is employed which codes for the antibody obtained from the hybridoma cell line. DNA coding for the monoclonal antibody is readily isolated and sequenced using conventional procedures (e.g., by using oligonucleotide probes that are capable of binding specifically to genes encoding the heavy or light chain of murine antibodies). Once isolated, the DNA may be placed, according to the aforementioned techniques, into expression vectors, which are then transfected into host cells such as CHO cells or COS cells. The DNA may be modified, for example, by substituting the coding sequence for human heavy- and light-chain constant domains for the homologous murine sequences (Morrison et al., Proc. Natl. Acad. Sci. USA, 81: 6581, 1984). Thus, chimeric and hybrid antibodies having a desired binding specificity can be prepared. Further, antibodies can be modified, including preparations of chimeric or hybrid antibodies by adaptations of known methods in synthetic protein chemistry, including those involving coupling agents as listed herein below.

Humanized antibodies are achievable by known techniques in the art (e.g., Jones et al., Nature, 321: pp.522-525 (1986); Riechmann et al., Nature, 332: pp.323-327 (1988); Verhoeyen et al., Science, 239: pp.1534-1536 (1988)). Human monoclonal antibodies can be achieved according to known techniques in the art. For producing human monoclonal antibodies, human myeloma cells and human-mouse hetero-myeloma cells are known in the art (Kozbor, J. Immunol., 133, pp.3001 (1984); Brodeur et al., Monoclonal Antibody Production Techniques and Applications, pp.51-63, Marcel Dekker, Inc., New York (1987)). Methods for making bispecific antibodies are known in the art (Millstein et al., Nature, 305: pp.537-539 (1983); WO93/08829; Traunecker et al., EMBO J., 10: pp.3655-3659 (1991); Suresh et al., "Methods in Enzymology", Vol. 121, pp.210 (1986)).

These antibodies may be treated with enzymes such as trypsin, papain, pepsin and others and occasionally be subjected to reduction to produce antibody fragments including Fab, Fab', and F(ab')₂. These antibody fragments may be occasionally used.

Representatives of the anti-galectin 9 Ab include those which bind to all of Gal-9L, -9M and -9S, those which bind to both of Gal-9L and -9M but are non-reactive with Gal-9S, those which bind to Gal-9L, but are non-reactive with Gal-9M and -9S, those which specifically bind to each of Gal-9L, -9M and -9S, those which are specifically reactive with any of galectin 9 C-terminal CRD and peptide fragments thereof, those which are specifically reactive with any of galectin 9 N-terminal CRD and peptide fragments thereof, those which specifically bind to each of link peptides and peptide fragments thereof, etc.

The antibodies are useful in any known assay, including competitive binding assays, direct and indirect sandwich assays, and immunoprecipitation assays (Zola, Monoclonal Antibodies: A Manual of Techniques, pp.147-158 (CRC Press, Inc., 1987)).

Any method known in the art may be employed for conjugating the antibody to each detectable moiety. Such methods include those described in David et al., Biochemistry, Vol. 13, pp. 1014-1021 (1974); Pain et al, J. Immunol. Meth., 40: pp. 219-231 (1981); and "Methods in Enzymology", Vol. 184, pp.138-163 (1990). The antibody to be labeled with a marker may include IgG fractions, and specific binding fragments Fab' obtainable by reduction after pepsin digestion. The labels include enzymes (e.g., peroxidases, alkaline phosphatases, beta-D-galactosidases, etc.), chemical substances, fluorescences, radioisotopes, and the like, as disclosed herein below.

In the present invention, detection (including prediction) and measurement (assay) can be carried out by immunostaining including, for example, immunohistochemistry (IHC) staining, immuno-electron microscopy, and immunoassays such as competitive and non-competitive immunoassays. The assays can also be conducted by radioimmunoassay (RIA), FIA, LIA, EIA, ELISA, etc., and with or without B-F separation. The assay is carried out preferably by RIA, EIA, FIA, LIA, and sandwich assays. In an embodiment of the sandwich assay, one of the antibodies is set against this inventive Gal-9L polypeptide or a Gal-9L-related peptide fragment while the other directed against a site with the C-terminal residues of galectin-9, wherein one of both the antibodies is detectably labeled (needless to say, other combinations are also possible and may be designed as suitable according to the purpose). The other antibody capable of recognizing the same antigen may be immobilized on a solid phase. As required, incubation is carried out to sequentially react a sample to be assayed, with labeled antibodies, and solid phased antibodies. After the non-binding antibodies are separated, the label is detected or measured. The amount of the measured label is proportional to the amount of an antigen, i.e., the amount of a galectin-9 polypeptide antigen. This assay is referred to as simultaneous sandwich assay, forward sandwich assay, or reverse-sandwich assay, based on the difference according to the addition sequence of the insolubilized antibody and the labeled antibody. For example, washing, stirring, shaking, filtration, pre-extraction for antigen, and other treatments are optionally adopted in the measurement or assay process under specific conditions. The other assay conditions including the concentrations of specific reagents, buffers, and others, temperatures, incubation times, and the like can vary according to elements, such as the concentration of antigens in the sample, or the nature of samples to be measured. Any person ordinary skilled in the art can suitably select and determine optimal conditions effective for each assay while using the general experimentation and perform the selected measurement.

Various carriers on which antigens or antibodies can be immobilized are available in the art, and they can be arbitrarily and suitably selected in the present invention. For the immobilization, various carriers are known which can be used for antigen-antibody interactions. It goes without saying that any well-known carrier can be selected and used in the present invention. Preferred examples are inorganic materials including, for example, glass such as aminoalkylsilyl glass and other activated glass, porous glass, silica gel, silica-alumina, alumina, magnetized iron, magnetized alloy, etc.; organic polymer substances, such as polyethylene, polypropylene, polyvinyl chloride, polyvinylidene fluoride, polyvinyl, polyvinyl acetate, polycarbonate, polymethacrylate, polystyrene, styrene-butadiene copolymer, polyacrylamide, crosslinked polyacrylamide, styrene-methacrylate copolymer, polyglycidyl methacrylate, and acrolein-ethylene glycol dimethacrylate copolymer; cross-linked albumin, collagen, gelatin, dextran, agarose, crosslinked agarose, natural and modified cellulose (for example, cellulose, microcrystalline cellulose, carboxymethylcellulose, cellulose acetate, etc.), crosslinked dextran, polyamides (for example, nylon, etc.), polyurethane, polyepoxy resin, etc.; products obtained by emulsion polymerization of such organic polymer substances; silicon gums; cells, erythrocytes, etc.; and such substances having a functional group introduced thereinto, as required, by using a silane coupling agent, etc.

Also included are solid materials (bodies) such as filter paper, beads, tubes, cuvettes, inner walls of test containers such as test tubes, titer plates, titer wells, microplates, glass cells, cells made of synthetic materials such as plastic resin cells, glass rods, rods made of synthetic materials, rods thickened or thinned at the end, rods provided with a round protrusion or a flat protrusion at the end, thin-plated rods, and surfaces thereof.

Antibodies can be coupled with these carriers. Preferably anti-galectin-9 antibodies (including antisera and purified antibodies) or monoclonal anti-galectin-9 antibodies, which react specifically with antigens obtainable according to the present invention, may be coupled to such a carrier as mentioned above. Coupling between the carrier and those partners associated with these antigen-antibody interactions can be carried out by techniques including physical method such as adsorption; a chemical method using a coupling agent, etc. or an activated reactant; a method using a chemically interactional coupling. The label may include.enzymes, enzyme substrates, enzyme inhibitors, prosthetic groups, coenzymes, enzyme precursors, apoenzymes, fluorescent substances, pigments, chemoluminescent compounds, luminescent substances, coloring substances, magnetic substances, metal particles such as gold colloids, non-metal particles such as selenium colloids, radioactive substances and the like. The enzyme may include dehydrogenases, oxidoreductases such as reductases and oxidases; transferases that catalyze the transfer of functional groups such as amino, carboxyl, methyl, acyl, and phosphate groups; hydrolases that hydrolyze bonds such as ester, glycoside, ether, and peptide bonds; lyases; isomerases; ligases; and the like. Plural enzymes may be used in a conjugated form for detection (for example, enzymatic cycling may also be utilizable). Typical radioactive isotopes for the label include [³²P], [¹²⁵I], [¹³¹I], [³H], [¹⁴C], [³⁵S], etc. Typical enzymes for the label include peroxidases such as horseradish peroxidase; galactosidases such as E. coli beta-D-galactosidase; maleate dehydrogenases; glucose-6-phosphate dehydrogenases; glucose oxidases; gluocoamylases; acetylcholine esterases; catalases; alkaline phosphatases such as calf intestinal alkaline phosphatase and E. coli alkaline phosphatase, and the like. In the case where alkaline phosphatase is used, measurements can be done by monitoring or inspecting fluorescence, luminescence, etc., generated with substrates such as umbelliferone derivatives including 4-methylumbellipheryl phosphate; phenol phosphate derivatives including nitrophenyl phosphate; enzymatic cycling systems utilizing NADP; luciferin derivatives; dioxetane derivatives; and the like. It is also possible to use luciferin/luciferase systems. When catalase is used, the reaction takes place with hydrogen peroxide to produce oxygen which can be detected with an electrode or the like. The electrode may be a glass electrode, an ionic electrode using an insoluble salt membrane, a liquid-membrane type electrode, a polymer membrane electrode and the like. The enzyme label may be replaced with a biotin label and an enzyme-labeled avidin (streptavidin). Such use of a biotin-avidin system, use of a secondary antibody, for example, an antibody against an anti-galectin antibody, and other sensitivity-enhancing methods known in the art may be employed. For the label, a plurality of various kinds of labels or markers can be used. In this case, it is possible to perform plural measurements continuously or discontinuously and/or simultaneously or separately.

According to the present invention, signal formation may be done using enzyme-reagent combinations, such as combinations of horseradish peroxidase or other peroxidases with a member selected from 4-hydroxyphenylacetic acid, o-phenylenediamine (OPD), tetramethylbenzidine (TMB), 5-aminosalicylic acid, 3,3-diaminobenzidine tetrahydrochloride (DAB), 3-amino-9-ethylcarbazole (AEC), tyramine, luminol, lucigenin-luciferin or derivatives thereof, Pholad luciferin, etc.; combinations of alkaline phosphatases with a member selected from lumigen PPD, (4-methyl)-umbelliferyl phosphate, p-nitrophenol phosphate, phenol phosphate, bromochloroindolyl phosphate (BCIP), AMPAK™ (DAKO), AmpliQ™ (DAKO), etc.; combinations of beta-D-galactosidases or glucose-6-phosphate dehydrogenases with a member selected from 4-methylumbelliferyl-beta-D-galactoside or other umbelliferyl galactosides, o-nitrophenol-beta-D-galactoside, other nitrophenyl galactosides, etc.; combinations of glucose oxidases with ABTS, etc. The signal may be formed with those capable of enzymatically forming quinole compounds such as hydroquinone, hydroxybenzoquinone, and hydroxyanthraquinone, thiol compounds such as lipoic acid and glutathione, phenol derivatives or ferrocene derivatives.

The fluorescent substances and chemiluminescent compounds may include fluorescein isothiocyanate (FITC); Rhodamine derivatives such as Rhodamine B isothiocyanate and tetramethyl Rhodamine isothiocyanate (RITC), and tetramethylrhodamine isothiocyanate isomer R (TRITC); 7-amino-4-cumarin-3-acetic acid, dancyl chloride (5-(dimethyl-amino)-1-naphtalenesulfonyl chloride), dancyl fluoride, fluorescamine (4-phenylspiro[furan-2(3H),1'-(3'H)-isobenzofuran]-3,3'-dione), phycobiliprotein, acridinium salts; luminol compounds such as lumiferin, luciferase and aequorin; imidazoles, oxalic acid esters, rare earth chelate compounds, cumarin derivatives, etc. Although the generated signals, etc. (including luminescence, fluorescence, etc.) can be detected visually, they may be inspected or monitored with a known device, such as a fluorophotometer and a plate reader. For the detection of signals emitted by a radioactive isotope, etc., a known detector, such as a gamma counter and a scintillation counter, may be used.

The labeling can be accomplished by the reaction of a thiol group with a maleimide group, the reaction of a pyridyldisulfide group with a thiol group, the reaction of an amino group with an aldehyde group, etc. Additionally, it can be suitably selected from widely known methods, techniques which can be easily put into practice by an artisan skilled in the art, and any of modifications derived therefrom. The coupling agents used for producing the foregoing immunoconjugate or for coupling with carriers are also applicable and utilizable. The coupling agents include, for example, formaldehyde, glutaraldehyde, hexamethylene diisocyanate, hexamethylene diisothiocyanate, N,N'-polymethylene bisiodoacetamide, N,N'-ethylene bismaleimide, ethylene glycol bissuccinimidyl succinate, bisdiazobenzidine, 1-ethyl-3-(3-dimethylaminopropyl) carbodiimide, succinimidyl 3-(2-pyridyldithio)propionate (SPDP), N-succinimidyl 4-(N-maleimidomethyl)cyclohexane-1-carboxylate (SMCC), N-sulfosuccinimidyl 4-(N-maleimidomethyl)-cyclohexane-1-carboxylate, N-succinimidyl (4-iodoacetyl)aminobenzoate, N-succinimidyl 4-(1-maleimidophenyl)butyrate, N-(ε-maleimidocaproyloxy)-succinimide (EMCS), iminothiolane, S-acetylmercaptosuccinic anhydride, methyl-3-(4'-dithiopyridyl)propionimidate, methyl-4-mercapto-butyrylimidate, methyl-3-mercaptopropionimidate, N-succinimidyl-S-acetylmercaptoacetate, etc.

According to the assay of the present invention, substances to be measured can be made to react sequentially with labeled antibody reagents (such as antisera, purified antibodies and monoclonal antibodies, labeled with enzymes or other markers) and then with antibodies coupled on a carrier, or all the members can be reacted each other simultaneously. The order of adding reagents (members) may vary depending on the type of carrier system selected. In the case where sensitized beads such as sensitized plastic beads are used, the labeled antibody regents (such as labeled antisera, purified antibodies and monoclonal antibodies) are first put into a suitable test tube, together with a sample including substances to be measured, followed by addition of the sensitized beads (such as the sensitized plastic beads). Measurement can be then carried out.

For measurements (and/or detections) according to the present invention, the immunological measurement (immunoassay) is applied. For the measurement (assay), the solid phase carriers used may include various materials and shapes which can be selected from balls, microplates, sticks, microparticles, test tubes, and the like, made of polystyrene, polycarbonate, polypropylene, polyvinyl and other materials capable of well adsorbing proteins such as antibodies.

The assay can be carried out in a suitable buffer system so as to maintain optimal pH (for example, between pH about 4 and about 9). The particularly preferred buffers may include acetate buffers, citrate buffers, phosphate buffers, Tris buffers, triethanolamine buffers, borate buffers, glycine buffers, carbonate buffers, Tris-HCl buffers, veronal buffers, etc. The buffers can be used optionally in a mixed form at any ratio. Preferably, the antigen-antibody interaction is carried out at a temperature between about 0 and 60°C.

The labeled antibody reagents (e.g., monoclonal antibodies labeled with enzymes or others, etc.), the immobilized antibody reagents (coupled to a carrier), and substances to be assayed can be incubated until equilibrium is reached. However, the reaction may be stopped after limited incubation wherein the solid phase is separated from the liquid phase at a time point well before the antigen-antibody interaction equilibrates, and the level of labels (such as enzymes) existing in either of the liquid and solid phases may be measured. Measurement operation can be performed with automated measuring instruments. A luminescence detector, a photo detector or the like may be used to measure or detect indication signals generated as a result of substrate conversion by the action of an enzyme. In the antigen-antibody interaction, adequate means can be taken so as to stabilize reagents to be used, samples to be assayed, and labels such as enzymes, respectively, and/or to stabilize antigen-antibody interactions per se. Further, proteins, stabilizers, surfactants, chelating agents or others, as mentioned herein below, can be added to incubation solutions for eliminating non-specific reaction, reducing inhibitory influences acting thereon, and/or activating assay reaction. The chelating agent may include preferably ethylenediamine tetraacetate (EDTA). The blocking techniques for preventing non-specific binding reaction, may be employed, which techniques are generally employed in the art or well-known among the persons skilled in the art. The blocking can be achieved by treatments with mammal normal serum, serum proteins, albumin, hemoglobin, ovalbumin (OVA), skim milk, fermented milk products, collagen, gelatin, or others. These methods or techniques can be used without any limitation so long as the use is for the purpose of preventing non-specific binding reaction. Further, a suitable liquid may be selected and used from the above-mentioned buffers and salt solutions for washing the sample and solid phase. The liquid used may be admixed with a substance selected from the group consisting of Tween 20 (trade name), Tween 80 (trade name), NP-40 (trade name), Triton X100 (trade name), Briji (trade name), and other nonionic surfactants, CHAPS and other zwitterionic surfactants, cationic surfactants, and anionic surfactants.

The samples to be assayed according to the present invention may include various forms of solutions such as colloid solutions, non-fluid samples and the like. Preferably, the samples are biological samples including, for example, all organs and tissues, such as thymus, testis, intestine, kidney, brain, breast tissues, ovary, uterus, prostate gland, colon/rectum, stomach, lungs, bronchus, pancreas, and liver; malignant tumors of such organs and tissues, leukemic cells, blood, sera, plasma, articular fluid, cerebrospinal fluid, pancreatic juice, bile, saliva, amniotic fluid, urine, and other body fluids, cell culture medium, tissue culture medium, tissue homogenate, biopsy samples, tissues, cells, etc.

In applying various analytic and quantitative assays including those individual immunological assays (immunoassays) to the measurements (assays) of the present invention, it is unnecessary to set up therefor any special condition, operation, etc. Assay systems for the targets of the present invention or target substances having a substantially equivalent activity thereto may be constructed by adaptations of technical consideration ordinarily given by artisans in the art over general conditions and operations suitable for each of the methods.

The assay systems for galectin-9 include, for example, protein assay systems, such as systems for immunostaining (METHODS, 24, 289-296 (2001); J Immunol Methods, 47(2), 129-144 (1981); ibid., 150(1-2), 5-21, 23-32 & 151-158 (1992); Cancer J, 7(1), 24-31 (2001), etc.) and immunoelectron microscopy (Mol Biotechnol, 7(2), 145-151 (1997); J Electron Microsc Tech., 19(1), 57-63 & 64-79 (1991); ibid., 19(3), 305-315 (1991), etc.), and expression gene assay systems, such as in situ hybridization systems, used effectively for tissues; protein assay systems, such as systems for EIA, RIA, FIA, LIA, and Western blotting (J Electron Microsc (Tokyo), 45(2), 119-127 (1996); Methods Biochem Anal., 33, 1-58 (1988); Methods Enzymol., 271, 177-203 (1996); ibid., 305, 333-345 (2000); J Immunol Methods, 152(2), 227-236 (1992); ibid., 170(2), 177-184 (1994); ibid., 195(1-2), 149-152 (1996); Yoshiyuki Kuchino, et al. ed., "Idenshi-Tanpakushitsu, Jikken Sosa Burottingu-ho" (Genes and Proteins, Experimental Procedures, Blotting Methods), Soft Science Co., Ltd., Japan, Nov. 10, 1987, etc.), and expression gene assay systems, such as systems for Northern blotting, dot blotting, RNase protection assay, RT-PCR (reverse transcription polymerase chain reaction), Real-Time PCR (Clinical Chemistry, 46: 11, 1738-1743 (2000)), used effectively for tissue extracts; and protein assay systems, such as systems for EIA, RIA,'FIA, LIA, and Western blotting, used effectively for blood and body fluids, etc. The assay systems for anti-galectin-9 antibodies include, for example, protein assay systems, such as systems for EIA, RIA, FIA, LIA, and Western blotting, used advantageously for blood and body fluids, etc.

With regard to EIA systems, for example competitive methods utilize solid-phase anti-galectin-9 Ab, a labeled antigen and a non-labeled antigen (the antigen may be galectin-9 or a peptide fragment thereof, etc.), while non-competitive methods, such as sandwich assays, do solid-phase anti-galectin-9 Ab, and labeled anti-galectin-9 Ab, as well as labeled or immobilized antibodies directed to anti-galectin-9 Ab without directly labeling or immobilizing anti-galectin-9 Ab. Sensitivity amplification or enhancement methods include, for example, combinations with non-enzyme-labeled primary Ab, including those using polymers, enzymes, primary Ab (adoptions of Envision reagents; Enhanced Polymer One-step Staining (EPOS)) and combinations with non-enzyme-labeled secondary Ab, including combinations of enzymes with anti-enzyme antibody conjugates such as the PAP (peroxidase-antiperoxidase method), combinations of biotin-labeled secondary Ab with biotin-labeled enzyme-avidin complexes such as the SABC (avidin-biotinylated peroxidase complex method), combinations of biotin-labeled secondary Ab and biotin-labeled enzyme-streptavidin complexes such as the ABC (streptavidin-biotin complex method) and the LSAB (labeled streptavidin-biotin method), combinations of SABC with biotin-labeled tyramide and enzyme-labeled streptavidin such as the CSA (catalyzed signal amplification), methods in which a secondary antibody and an enzyme are labeled with a polymer, etc.

For details of those conventional techniques, a variety of reviews, texts, books, etc. may be referred to. They are, for example, Hiroshi Irie (ed.), "Radioimmunoassay", Kodansha Ltd., Japan, 1974; Hiroshi Irie (ed.), "Zoku-Radioimmunoassay" (Radioimmunoassay; Second Edition), Kodansha Ltd., Japan, 1979; Eiji Ishikawa et al. (ed.), "Koso Meneki Sokuteiho" (Enzyme Immunoassays), Igaku-Shoin Ltd., Japan, 1978; Eiji Ishikawa et al. (ed.), "Koso Meneki Sokuteiho" (Enzyme Immunoassays) (2nd Edition), Igaku-Shoin Ltd., Japan, 1982; Eiji Ishikawa et al. (ed.), "Koso Meneki Sokuteiho" (Enzyme Immunoassays) (3rd Edition), Igaku-Shoin Ltd., Japan, 1987; H. V. Vunakis et al. (ed.), "Methods in Enzymology", Vol. 70 (Immunochemical Techniques, Part A), Academic Press, New York (1980); J. J. Langone et al. (ed.), "Methods in Enzymology", Vol. 73 (Immunochemical Techniques, Part B), Academic Press, New York (1981); J. J. Langone et al. (ed.), "Methods in Enzymology", Vol. 74 (Immunochemical Techniques, Part C), Academic Press, New York (1981); J. J. Langone et al. (ed.), "Methods in Enzymology", Vol. 84 (Immunochemical Techniques, Part D: Selected Immunoassays), Academic Press, New York (1982); J. J. Langone et al. (ed.), "Methods in Enzymology", Vol. 92 (Immunochemical Techniques, Part E: Monoclonal Antibodies and General Immunoassay Methods), Academic Press, New York (1983); J. J. Langone et al. (ed.), "Methods in Enzymology", Vol. 121 (Immunochemical Techniques, Part I: Hybridoma Technology and Monoclonal Antibodies), Academic Press, New York (1986); J. J. Langone et al. (ed.), "Methods in Enzymology", Vol. 178 (Antibodies, Antigens, and Molecular Mimicry), Academic Press, New York (1989); M. Wilchek et al. (ed.), "Methods in Enzymology", Vol. 184 (Avidin-Biotin Technology), Academic Press, New York (1990); J. J. Langone et al. (ed.), "Methods in Enzymology", Vol. 203 (Molecular Design and Modeling: Concepts and Applications, Part B: Anibodies and Antigens, Nucleic Acids, Polysaccharides, and Drugs), Academic Press, New York (1991); etc. and references quoted in the above documents, the disclosures of which are incorporated herein by reference.

The active components of the present invention [e.g., (a) the Gal-9 polypeptides, peptide fragments thereof, salts thereof, their related peptides, etc.; (b) the Gal-9-coding or its related peptide-coding nucleic acids (including DNA, etc.), or the like; (c) the antibodies and their fragments (including monoclonal antibodies) as set forth in the present invention or derivatives thereof; (d) the compounds, or salts thereof, which control or regulate said interesting activities (or functions) exerted by Gal-9 (the compounds which promote or suppress/inhibit Gal-9 biological activities, including phenomena that they promote or suppress/inhibit Gal-9 protein-dependent cytotoxic actions, apoptosis-inducing actions, Gal-9 abilities of exerting desirable efficacies without any adverse effect on normal cells, etc. and degeneration, overproduction, or degradation of tissues or proteins); compounds, or their salts, which control or regulate said protein production; (e) the compounds identified or characterized by means of the present invention; etc.] can be employed as pharmaceutical agents usually in the form of a pharmaceutical composition or preparation alone or in admixture with a variety of pharmaceutically acceptable aids. For example, the active components can be administered alone or in the form of a pharmaceutical composition or preparation in admixture with any of various pharmaceutically acceptable aids. Preferably, it may be administered in the form of a convenient pharmaceutical composition or formulation suitable for oral, topical, parenteral application, or the like. Any of dosage forms (including those for inhalation and rectal administration) may be selected depending on purpose. The active components of the present invention can be used in combination with any of various drugs, including antitumor drugs (antineoplastic drugs), tumor metastasis-inhibitors, inhibitors for thrombogenesis, therapeutic drugs for joint destruction, analgesics, anti-inflammatory drugs, and/or immunosuppressants, which can be employed as not being restricted to particular species as long as they serve effectively or advantageously. For instance, they can be optionally selected from those known in the art.

The parenteral administration includes topical, percutaneous, intravenous, intramuscular, subcutaneous, intracutaneous, and intraperitoneal routes. It is also possible to apply the drug directly to affected sites, and, in a certain case, the direct application is suitable. Preferably mammal animals including human can receive the drug orally or parenterally (e.g., intracellularly, intra-tissularly, intravenously, intramuscularly, subcutaneously, intracutaneously, intraperitoneally, intrapleurally, intraspinally, by instillation, enterally, per rectum, by instillation into the ear, eye, or nose by swabbing or application on the teeth, skin or mucosa, etc.). Specific dosage forms of the pharmaceutical preparations and formulations include pharmaceutical solutions, pharmaceutical dispersions, semisolid preparations, particulate preparations, shaped preparations, extractives, etc. Examples of the dosage forms are tablets, coated tablets, sugar coated tablets, pills, troches, hard capsules, soft capsules, microcapsules, implants, powders, pulvis, granules, fine granules, injections, liquids and solutions, elixirs, emulsions, irrigations, syrups, mixtures, suspensions, liniments, lotions, aerosols, sprays, inhalations, nebula, ointments, plasters, patches, pastes, cataplasms, creams, oleates, suppositories (e.g., rectal suppositories), tinctures, dermatologic waters, ophthalmic solutions, collunariums, auristillae, paints, transfusions, powders for injection solutions, lyophilized preparations, conditioned gels, etc.

The pharmaceutical compositions can be formulated in accordance with conventional techniques. For example, the pharmaceutical composition or formulation may comprise at least one of said compounds (active components including proteins) of the present invention or a salt alone or in admixture with physiologically allowable carriers, pharmaceutically acceptable carriers, adjuvants, vehicles, excipients, diluents, etc. The compound (active component or protein) of the present invention or a salt thereof is usually admixed with a single member selected from the group consisting of physiologically allowable carriers, pharmaceutically acceptable carriers, adjuvants, vehicles, excipients, diluents, flavoring agents, perfuming agents, sweetening agents, expanders, antiseptics, stabilizers, binders, pH regulators, buffering agents, detergents (surfactants), bases, solvents, fillers, bulking agents, solution adjuvants, solubilizers, tonicity agents, emulsifiers, suspending agents, dispersers, viscosity-increasing agents, thickening agents, gelling agents, stiffening agents, absorbents, adhesives, elastomers, plasticizers, disintegrants, aerosol propellants, preservatives, antioxidants, opacifying agents, humectants, emollients, charge protectors, soothing agents, etc., or suitably in a combination thereof, depending on necessity, to give a unit dose form which is required for generally approved pharmaceutical practices.

Formulations suitable for parenteral routes include aseptic solutions or suspensions containing at least one active component in admixture with water or other pharmaceutically acceptable media. Examples of such parenteral formulations are injections. Preferred liquid carriers for injection generally include water, saline, dextrose solution, other related saccharide solutions, ethanol, glycols such as propylene glycol and polyethylene glycol, etc. For the preparation of injections, the active component is usually admixed with any of carriers such as distilled water, Ringer's solution, physiological saline, suitable dispersing agents, moistening agents, suspending agents, and other materials to form injectable formulations including solutions, suspensions, emulsions, etc. by known techniques in the art.

Examples of aqueous liquids for the injection are a physiological saline and isotonic solutions containing glucose and other aids (e.g. D-sorbitol, D-mannitol, sodium chloride, etc.) where they may be used in combination with a suitable pharmaceutically acceptable auxiliary solubilizer such as alcohol (e.g. ethanol, etc.), polyalcohol (e.g. propylene glycol, polyethylene glycol, etc.), nonionic surface-active agent (e.g. Polysorbate 80™, HCO-50, etc.), etc. The injectable oily liquids may include sesame oil, soybean oil, etc. where they may be used in combination with benzyl benzoate, benzyl alcohol, and other materials as auxiliary solubilizers. In addition, buffers (e.g. phosphate buffer, sodium acetate buffer, etc.) or agents for osmoregulation, analgesic agents (e.g. benzalkonium chloride, procaine hydrochloride, etc.), stabilizers (e.g. human serum albumin, polyethylene glycol, etc.), preservatives (e.g. benzyl alcohol, phenol, etc.), antioxidants such as ascorbic acid, absorbefacients, etc. may be admixed therewith too. The prepared injection solution is usually filled in suitable ampoules.

For parenteral administration, solution or suspension unit dosage forms are prepared in pharmaceutically acceptable sterile fluids such as water, ethanol, and oils, in admixture with or without detergent and other pharmaceutically acceptable aids. The oily vehicle and solvent used in the parenteral formulation may include natural, synthetic or semi-synthetic mono-, di-, or triglycerides; natural, semi-synthetic or synthetic fats and oils; and fatty acids. Examples of such oily vehicles and solvents are plant oils such as peanut oil, corn oil, soybean oil, and sesame oil. For example, this injection can usually be prepared to form unit doses each containing approximately from 0.1 to 10 parts of the compound of the present invention per 100 parts by weight of the dose composition.

The formulation suitable for topical use, such as buccal or rectal application, includes mouthwashes and gargles, dentifrices, sprays for buccal cavity, inhalants, ointments (salves), dental fillers, dental coating agents, dental pastes, suppositories, etc. The mouthwashes and other dental agents are prepared by conventional techniques, using pharmaceutically acceptable carriers. For the sprays for buccal cavity and inhalants, the compound of the present invention can be applied to teeth or other sites after dissolving alone or together with pharmaceutically acceptable inert carriers, in an aerosol or solution for nebulizers, or in the form of powders for inhalation. The ointments (salves) are prepared by conventional techniques, in admixture with conventionally employed pharmaceutical bases such as ointment bases (white petrolatum, paraffin, olive oil, macrogol 400, macrogol ointment, etc.).

The pharmaceutical drugs for topical application (including painting) to teeth and skin can be prepared in the form of a solution or suspension utilizing suitably sterilized water or non-aqueous vehicles. The additives used include buffering agents such as sodium bisulfite and disodium edetate; preservatives including antiseptic, antimicrobial and antifungal agents such as acetic acid, phenylmercuric nitrate, benzalkonium chloride and chlorhexidine; and thickeners such as hypromellose.

The suppositories can be prepared by conventional techniques utilizing carriers well known in the art, preferably suitable non-irritative excipients. Examples of the excipients are those which are solid at room temperature but liquid at rectal temperature wherein such substances melt in the rectum to deliver a drug, such as polyethylene glycols, lanolin, cacao butter, and fatty acid triglycerides. In the suppositories, the compounds of the present invention are applied in the form of compositions containing approximately from 0.1 to 95 percent (weight per volume). The compound, depending on the vehicle and concentration used, can be either suspended or dissolved in the vehicle. Adjuvants such as a local anesthetic, preservative and buffering agent can be dissolved in the vehicle. The formulations suitable for oral application include solid compositions such as tablets, pills, capsules, powders, granules, and troches; fluid compositions such as solutions, syrups, and suspensions; etc. In preparing oral formulations, pharmaceutical adjuvants known in the art are employed. The tablets and pills can be prepared further by enteric coating. When the unit dosage form is a capsule, fluid carriers such as fats and oils can be contained in addition to the aforementioned materials.

When the active components are proteins or polypeptides, conjugation to polyethylene glycol (PEG) is particularly useful, because its toxicity is extremely low in mammals. Further, the conjugation with PEG can sometimes reduce the immunogenicity and antigenicity of a heterologous compound effectively. The compound may be given after being put in a microcapsule device. A polymer such as PEG can be easily attached to an α-amino group of amino-terminal amino acids, an ε-amino group of lysine side chains, a carboxyl group of aspartic acid or glutamic acid side chains, an α-carboxyl group of carboxyl-terminal amino acids, or an activated derivative of glycosyl chains attached to certain asparagine, serine or threonine residues. Various activated forms of PEG suitable for direct reaction with proteins are known. PEG reagents useful for reaction with amino groups of a protein include active esters of carboxylic acids and carbonate derivatives, particularly those having N-hydroxysuccinimide, p-nitrophenol, imidazole, or 1-hydroxy-2-nitrobenzene-4-sufonate as a leaving group. Similarly, PEG reagents having an aminohydrazine or hydrazide group are useful for reaction with aldehydes produced by periodate oxidation of proteins.

Further, for the aforementioned therapeutic and/or prophylactic agents containing the nucleic acids (including DNA) of the present invention, said nucleic acid can be applied alone, or by ligation with suitable vectors used in the aforementioned genetic recombination techniques, including virus-derived vectors such as vectors derived from retrovirus. The nucleic acids (including DNA) of the present invention can be administered by conventional known techniques, in unmodified forms or in forms formulated in admixture with suitable aids or physiologically acceptable carriers, for example, to promote transfer into intracellular compartments. The nucleic acids (including DNA) of the present invention can be presented for administration to humans and animals in pharmaceutical compositions or preparations as aforementioned. For administration of the nucleic acids (including DNA) of the present invention, techniques known as gene therapy can also be applied.

Dose levels of said active components may vary within a wide range. Specific dose levels and administration cycles for any particular patient will be employed depending upon a variety of factors including the activity of specific compounds employed, the sex, age, body weight, general health, diet, time of administration, route of administration, rate of excretion, drug combination, and the severity of the particular disease undergoing therapy.

For the manufacture of pharmaceutical compositions and preparations, the additives, other materials, preparation methods and the like can be suitably selected from those disclosed in Nippon Yakkyokuho Kaisetsusho Henshu Iinkai (Ed.), "14th Edition Nippon Yakkyokuho Kaisetsusho (Commentary on The Japanese Pharmacopoeia 14th Edition (JPXIV))", June 27, 2001, Hirokawa Pub. Co., Tokyo, Japan; Hisashi Ichibagade et al. (Ed.), "Iyakuhin no Kaihatsu (Pharmaceutical Research and Development, Ikuo Suzuki, chief editor), Volume 12 (Seizai Sozai I (Pharmaceutical Necessities 1))", October 15, 1990, Hirokawa Pub. Co., Tokyo, Japan; ibid., Volume 12 (Seizai Sozai II (Pharmaceutical Necessities 2)), October 28, 1990, Hirokawa Pub. Co., Tokyo, Japan; etc., depending on necessity, and can be adapted by referring to the disclosures therein.

The active substances or components according to the present invention include (a) galectin-9 and its analogues, (b) polynucleotides encoding galectin-9 or polypeptides having a biological activity substantially equivalent to that of galectin-9, (c) galectin-9 production/release-inducing factors, (d) anti-galectin-9 receptor antibodies, and (e) antibodies against galectin-9-binding saccharide chains, as described herein. These substances and components are useful for utilizing the following properties of galectin-9: exerting cytotoxity toward tumor cells, but not toward normal cells; inducing apoptosis in tumor cells, but not in normal cells; inhibiting metastasis of malignant cells; and inducing apoptosis in activated immune cells, in particular, in activated CD4-positive T cells, but not in resting T cells, in particular, in CD4-positive T cells (helper T cells). Thus, the above-mentioned substances and components are promising to serve as drugs utilizing activities similar to those of antineoplastic agents, anti-allergy agents, immunosuppressants, therapeutic agents for autoimmune disease, anti-inflammatory agents, and adrenocortical steroid hormones.

### [Screening of galectin-9-binding proteins]

By using galectin-9, screening and identification of galectin-9-binding molecules participating in achievement of novel functions as disclosed in the above-mentioned description. In particular, screening and identification of galectin-9-binding proteins participating in apoptosis can be performed. Raw materials derived from various organisms can be used as targets to be screened or assayed without any limitation. In some exemplary cases, a cell line such as a human T cell-derived cell line, MOLT-4, in which apoptosis is induced by the addition of galectin-9, can be preferably used as a starting source material. Molecular interaction screening can be performed by various methods known in the technical field of this invention alone or in combination of them. For example, the identification of a galectin-9-interacting protein from candidate proteins can be carried out by suitable known methods. By the identification of the protein that interacts with galectin-9, a novel function owned by the target protein and a control mechanism for the target protein, for example, a control mechanism through galectin-9, can be disclosed and verified.

Methods for the identification include, but not limited to, (1) immunoprecipitation, (2) West-Western blotting (or far-Western method including ligand-blotting technology), (3) intermolecular cross-linking, (4) expression cloning analysis, (5) two-hybrid system, (6) phage display analysis, (7) surface plasmon resonance technology, and (8) fluorescence polarization, and also include other methods known in this technical field and modified methods thereof.

In the immunoprecipitation, an antibody specific to a target protein is added to sample solutions of various proteins. A protein that interacts with the target protein is isolated as an immune precipitate of an immune complex, and is then identified by SDS-PAGE or further Western blotting. This method is effective for investigating the presence of proteins that interact with the target protein. In this method, specific antibodies against the target protein, specific antibodies against known proteins, or resins such as protein A- or protein G-Sepharose which traps antibodies, can be used. Additionally, a solution of protein labeled with RI or the like is preferably prepared. Commercially available kits can be also used. Examples of such kits include Affi-Prep 10, Affi-Gel Hz (BIO-RAD Laboratories), and NHS Sepharose HP (Pharmacia).

The proteins to be measured or assayed include those prepared and purified in the form of hybrid fusion proteins. The use of the fused proteins is advantageous because antibodies against Tag can be effectively utilized. Recombinant proteins can be prepared by utilizing Escherichia coli, yeast cells, or mammalian host cell expression systems, and can be also prepared by utilizing cell-free reticulocyte translation systems. Co-purification can be conveniently used as a purification of an objective fused protein by adding an excess amount of the fused protein to a protein solution. These methods can be referred to, for example, Rudner, D.Z., et al.: Mol. Cell Biol., 116, 1765-1773, 1998 (utilization of His-tag), Cleveland, D.W., et al.: J. Mol. Biol., 116, 207-225, 1977 [utilization of tau (microtubule-associated protein)].

In West-Western blotting or far-Western method, which is a modified Western blotting, protein probes including labeled target proteins and known proteins are used instead of antibodies, and the binding of the protein probe to proteins transferred to a membrane is detected or measured. With these methods, distribution, localization, and molecular weight of the protein that binds to the target protein used as the protein probe can be investigated. The method can be used in screening of an expression library for cDNA cloning (T. Nomura, et al. "Men-eki '92" (Immunity '92): cDNA cloning using protein probes, pp. 169-175, Nakayama Shoten Co., Ltd., 1992). A protein which is phosphorylated with protein kinase can be effectively used for these methods as a probe. The ligand-blotting is one of far-western blotting methods for analyzing a protein that binds to a ligand. When an RI-labeled ligand is used, the RI is detected. When a biotin-ligand is used, the ligand is detected by using a streptavidin-conjugated antibody. When an unlabeled ligand is used, the ligand is detected by using an antibody specific to the ligand and a labeled secondary antibody. Non-RI labeling agents can be also used. These methods can be referred to, for example, Soutar, A.K. & Wade, D.P., Protein function: a practical approach (Creighton, T.E. eds.), IRL press, pp. 55-76, 1989.

In the intermolecular cross-linking, protein-protein cross-linking is formed with a chemical cross-linking agent, and then separation by SDS-PAGE is performed. The detection is performed by Western-blotting, immunoprecipitation, or the like. This method is effective for analyzing an oligomer structure of the target protein, for investigating a protein (or domain) interacting with or being near the target protein, and for analyzing a subunit structure such as a receptor. Information about the chemical cross-linking agents can be found at the website (http://www.piercenet.com/) of Pierce Biotechnology, Inc. This method can be referred to, for example, Hermanson, G.T., Bioconjugate Techniques, Academic Press, 1996.

In the expression cloning analysis, arbitrary genes from a cDNA pool are expressed in cells, and the target protein or tag fusion protein is used as a probe to screen the cells. By detecting cells which interact with the probe, the specified cDNA is cloned from the cDNA pool. Thus, a receptor, ligand, or the like, is analyzed by the cloning. Expression systems known in this technical field can be utilized for performing the expression cloning. For example, expression systems using prokaryotic cells such as Escherichia coli, expression systems using cultured cells such as mammalian cells, and expression systems using Xenopus laevis oocytes are included. This method can be referred to, for example, Sasaki, H. (Ed.) "Muteki no Baiotekunikaru Sirizu Tokubetsu-hen, Baio Zikken no Susumekata (Bio technical series, Special version, How to proceed experiments in Bio)", Chapter 6, YODOSYA Co., Ltd., (1997). In the expression cloning using E. coli or the like, the transformation of E. coli cells or the like is performed by using, for example, λgt11 or λZAPII cDNA library, and then the screening is carried out. Basically, far-Western method is utilized. In the expression cloning of cultured cells, after transfection of the cultured cells with an expression vector carrying a certain cDNA, cells that bind to the target protein are selected from cDNA-expressing cells for cloning. Thus, screening can be carried out. The selection of the cells is preferably performed by, but not limited to, an enzyme-linked immunosorbent assay (ELISA) or fluorescence activated cell sorting (FACS). Alternatively, microinjection of Xenopus laevis oocytes with mRNA transcribed from the certain cDNA in vitro can be performed. The selection and cloning are carried out by utilizing the interaction between the expressed proteins and the target protein or the cell reaction caused by the interaction.

The two-hybrid systems are cloning systems for screening of interacting proteins wherein functional recovery phenomenon of a transcription activator is utilized. The transcription activity of a reporter gene is reactivated by interaction between the target protein (or a domain portion of the target protein) and a domain of a foreign protein. Such systems as used herein may include commercially available premade libraries. Examples of the premade libraries include, but not limited to, MATCHMAKER GAL4 cDNA LIBRARY and MATCHMAKER LexA cDNA LIBRARY (Clontech). Examples for preparing in-house libraries include, but not limited to, HybriZAP Two-hybrid vector system (Stratagene). This system can be referred to, for example, Yamamoto, M. (Ed.) "Baio Manyuaru Sirizu 1. Idenshi Kogaku no Kiso Gizyutu (Bio manual series 1. Fundamental technology for gene engineering)", YODOSYA Co., Ltd., 1993 and Downward, J., FEBL Lett., 338, 113-117, 1994.

In the phage display, phages that bind to the target protein are collected by using libraries of phages having surfaces containing random sequences of 5-7 amino acid residues, and then the phages are propagated. By repeating these processes, amino aid sequences having high specificity can be identified. Furthermore, proteins equivalent to the amino acid sequences can be selected from known proteins by retrieval of protein data bases based on the results. This method can be referred to, but not limited to, Smith, G.P. & Scott, J.K., Methods in Enzymology, Vol. 217, pp. 228-257, 1993.

The surface plasmon resonance has been typically developed with the purpose of monitoring biomolecular interactions on a sensor chip in real time with BIACORE™ systems. In BIACORE™, a sensor chip (gold thin film) is irradiated with light so that the light is totally reflected at a surface where biomolecules are not immobilized. Reduction of the reflected light intensity (generation of a SPR signal) is partially observed. The angle (change of refractive index) of the dark light depends on mass on the sensor chip. Examples of the sensor chips include sensor chip CM5 which has a surface of carboxymethyl dextran, sensor chip SA which has immobilized streptavidin, and sensor chip NTA which has immobilized NTA so as to immobilize poly-His fusion protein with nickel chelating. This method can be referred to, for example, Hashimoto, S., "Bunseki 5 Hyoumen Purasumon Kyomei Gensyo wo Riyousuru Seitai Bunshi Sogosayou no Kaiseki (Analysis 5: Analysis of biomolecular interaction using surface plasmon resonance)", pp. 362-368, 1997 and Natsume T., "Baio Manual UP Sirizu, Tanpakusitu no Bunshikan Sogsayou Zikkenhou (Bio manual UP Series: Experiment of molecular interaction of protein)" pp. 211-230, YODOSYA Co., Ltd., 1996.

The fluorescence polarization utilizes the principle as follows: when a fluorescent label-conjugated molecule is excited with plane polarized light and the molecule rotates and tumbles out of this plane during the excited state, the fluorescent light is emitted in different planes from the excitation plane. Degree of molecular movement depends on molecular size. If a molecule becomes very large by, for example, the formation of a complex, the emitted light remains highly polarized, but if a molecule is small, the movement is quick and the emitted light is depolarized. Therefore, molecular interactions can be studied by measuring this polarization. Various types of fluorescent labels, for example, FS, FITC, and FXS, can be used. The measurement can be performed with, for example, FBEACON™.

Apoptosis-associated galectin-9-binding proteins can be preferably screened and identified by using an affinity column. Synthetic peptides, fusion proteins, and antibodies can be used as ligands immobilized on the affinity column. A method for obtaining a galectin-9-binding protein by using a solution of MOLT-4 cell breakage as a starting source material will be described as a typical example of a method of isolation. The solution of MOLT-4 cell breakage is applied to a galectin-9 CT (C-terminal region) column. Then, galectin-9-binding proteins are yielded. The yielded proteins derived from the cell line are applied to an electrophoresis gel to separate the proteins based on their molecular weights. The resulting gel fragments are used for analyzing amino acid sequences of the proteins; thus, galectin-9-binding proteins can be identified. Galectin-9-binding protein candidates can be determined by using the above-mentioned methods for identifying proteins that interact with galectin-9.

Thus, the galectin 9-binding protein may be selected from the following human proteins:
4F2 heavy chain antigen (177216); ATPase, Na⁺/K⁺ transporting, alpha 1 polypeptide (21361181); sodium-dependent neutral amino acid transporter type 2 truncated isoform (15004317); stromal cell derived factor receptor 1 isoform a (9257240); stromal cell derived factor receptor 1 isoform b; heat shock 90kDa protein 1, beta (20149594); heat shock 90kDa protein 1, alpha; heat shock 70kDa protein 5 (glucose-regulated protein, 78kDa) (16507237); heat shock 70kDa protein 8 isoform 2 (24234686); heat shock 70kDa protein 9B precursor (24234688); fatty-acid-Coenzyme A ligase, long-chain 3 (27469830); NADH dehydrogenase (ubiquinone) Fe-S protein 1, 75kDa (NADH-coenzyme Q reductase) (4826856); S-adenosylhomocysteine hydrolase-like 1 (21361647); programmed cell death 8 isoform 1 (4757732); 60 kDa heat shock protein, mitochondrial precursor (129379); ATP synthase, H⁺ transporting, mitochondrial F1 complex, alpha subunit, isoform 1, cardiac muscle (4757810); ribophorin II precursor (88567); farnesyl-diphosphate farnesyltransferase 1 (4758350); Ubiquinol-cytochrome C reductase complex core protein 2, mitochondrial precursor (21903482); dolichyl-diphosphooligosaccharide-protein glycosyltransferase (21104416); calcium-binding transporter (6841066); NADH dehydrogenase-ubiquinone Fe-S protein 2 precursor (3540239); actin, beta (14250401); translation elongation factor EF-Tu-like protein P43 precursor, mitochondrial (7443384); metaxin 1 (4505281); sideroflexin 1 (23618867); TCR beta chain (2982508); Hnrnp A1 (2194069); phosphate carrier precursor isoform 1b (4505775); ATP synthase, H⁺ transporting, mitochondrial F1 complex, gamma polypeptide 1 (4885079); voltage-dependent anion channel 1 (4507879); hyaluronan-binding protein precursor (8699626); androgen-regulated short-chain dehydrogenase/reductase 1 (20070798); solute carrier family 25 (mitochondrial carrier; oxoglutarate carrier), member 11 (21361114); 3-hydroxybutyrate dehydrogenase precursor (17738292); B-cell receptor associated protein (1673514); ATP synthase, H⁺ transporting, mitochondrial F1 complex, O subunit (4502303); ATP synthase, H⁺ transporting, mitochondrial F0 complex, subunit d (5453559); ATP synthase, H⁺ transporting, mitochondrial F0 complex, subunit b, isoform 1 (21361565); small GTP-binding protein (13569962); NADH dehydrogenase (ubiquinone) Fe-S protein 8, 23kDa (NADH-coenzyme Q reductase) (4505371); vesicle trafficking protein sec22b (4759086); mitochondrial import receptor Tom22 (9910382); signal sequence receptor, delta (5454090); ATP synthase, alpha chain (114517 or P25705); ATP synthase, beta chain (114549 or P06576); Sodium/potassium-transporting ATPase beta-3 chain (1703470 or P54709); ADP, ATP carrier protein (113463, P12236, 113459, P05141, 113455 or P12235); ubiquinol-cytochrome C reductase complex core protein 1 (731047 or P31930); and Cytochrome c oxidase polypeptide II (117020 or P00403).

In particular, in connection with a galectin 9-binding protein selected from the following human proteins:
4F2 heavy chain antigen (177216); ATPase, Na⁺ /K⁺ transporting, alpha 1 polypeptide (21361181); sodium-dependent neutral amino acid transporter type 2 truncated isoform (15004317); stromal cell derived factor receptor 1 isoform a (9257240); stromal cell derived factor receptor 1 isoform b; heat shock 90kDa protein 1, beta (20149594); heat shock 90kDa protein 1, alpha; heat shock 70kDa protein 5 (glucose-regulated protein, 78kDa) (16507237); heat shock 70kDa protein 8 isoform 2 (24234686); heat shock 70kDa protein 9B precursor (24234688); S-adenosylhomocysteine hydrolase-like 1 (21361647); programmed cell death 8 isoform 1 (4757732); 60 kDa heat shock protein, mitochondrial precursor (129379); ribophorin II precursor (88567); farnesyl-diphosphate farnesyltransferase 1 (4758350); dolichyl-diphosphooligosaccharide-protein glycosyltransferase (21104416); calcium-binding transporter (6841066); TCR beta chain (2982508); hyaluronan-binding protein precursor (8699626); androgen-regulated short-chain dehydrogenase/reductase 1 (20070798); B-cell receptor associated protein (1673514); and Sodium/potassium-transporting ATPase beta-3 chain (1703470 or P54709), the properties of proteins which interact with galectin 9 can be examined in detail by adaptations of the aforementioned ① Immunoprecipitation, ② West-Western Blotting, or Far-Western Blot Synthesis, including Ligand Blotting, ③ Crossliking, ④ Expression Cloning, ⑤ Two-Hybrid System, ⑥ Phage Display, ⑦ Surface Plasmon Resonance (SPR),
⑧ Fluorescence Polarization and other techniques.

It should be noted that protein information data as well as protein-coding nucleic acid (including DNA) information data can be acquired when an entry of each number in parentheses is made at the NCBI internet home page (http://www.ncbi.nlm.nih.gov/).

Accordingly, said amino acid sequence information data and nucleotide (such as DNA) sequence information data can be utilized, that is, said Gal-9 binding proteins and their coding gene sequences can be treated and/or used by adaptations of the techniques and means which are described herein with reference to galectin 9 species in the same fashion. Such applicable techniques and means include those for producing and utilizing antibodies, including monoclonal antibodies and their applications. Accordingly, the present disclosures herein should be comprehended in connection with the aforementioned Gal 9-binding molecules as if said Gal 9-binding molecules were used in place of galectin 9. All the exchanged contents derived from the above adoptions are encompassed herein.

For instance, the present invention can provide a diagnostic method which comprises assaying for the amounts of polynucleotides encoding galectin-9-binding molecules in biological samples of subjects to be tested, comparing the resultant amounts with those of normal subjects, and determining subjects who are considered to be galectin-9-sensitive or who can be expected of a physiological effect induced by galectin-9. In a similar way, the present invention can provide a diagnostic method which comprises assaying for the amounts of galectin-9-binding molecules in biological samples of subjects to be tested, comparing the resultant amounts with those of normal subjects, and determining subjects who are considered to be galectin-9-sensitive or who can be expected of a physiological effect induced by galectin-9. The present invention can also provide oligonucleotides or polynucleotides that hybridize under stringent conditions to polynucleotides encoding galectin-9-binding molecules; DNA microarrays containing oligonucleotides that hybridize under stringent conditions to polynucleotides encoding galectin-9-binding molecules or DNA microarrays containing polynucleotides encoding galectin-9-binding molecules; primer sets for PCR amplification of polynucleotides encoding galectin-9-binding molecules, antibodies recognizing galectin-9-binding molecules, and antibodies that bind to epitopes other than the epitopes recognized by the above-mentioned antibodies.

Furthermore, the present invention can provide: methods for measuring the sensitivity to galectin-9 or the degree of expectation of a physiological effect induced by galectin-9 and kits used for said methods, which comprise at least the following elements: (a) an antibody that recognizes a galectin-9-binding molecule, and (b) a labeling-agent conjugated antibody that recognizes an epitope other than the epitope which is recognized by the former antibody as set forth in (a);
methods for measuring or detecting the sensitivity to galectin-9 or the level of expectation of a physiological effect induced by galectin-9 and kits used for said methods, which comprise at least the following elements: (a) a plate or membrane immobilized with a antibody that recognizes a galectin-9-binding molecule, and (b) a labeled antibody that recognizes an epitope other than the epitope which is recognized by the antibody as set forth in (a);
methods for measuring the degree of a physiological reaction associated with galectin-9, which comprise at least the steps of: (a) bringing a biological sample of a subject in contact with a solid phase immobilized with Ab, (b) washing the solid phase after the contact with the biological sample, (c) bringing the solid phase after the washing in contact with the labeled Ab, (d) measuring immobilized labels or free labels, (e) comparing the resultant label amount from (d), as an indicator for the amount of galectin-9-binding molecule, with a result of a control biological sample, and (f) using, as an indicator for the sensitivity to galectin-9 or the degree of expectation of a physiological effect induced by galectin-9, the significantly-high amount of galectin-9-binding molecule in comparison with the control biological sample;
methods for measuring the degree of a physiological reaction associated with galectin-9, which comprise at least the steps of: (a) preparing RNA from a biological sample of a subject to be tested, (b) subjecting the RNA prepared in step (b) to electrophoresis separating, (c) hybridizing the RNA separated in step (b) with labeled nucleotide probes that hybridize under stringent conditions to a polynucleotide encoding galectin-9-binding molecule, (d) comparing the amount of labeled nucleotide probe hybridized in step (c), as an indicator for the expression level of polynucleotides encoding galectin-9-binding molecules, with a result of a control biological sample, and (e) using, as an indicator for the sensitivity to galectin-9 or the degree of expectation of a physiological effect induced by galectin-9, the significantly-high expression level of polynucleotide encoding galectin-9-binding molecule in comparison with the control biological sample;
methods for measuring the degree of a physiological reaction relating to galectin-9, which comprise at least the steps of: (a) preparing RNA from a biological sample of a subject to be tested, (b) synthesizing a first cDNA strand with the RNA prepared in step (a) as a template and dT primers, (c) PCR amplification using the first cDNA synthesized in step (b) as a template with a primer set designed for PCR amplification of a polynucleotide encoding galectin-9-binding molecule, (d) separating PCR products obtained in step (c) by electrophoresis, (e) hybridizing the PCR products separated in step (d) to labeled nucleotide probes that hybridize under stringent conditions to a polynucleotide encoding galectin-9-binding molecule, (f) comparing the amount of labeled nucleotide probe hybridized in step (e), as an indicator for the expression level of polynucleotide encoding galectin-9-binding molecule, with a result of a control biological sample, and (g) using, as an indicator for the sensitivity to galectin-9 or the degree of expectation of a physiological effect induced by galectin-9, the significantly-high expression level of polynucleotide encoding galectin-9-binding molecule in comparison with the control biological sample;
methods for measuring the degree of a physiological reaction relating to galectin-9, which comprise at least the steps of: (a) preparing a fixed tissue specimen from a biological sample of a subject to be tested, (b) slicing the fixed tissue specimen prepared in step (a), (c) performing immunostaining of the sliced tissue preparations with an antibody that recognizes galectin-9-binding molecule, (d) comparing the degree of the immunostain in the biological sample of the subject with a result of a control biological sample, and (e) using, as an indicator for the sensitivity to galectin-9 or the degree of expectation of a physiological effect induced by galectin-9, the significantly-high amount of galectin-9-binding molecule in comparison with the control biological sample;
methods for measuring the degree of a physiological reaction relating to galectin-9, which comprise at least one step selected from the group consisting of: (a) PCR amplification of a biological sample from a subject to be tested with a primer set designed for PCR amplification of a polynucleotide encoding galectin-9-binding molecule, (b) DNA microarray analysis of a nucleic acid fraction separated from the biological sample of the subject, and (c) hybridization of the nucleic acid fraction separated from the biological sample of the subject to oligonucleotides or polynucleotides that hybridize under stringent conditions to a polynucleotide encoding galectin-9-binding molecule, and determining the amount of polynucleotides encoding galectin-9-binding molecule in the biological sample of the subject, and then comparing the amount of the polynucleotide with that of a normal subject;
the method for measuring the degree of a physiological reaction relating to galectin-9 according to any one of the above-mentioned methods, wherein the amount of galectin-9-binding molecule or the expression level of polynucleotide encoding galectin-9-binding molecule is quantitatively measured;
reagents comprising any of the antibodies, for applications of any one of the above-mentioned methods for measuring the degree of a physiological reaction relating to galectin-9;
methods for measuring or diagnosing the sensitivity to galectin-9, the degree of expectation of a physiological effect induced by galectin-9, or the degree of a physiological reaction relating to galectin-9, which comprise measuring the amount of galectin-9-binding molecule or the expression level of polynucleotide encoding galectin-9-binding molecule in a sample with an antibody that recognizes galectin-9-binding molecule; and
reagents for measuring or diagnosing the sensitivity to galectin-9, the degree of expectation of a physiological effect induced by galectin-9, or the degree of a physiological reaction relating to galectin-9, wherein the reagent comprises an antibody that recognizes galectin-9-binding molecule and is used for measuring the amount of galectin-9-binding molecule or the expression level of polynucleotide encoding galectin-9-binding molecule. Specific criteria for the diagnosis and measurement are cases wherein the subject polynucleotide level is 10% or more, preferably 30% or more, more preferably 70% or more, most preferably 100% or more greater than that of a control.

Two manufacturing methods of microarrays are known, i.e. a method of directly synthesizing oligonucleotides on a surface of a solid carrier (on-chip method) and a method of immobilizing oligonucleotides or polynucleotides that are synthesized in advance on a surface of a solid carrier. Both methods can be applied to manufacturing microarrays used in the present invention. In the on-chip method, it is possible to achieve a selective synthesis on a predetermined microscopic matrix region via using a protective group which can be selectively removed by light irradiation, and a combination of a photolithography technology used in semiconductor preparation and a solid-phase synthesis technology (masking technology: for example, Fodor, S.P.A.: Science 251, 767, 1991). In the immobilization of previously prepared oligonucleotides or polynucleotides on the surface of a solid carrier, a functional group is incorporated into synthesized oligonucleotides or polynucleotides and the resulting oligonucleotides or polynucleotides are spotted and attached to a surface-treated solid-carrier for covalent bond formation (for example, Lamture, J.B., et al.: Nucl. Acids Res., 22, 2121-2125, 1994; Guo, Z., et al.: Nucl. Acids Res., 22, 5456-5465, 1994).

In general, oligonucleotides or polynucleotides are covalently bound to a surface-treated solid carrier via a spacer or a cross-linker. A method for covalently binding synthetic oligonucleotides after arraying micro pieces of polyacrylamide gel on a glass surface is also known (Yershov, G., et al.: Proc. Natl. Acad. Sci. USA, 94, 4913, 1996). Known techniques for allowing rapid and strict hybridization include steps of preparing microelectrodes on a silicon Microarray, forming a penetration layer of agarose containing streptavidin on each electrode to provide reaction sites, positively charging such reaction sites to immobilize biotinized oligonucleotides, and controlling the charge of each site (Sosnowski, R.G., et al.: Proc. Natl. Acad. Sci. USA, 94, 1119-1123, 1997).

In diagnoses or assays with the microarray, cDNA is synthesized using mRNA isolated from, for example, cells of a subject as a template, and then is amplified by PCR. In this process, labeled dNTP is incorporated to produce labeled cDNA. The labeled cDNA is broght into contact with the microarray. Complementary DNA hybridized to a capture probe (oligonucleotide or polynucleotide) on the microarray is detected. The hybridization can be performed by dispensing an aqueous labeled cDNA solution to a 96-well or 384-well plastic plate of the microarray. The amount of the solution may range about 1 to 100 nL. Preferably, the hybridization is performed at the ranges from room temperature to 70°C for 6 to 20 hours. After the completion of hybridization, the plate is washed with a solution mixture of a surfactant and a buffer to remove unreacted labeled cDNA. Sodium dodecyl sulfate (SDS) is a preferable surfactant. Examples of the buffer include a citric acid buffer, a phosphoric acid buffer, a boric acid buffer, a tris buffer, and a Good's buffer. Preferably, the citric acid buffer is used.

The measurement of galectin-9-binding molecule polynucleotides can be performed by assaying the expression level of the polynucleotide (specifically, mRNA) with a primer set. Preferably, an RT-PCR method is used. Quantitative measurement by a competitive PCR method is also preferable. The primer set is designed based on known nucleotide sequences, and can be prepared through synthesis and purification processes. Similarly, the primers for competitive detection can be designed and synthesized based on a nucleotide sequence of the galectin-9-binding molecule gene. In designing the primers, for example, the followings are important: The primer size (the number of nucleotides) is 15 to 40 nucleotides, preferably, 15 to 30 nucleotides, for satisfactory specific annealing with a template DNA. However, in long accurate (LA) PCR, the use of at least 30 nucleotides is effective. A pair of (two) primers consisting of a sense strand (5'-terminal side) and an antisense strand (3'-terminal side) is avoided to have sequences complementary to each other so as not to anneal with each other. The primers are also avoided to have a self-complementary sequence so as not to form a hairpin structure within the primers. Furthermore, the primers should contain GC at a content of about 50% and should be avoided to have GC-rich or AT-rich regions, in order to secure a stable binding with the template DNA. Since the annealing temperature depends on the melting temperature (Tm), primers having Tm values of 55°C to 65°C and the values close to each other are selected so that highly specific PCR products are obtained. Furthermore, it is required to adjust the primer final concentration used in the PCR to about 0.1 to about 1 µM. Commercially available software for designing primers, for example, OligoTM (National Bioscience Inc., USA) and GENETYX (Software Development Co., Japan) can be used.

Gal-9 binding molecule-expressing genes (including DNA such as cDNA and RNA such as mRNA) can be detected and/or measured according to the aforementioned "gene recombination techniques" by adaptations of known methods for detection and/or measurement of specific gene expression in the art, such as in situ hybridization, Northern blotting, dot blotting, RNase protection assay, RT-PCR, Real-Time PCR (Journal of Molecular Endocrinology, 25, 169-193 (2000) and reference documents quoted therein), and DNA array analysis (Mark Shena (Ed.), "Microarray Biochip Technology", Eaton Publishing (March, 2000)) to achieve detection of Gal 9-related physiological events as disclosed herein and phenomena associated with biological actions exerted by Gal-9 binding molecules. Gal 9-binding molecule-expressing gene assay systems, apoptosis detection systems, assay systems for anti-tumor activity, and reagents, methods, processes, or analysis programs applied to said systems, which are utilizing such techniques, are all encompassed in the techniques and applied systems of the present invention. The in situ hybridization technique may include, for example, non-RI in situ hybridization. It may also include, for example, direct and indirect methods. The direct method is based on, for example, direct labels where a detectable molecule (reporter) is directly linked with a nucleic acid probe, whereas the indirect method is based on, for example, indirect ones where a signal is amplified using an antibody directed to a reporter molecule. Functional groups (e.g., primary aliphatic amino groups, SH groups, etc.) are incorporated into oligonucleotides in the nucleic acid probe, and may be coupled with haptens, fluorescent dyes, enzymes and the like. Representatives of labels for the nucleic acid probes include digoxigenin (DIG), biotin, fluorescein and the like. The labels as used herein can be suitably selected from those described in connection with the aforementioned antibodies. Multiple labeling can also be utilized. Further labeled antibodies can also be utilized. The labeling technique for nucleic acid probes can be suitably selected from methods known in the art, and include random prime, nick-translation, DNA amplification with PCR, labeling/tailing, in vitro transcription, and other methods. The treated samples can be observed with any technique suitably selected from those known in the art, including, for example, dark-field microscopy, phase-contrast microscopy, reflection-contrast microscopy, fluorescent microscopy, digital imaging microscopy, electron microscopy, and other techniques. Further, they can be observed with flow cytometry. In the present invention, galectin 9-binding molecule proteins and their related polypeptides or oligopeptides and galectin 9-binding molecule-expressing genes are utilized as various markers or anti-tumor markers, and carcinogenesis or cancer exacerbation markers, thereby enabling the production or construction of detection agents for a variety of pathological conditions and symptoms, or risk detection and/or risk assay agents, detection methods for galectin 9-related physiological phenomena, risk detection methods and/or risk assays, and further reagent kits or systems for such detections or risk-detections and/or risk assays. As a result, they are not only useful but also advantageous in diagnosis, prophylaxis, and therapy for various diseases and pathological conditions as disclosed herein. Furthermore, they can serve, in connection with post-treatment, i.e., prognosis, as reoccurrence-detecting agents or prognostic detection and/or assay reagents, detection methods, risk-detection methods and/or risk-assays for prognostication, and further reagent kits or systems for such detection and/or assays, wherein their targets are prognostic stages. Thus, it is expectable that they will function and act advantageously in view of prognosis.

Provided are assessments which comprise assaying biological samples from a subject for the presence and amount of galectin 9-binding proteins, and then, when the amount of galectin 9-binding proteins is larger than that in reference samples, judging the galectin 9-susceptibility, the galectin 9-inducible physiological activity expectancy, or the galectin 9-related physiological response occurrence to be high or significant. Embodiments of such assessment standards are those wherein the galectin 9-binding protein amount ratio of a test sample to a reference is 10% or more, preferably 30% or more, more preferably 70% or more, and most preferably 100% or more. The presence and amount of galectin 9-binding proteins can be measured with antibodies which recognize galectin 9-binding molecules or antibodies which bind specifically to galectin 9-binding molecules (anti-galectin 9-binding molecule Ab). Said antibody may be polyclonal or monoclonal, and includes all the entire molecule of antibodies which can bind to galectin 9-binding molecule protein epitopes, Fab, F(ab')₂, Fv fragments thereof, and other species.

The advantageously utilizable assay systems include protein assay systems for tissues, such as immunostaining, and immuno-electron microscopy; expression gene assay systems for tissues, such as in situ hybridization systems; protein assay systems for tissue extracts, such as EIA, RIA, FIA, LIA, and Western blotting; expression gene assay systems for tissue extracts, such as Northern blotting, Southern blotting, dot blotting, RNase protection assay, RT-PCR (reverse transcription polymerase chain reaction), Real-Time PCR, and competitive PCR; protein assay systems for blood samples and body fluids, and the like, such as EIA, RIA, FIA, LIA, and Western blotting; and others. For EIA systems, competitive assays employ, for example, anti-galectin 9-binding molecule Ab as solid-phased Ab, with labeled antigens and non-labeled antigens (the antigen includes galectin 9-binding molecules or peptide fragments thereof), while non-competitive assays do, in connection with sandwich assays, solid-phased anti-galectin 9 binding molecule Ab, and labeled anti-galectin 9 binding molecule Ab. Alternatively, the non-competitive sandwich assays can be conducted with anti-galectin 9 binding molecule Ab after directly labeling or without solid-phasing, and with Ab directed to anti-galectin 9-binding molecule Ab after labeling or solid-phasing. The performance-enhancing technique (amplification of sensitivity) includes, for example, non-enzyme-labeled primary antibody-based systems, including those utilizing polymers, enzymes and primary antibodies (applications of EnVision reagents; Enhanced polymer one-step staining (EPOS)); non-enzyme-labeled secondary antibody-based systems, including combinations of enzymes with anti-enzyme antibody conjugates such as PAP (peroxidase-antiperoxidase); combinations of biotinylated linking antibodies with biotin-labeled enzyme-avidin conjugates, such as SABC (avidin-biotinylated peroxidase complex); combinations of biotinylated linking antibodies with biotin-labeled enzyme-streptavidin conjugates, such as ABC (streptavidin-biotin complex) and LSAB (labeled streptavidin-biotin); combinations of SABC, biotinylated tyramide, and biotin-labeled streptavidin, such as CSA (catalyzed signal amplification); those in which secondary antibodies and enzymes are labeled with polymers; and others.

### Examples

Details of the present invention are described by the following examples but such examples are provided only for illustrative purposes, and for referential embodiments of the present invention. These examples have been described herein for the purpose of illustrating specific embodiments of the present invention but should in no way be construed as limiting and restricting the scope of the invention disclosed herein. It should be understood in the present invention that various embodiments can be made or executed within the spirit, scope and concept disclosed herein.

All the examples were carried out or can be carried out, unless otherwise disclosed herein specifically, by standard techniques which are well known and conventional to those skilled in the art.

### Example 1

### (1) Materials and method

### (a) Cell culture

MOLT-4 (T cell), Jurkat (T cell), BALL-1 (B cell), THP-1 (acute monocytic leukemia derived cell), and HL60 (acute promyelotic leukemia derived cell) cells were obtained from American Type Culture Collection (ATCC). All the cell lines were maintained in an RPMI-1640 medium (Sigma, St. Louis, US) supplemented with 10% FCS in 5% CO₂ at 37°C. Lactose (30 mM) was added to the culture medium to inhibit Gal-9 activity. The same concentration of sucrose was used as a control.

### (b) Expression and purification of recombinant Gal-9 (rGal-9)

Recombinant Gal-9 (rGal-9) was expressed and purified in the form of (His)₆-galectin-9 (short type; (His)₆-Gal-9(S)) in known methods (e.g. Matsushita, N. et al., J. Biol. Chem., 275: 8355 (2000); and Nishi, N. et al., Endocrinology, 141: 3194 (2000)). More specifically, E. coli BL-21 cells carrying the Gal-9 expression plasmid were cultured in an LB medium (Gibco BRL, Rockville, Maryland, US) containing 100 µg/mL ampicillin.

IPTG (isopropyl β-D-(-)-thiogalactopyranoside, Wako Pure Chemical Industries Ltd, Osaka, Japan) was added to induce the fusion protein expression. E. coli extracts were applied to lactose-agarose (SEIKAGAKU CORPORATION, Tokyo, Japan) affinity chromatography. Adsorbed proteins were eluted with 200 mM lactose. Elution fractions were collected, and separated by SDS polyacrylamide gel electrophoresis, followed by staining with coomassie brilliant blue R250 for analysis. Fractions containing rGal-9 were pooled, and dialyzed against PBS containing 0.1 mM dithiothreitol (DTT).

Construction of the wild type galectin-9 (galectin-9S, galectin-9M, and galectin-9L) expression vectors, and expression-purification of recombinant proteins were performed as described by M. Sato et al. (Glycobiology, Vol. 12, No.3, pp.191-197 (2002))

### (c) Apoptosis assay

### (1) Cell cycle (apoptosis) analysis with PI (PI method)

Cells that underwent apoptosis induction were centrifuged at 4°C for 5 min at 1,000 rpm, and the cell pellet was resuspended in PBS (300 µL), and adjusted to a final concentration of 70% by gradually adding 100% cold ethanol (700 µL) to the suspended cells with vortexing. Cells were fixed by incubation at 4°C for 30 min, and centrifuged at 4°C for 5 min at 1,000 rpm after adding PBS (1 mL), and the cell pellet was resuspended in PBS (440 µL). Cells were incubated with ribonuclease A (10 µL; the final concentration 50 µg/mL, Sigma, St. Louis, Missouri, US) at 37°C for 30 min, and with 2.5 mg/mL propidium iodide (4 µL; the final concentration of PI 20 µg/mL, Sigma) at 4°C for 10 min in the dark. Stained cells were analyzed by flow cytometry (Sandstrom, K. et al., J Immunol Methods, 240: 55 (2000) and Zhang L. et al., Cancer Lett, 142: 129 (1999)) after removing aggregated cells through a nylon mesh and increasing the cell volume with PBS.

### (2) TUNEL (TdT-mediated dUTP-biotin nick end labeling) assay

DNA fragmentation within the cell nucleus, a distinctive feature of apoptosis, was detected by incorporating labeled nucleotides (dUTP-biotin or FITC-dUTP, etc.) into the ends generated by DNA fragmentation with an enzyme that adds nucleotides at DNA ends (TdT; Terminal deoxynucleotidyl transferase).

MEBSTAIN Apoptosis Kit Direct (MBL, Nagoya, Japan) was used in the experiment. Experiments were carried out according to the manufacturer's instruction as described below. Briefly, cells that underwent apoptosis induction (approx. 2×10⁵ cells/sample) were washed with PBS containing 0.2% FSA, and fixed at 4°C for 30 min after adding 4% paraformaldehyde (0.1 M NaH₂PO₄, pH 7.4), and washed with PBS containing 0.2% FSA. The cell pellet was incubated at -20°C for 30 min after adding 70% cold ethanol to increase the permeability. After washing with PBS containing 0.2% FSA, TdT reaction mixture (mixture of TdT, FITC-dUTP and TdT buffer) was added and incubated at 37°C for 1 hr after mixing, and stained cells resuspended in PBS containing 0.2% FSA were analyzed by flow cytometry after washing with PBS containing 0.2% FSA.

### (3) Cultured cell lines were incubated with rGal-9 for the appropriate length of time, 24 hr for instance.

Apoptosis analysis was conducted by the above-described PI method (1).

MEBCYTO (registered trademark) Apoptosis kit (MBL, Nagoya, Japan) was used for apoptosis analysis with Annexin V-PI. Experiments were carried out according to the manufacturer's instruction as follows. Briefly, cells that underwent apoptosis induction (2×10⁵ cells/sample) were washed with PBS, and resuspended in the binding buffer. After adding Annexin V-FITC and 5 µL of PI (final concentration 5 µg/mL) in the kit to the cell suspension, cells were incubated for 15 min at room temperature in the dark. Analysis was conducted using flow cytometry (EPICS XL-MCL, CouHer, Miami, Florida, US).

Cells were incubated with Gal-9 in the presence of Z-VAD-FMK (pan-caspase inhibitor), Z-YVAD-FMK (caspase-1 inhibitor), Z-IETD-FMK (caspase-8 inhibitor), Z-LEHD-FMK (caspase-9 inhibitor), Z-AEVD-FMK (caspase-10 inhibitor), or Z-LLY-FMK (calpain inhibitor) (BioVision, CA, US), at 10 µM, and the participation of caspase or calpain in the Gal-9-induced apoptosis was examined. Z-FA-FMK (BioVision, CA, US) was used as a control (Vu C. C. et al., J. Biol. Chem., 276: 37602 (2001) and Sweeney E. A. et al., FEBS Lett., 425: 61 (1998)). Each inhibitor was added 1 hr before Gal-9 stimulation.

The following agents were purchased from suppliers below: DEX (BioVision, CA, US), anti-Fas Ab (clone CH-11, MBL), TNFα (Genzyme, Cambridge, MA, US), C₂ ceramide (SIGMA), and etoposide (BioVision).

### (d) T cell analysis

To prepare a well plate coated with anti-CD3 Ab, 24 well palates were incubated with TBS solution (pH8.0) containing 3 µg/mL anti-CD3 Ab (Immunotech, Marseille, France) per well at 4°C overnight, and each well was washed with PBS after removing anti-CD3 Ab solution.

Mononuclear leukocyte fractions were isolated from heparinized blood using HISTOPAQUE (Registered trademark, SIGMA). CD4-Positive Isolation Kit (DYNAL, Oslo, Norway) was used for isolation of CD4+ T cells and Dynabeads M-450 CD8 (Registered trademark, DYNAL, Oslo, Norway) for isolation of CD8+ T cells, respectively, according to the manufacturer's instructions.

To activate T cells, CD4+ or CD8+ cells at 1×10⁶ cells/mL in an RPMI-1640 medium containing 10% FCS were incubated on the plate coated with anti-CD3 Ab for 20 to 24 hr at 37°C in a 5% CO₂ incubator, and incubated with rGal-9 [ (His)₆-Gal-9(S)] at 37°C in a 5% CO₂ incubator. Then apoptosis assay was conducted as described in (c). That is, cells were incubated with 50 µg/mL PI (SIGMA) at 37°C for 10 min in the dark. Stained cells were analyzed by flow cytometry (Sandstrom, K. et al., J Immunol Methods, 240: 55 (2000) and Zhang L. et al., Cancer Lett, 142: 129 (1999)).

Apoptosis assay was also conducted for non-activated (resting) T cells as described above after incubating them with rGal-9 [(His)₆-Gal-9(S)] at 37°C in a 5% CO₂ incubator.

For comparison, galectin-1 and -3 treated cells were analyzed.

### (e) Ca²⁺ influx

Cells (approx. 10⁶ to 10⁷ cells/mL) in the culture medium (10% FCS, RPMI-1640 containing 10mM HEPES, pH 7.2) were incubated with fluo-3 AM (final concentration 10 µM, Dojindo, Kumamoto, Japan), an intracellular calcium indicator, at 37°C for 30 min (Sharp, B. M. et al., Proc. Natl. Acad. Sci. USA, 93: 8294 (1996)). Then the cells were washed and resuspended in the culture medium (final concentration, approx. 2×10⁵ cells/mL per sample). Intracellular calcium level was measured by flow cytometry. The resting cells were stimulated at 1 min after their fluorescence intensities were measured.

Next, sequential measurement was restarted until different stimuli were applied. In lactose inhibition assay, cells were stimulated in the presence of 30 mM lactose. Sucrose (30 mM) was used as a control (Sano, H. et al., J. Immunol., 165: 2156 (2000)). A23187 (calcium ionophore, final concentration 5 ng/mL, Wako, Osaka, Japan) was used as a positive control.

### [Results]

### (1) Apoptosis induction by Gal-9

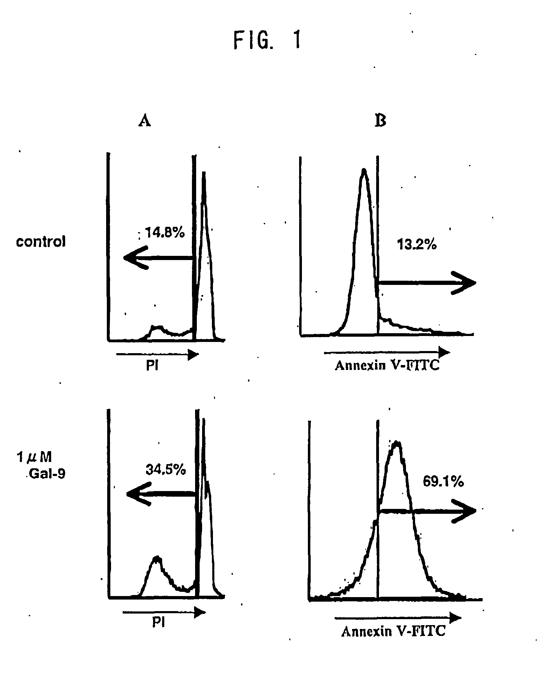

Apoptosis induction by Gal-9 was examined in each cell line. The result was that MOLT-4 cells incubated with rGal-9 were stained by PI. The stained cells were counted as cells that underwent apoptosis. As shown in Fig. 1A, rGal-9 (1 µM) induced apoptosis in MOLT-4 cells (PI-stained cells, which underwent apoptosis, are at 14.8% for control cells treated with PBS, while at 34.5% for cells treated with Gal-9). Such apoptosis induction by rGal-9 was time-dependent and dose-dependent. Apoptosis detected by the PI method increased with higher concentrations of rGal-9 and longer incubation periods (for example, 0.3 µM rGal-9 + 24 hr incubation period or 1 µM rGal-9 + 6 hr incubation period).

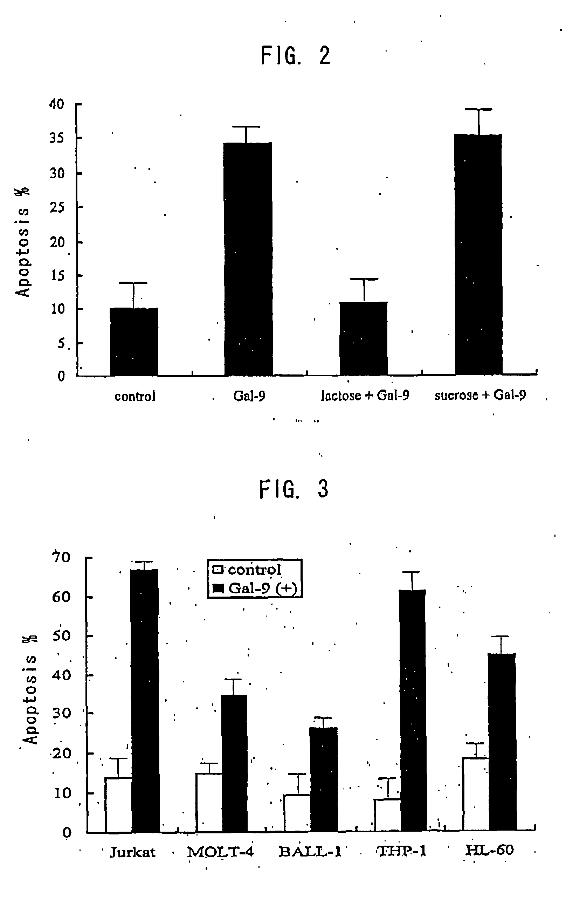

Since phosphatidylserine generated during apoptosis was detectable by staining, pro-apoptotic activity of Gal-9 was examined using Annexin V staining. Cells that underwent apoptosis and were detectable by Annexin V staining, increased from 13.2% for the control to 69.1% for 1 µM Gal-9 (Fig. 1B). This demonstrated that Gal-9 had pro-apoptotic activity in MOLT-4 cells. Then effects of lactose on apoptosis induction by Gal-9 were examined, and as shown in Fig. 2, pro-apoptosis activity by Gal-9 was almost completely inhibited with 30 mM lactose but not with sucrose. This indicated that Gal-9-induced apoptosis required β-galactoside binding activity.

### (2) Apoptosis induction by Gal-9 in various cells

Pro-apoptotic activities of Gal-9 in other cell lines were examined. That is, cells were cultured in the presence or absence of rGal-9 at 1 µM for 24 hr to examine the apoptosis induction. As shown in Fig. 3, apoptosis was induced by Gal-9 not only in T cells such as Jurkat and MOLT-4 cells but in B cells (BALL-1), monocytic cells (THP-1), and bone marrow cells (HL60). Meanwhile, black-filled bars in Fig. 3 indicate the Gal-9 treated group and white-filled bars do the control group, respectively. In addition, pro-apoptotic activity of Gal-9 was inhibited by lactose but not by sucrose.

Next, apoptosis induction by Gal-9 in human peripheral blood T cells was examined. T cells were separated into CD4+ and CD8+ T cells followed by incubation in the presence or absence of anti-CD3 Ab. Resting T cells and anti-CD3 Ab-activated T cells were incubated with 1 µM rGal-9. The results are shown in Table 1.

**Table 1**

| CD3 | Galectin-9 | CD4 | CD8 |
|---|---|---|---|
| (-) | (-) | 6.2 | 7.7 |
| (-) | (+) | 10.8 | 17.8 |
| (+) | (-) | 8 | 8.5 |
| (+) | (+) | 41.7 | 25.7 |
| | | | |

| CD3 | Galectin-1 | CD4 | CD8 |
|---|---|---|---|
| (-) | (-) | 6.2 | 7.7 |
| (-) | (+) | 8.1 | 9.6 |
| (+) | (-) | 8 | 8.5 |
| (+) | (+) | 13.9 | 19 |
| | | | |

| CD3 | Galectin-3 | CD4 | CD8 |
|---|---|---|---|
| (-) | (-) | 6.2 | 7.7 |
| (-) | (+) | 9.6 | 14.7 |
| (+) | (-) | 8 | 8.5 |
| (+) | (+) | 15.7 | 20.9 |

In the Table 1, CD3 (+) stands for the activated cells treated on plates coated with anti-CD3 Ab, and CD3 (-) does for the untreated resting cells, respectively. The mark (+) under each galectin stands for treatment in the presence of gelatin and (-) under each galectin does for treatment in the absence of gelatin, respectively. CD4 stands for CD4+ T cells and CD8 does for CD8+ T cells, respectively.

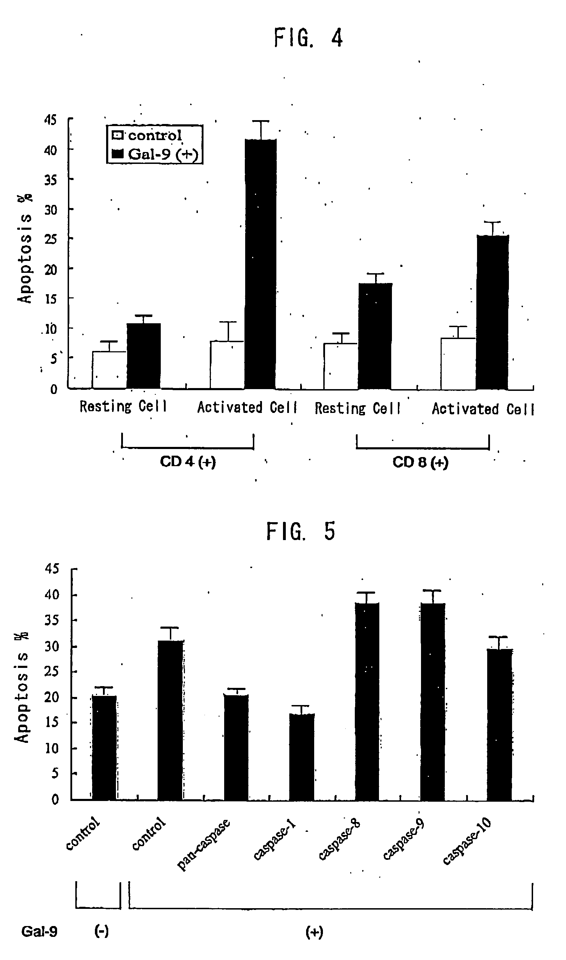

In Fig. 4, higher apoptosis induction by Gal-9 was evident in both CD3-activated CD4+ T cells and CD3-activated CD8+ T cells, compared with resting CD4+ and CD8+ T cells. Meanwhile, black-filled bars in Fig. 4 stand for the group treated in the presence of Gal-9 and white filled bars in Fig. 4 do for the control group, respectively. In addition, higher sensitivity to Gal-9 was verified in CD4+ T cells compared with CD8+ T cells (Fig. 4).

### (3) Caspase inhibition

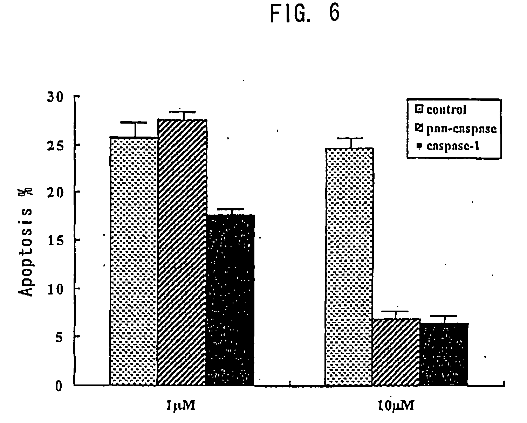

Participation of the caspase activation pathway in Gal-9-induced apoptosis was examined. First, MOLT-4 cells were incubated with Z-VAD-FMK, a pan-caspase inhibitor, and then with rGal-9. Gal-9-induced apoptosis was almost completely inhibited with this pan-caspase inhibitor (Fig. 5 and Fig. 6). For example, inhibition of apoptosis induced by stimuli such as dexamethasone (DEX), anti-Fas Ab, and TNF-α, C2 ceramide was observed with this pan-caspase inhibitor.

Experiments were conducted using various caspase inhibitors; Z-YVAD-FMK for caspase-1, Z-IETD-FMK for caspase-8, Z-LEHD-FMK for caspase-9, and Z-AEVD-FMK for caspase-10.

The results are shown in Fig. 5. Gal-9-induced apoptosis was inhibited only by Z-YVAD-FMK (caspase-1 inhibitor), but not by the other inhibitors. At the same time, it was found that the said caspase-1 inhibitor inhibited DEX-induced apoptosis, the said caspase-8 and caspase-10 inhibitors did anti-Fas Ab-induced apoptosis and TNF-α-induced apoptosis, and the said caspase-9 inhibitor did C2 ceramide-induced apoptosis, respectively. In addition, as shown in Fig. 6, both pan-caspase and caspase-1 inhibitors inhibited Gal-9 induced apoptosis in a dose-dependent manner. These inhibitory phenomena were also observed in experiments using other cell lines. Based on these results, Gal-9 is presumed to induce apoptosis via caspase-1, but not via any other caspase pathways.

### (4) Calcium-calpain pathway

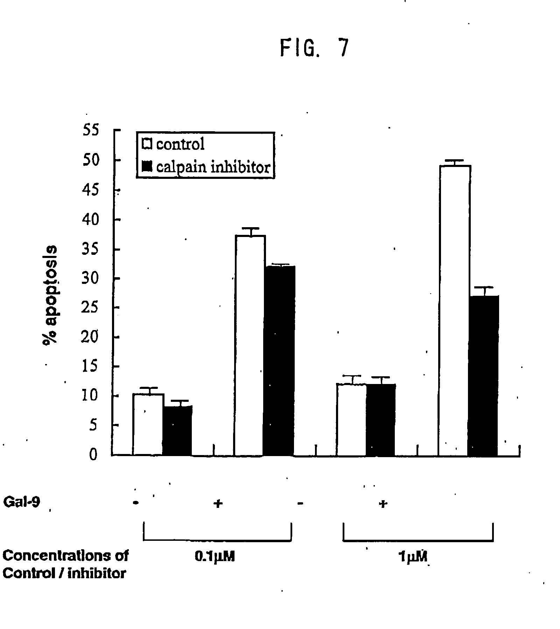

Since caspase-1 requires a calcium dependent protease calpain for its activation, the association of the calpain-caspase-1 activation pathway with Gal-9-induced apoptosis in MOLT-4 cells was examined. As shown in Fig. 7, Z-LLY-FMK, a calpain inhibitor, inhibited the Gal-9-induced apoptosis in a dose dependent manner.

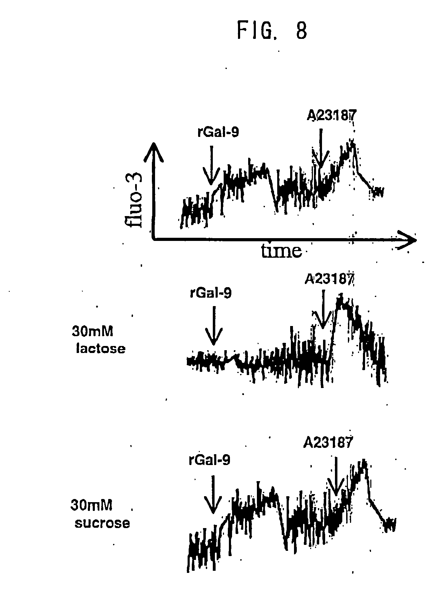

In addition, Fluo-3 assay was conducted to examine the induction of calcium influx by Gal-9 in the MOLT-4 cells. As shown in Fig. 8, addition of Gal-9 increased calcium influx in the MOLT-4 cells within 10-20 sec, and lactose inhibited the calcium influx induced by Gal-9. Meanwhile, the Gal-9 induced calcium influx was not inhibited with sucrose. Calcium influx was significantly induced in MOLT-4 cells with A23187 (final concentration: 5 ng/mL), which was used as a control (Fig. 8).

Also in other cells, involvement of calpain and calcium influx in Gal-9-induced apoptosis could be confirmed.

### (5) Effects of Gal-9 on DEX-, anti-Fas Ab-, or etoposide-induced apoptosis

Effects of Gal-9 on the apoptosis induced by other stimuli (e.g. DEX, anti-Fas Ab, and etoposide) were examined.

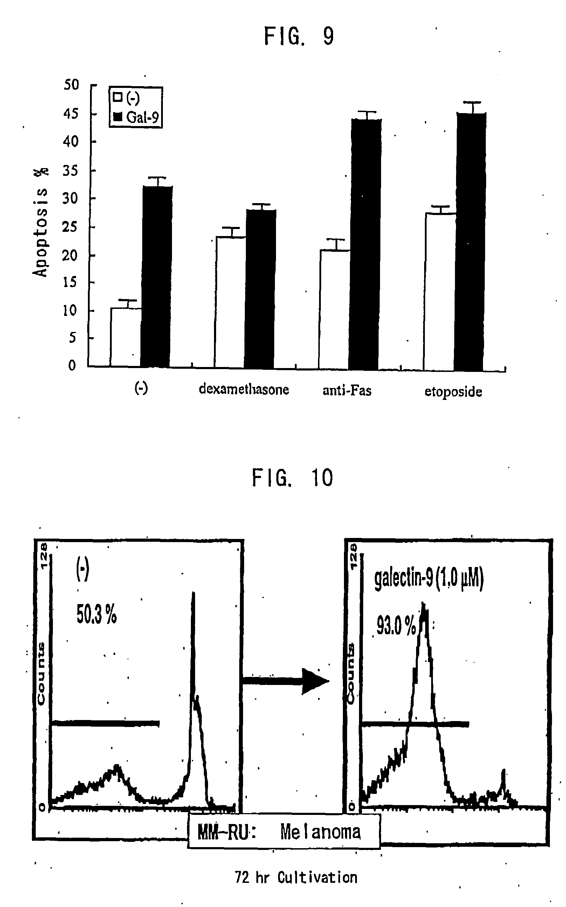

All the stimuli including rGal-9, DEX, anti-Fas Ab, and etoposide, induced apoptosis in MOLT-4 cells significantly (Fig. 9).

When MOLT-4 cells were incubated with rGal-9 or DEX, scarcely little or no difference in the percentage (%) of apoptotic cells was observed. Meanwhile, rGal-9 showed additional effects on anti-Fas Ab-induced and etoposide-induced apoptosis (Fig. 9). These results indicated a different apoptosis pathway induced by Gal-9 from those by other stimuli other than DEX.

As described above, Gal-9 has been found to induce apoptosis in various cells, especially in all the tested immune cells (Fig. 3 and Fig. 4). This indicates the function of Gal-9 as a factor with pro-apoptotic activity in many cell types.

Each apoptotic pathway was analyzed for other stimuli such as glucocorticoid (GC), anti-Fas Ab, and oxidative stress. For example, anti-Fas Ab binds to a Fas ligand and induces apoptosis via caspase-8 activation. Etoposide impairs DNA, releases mitochondrial cytochrome C, activates caspase-9, and ultimately induces apoptosis (Sun X. M. et al., J. Biol. Chem., 274: 5053 (1999)). DEX activates calcium-dependent endonuclease and caspase-1, and induces apoptosis (Cheneval, D. et al., J. Biol. Chem., 273: 17846 (1998); McConkey, D. J., J. Biol. Chem., 271: 22398 (1996) and Cohen, J. J. et al., J Immunol., 132: 38 (1984)). GC induces apoptosis in many cell types as well as in T cells (Dobashi H. et al., FASEB, 15: 1861 (2001) and Nittoh, T. et al., Eur. J. Pharmacol., 354: 73 (1998)). Analysis of its mechanism demonstrated calcium-dependent apoptosis induced by GC or caspase-1 in thymocytes (McConkey, D. J., J. Biol. Chem., 271: 22398 (1996) and Cohen, J. J. et al., J Immunol., 132: 38 (1984)).

In the present invention, Gal-9 has been found to induce apoptosis in the wake of Ca²⁺ influx (Fig. 8), and calpain participation (Fig. 7) and caspase-1 activation (Fig. 5 and Fig. 6), indicating the apoptosis induction by Gal-9 via the calcium/calpain/caspase-1 pathway like GC.

It has been demonstrated that calcium induces apoptosis in U937 cells via both the calcium/calpain pathway and mitochondrial pathway (Li M. et al., J. Biol. Chem., 275: 39702 (2000)). In addition, Gal-9 increases apoptosis induced by anti-Fas Ab or etoposide.

Gal-1 gene is overexpressed during apoptosis induced by GC (Goldstone S. D. et al., Biochem. Biophys. Res. Commun., 178: 746 (1991)). In addition, Gal-1 induces Ca²⁺ influx and apoptosis in Jurkat cells (Walzel, H. et al., Glycobiology, 10: 131 (2000)). These indicate that the calcium/calpain/caspase-1 pathway is at least partially participating in Gal-1 mediated apoptosis. In fact, Gal-1-induced apoptosis does not require an increased intracellular calcium level (Pace, K. E. et al., J Immunol., 165: 2331 (2000)). Meanwhile, Gal-7 seems to be a galectin with JNK activity and pro-apoptotic activity that intracellularly functions upstream for the release of cytochrome C (Kuwabara, I. et al., J. Biol. Chem., 277: 3487 (2002) and Bernerd, F. et al., Proc. Natl. Acad. Sci. USA, 96: 11329 (1999)). This indicates that pro-apoptotic activation pathways of Gal-7 are different from those of Gal-1 and Gal-9.

The inventor et al. have heretofore discovered that Gal-9 binds to the surface of the tested cells. The relevant receptors relocate into the nucleus after being bound with GC in the cytoplasm (Galigniana, M. D. et al., J. Biol. Chem., 274: 16222 (1999) and Guiochon-Mantel, A. et al., EMBO J., 10: 3851 (1991)). Meanwhile, Gal-9 is presumed to bind to the bound molecules on the cell surface and induce Ca²⁺ influx, calpain/caspase-1 activation, and apoptosis of the cell. Gal-9 receptors and their location are different from those of DEX, but Gal-9 is presumed to induce apoptosis via an apoptosis pathway similar to that of GC. In fact, Gal-9 shows additional effects on both anti-Fas Ab- and etoposide-induced apoptosis, but has little or no effect on the DEX-induced apoptosis (Fig. 9). Also, they have found that Gal-9 stimulates the anti-Fas Ab-induced apoptosis, but inhibits the DEX-induced apoptosis of eosinophils.

It has been found that DEX induces apoptosis in both CD4+ and CD8+ T cells in rats (Tsuchiyama, Y. et al., Kidney Int., 58: 1941 (2000)), and in human T cells (Dobashi H. et al., FASEB, 15: 1861 (2001)). In addition, Gal-9 induces apoptosis selectively in rat spleen CD8+ T cells activated by kidney inflammation (Tsuchiyama, Y. et al., Kidney Int., 58: 1941 (2000)).

In the present invention, it has been found that Gal-9 is more likely to induce apoptosis in activated CD4+ T cells (helper T cells) than in activated CD8+ T cells (suppressor T cells and cytotoxic T cells), which are derived from human peripheral blood cells (Fig. 4). This suggests the possibility that Gal-9 functions as an immunosuppressive factor. Thereby, Gal-9 seems to play an important role in immune regulation by inducing apoptosis in various immune cells such as CD4+ T cells, along with activation of eosinophils.

### Example 2

### (1) Cytotoxicity of galectin-9

Cytotoxicities in Jurkat T cells, K-562 leukemic cells, and normal lymphocytes were measured by flow cytometry analysis using PI. That is, PI invades into the damaged target cells when PI is added for the last 15 min of 16 hr incubation with 1 mM galectin-9. Fluorescence generated by PI is measured by FACS. Untreated cells were used as a negative control group, formalin-fixed cells as a 100% dead cell group, and H₂O₂ (hydrogen peroxide solution)-treated cells as a positive control group, respectively. As shown in Table 2, galectin-9 showed obvious cytotoxicities in Jurkat cell line and K-562 cells, but not in normal lymphocytes.

**Table 2**

| | Stimulus | 16 hours |
|---|---|---|
| Jurkat | (-) | 1.02 |
| | Galectin-9 | 46.2 |
| | H₂O₂ | 97.7 |
| K-562 | (-) | 0.73 |
| | Galectin-9 | 23.4 |
| | H₂O₂ | 99.2 |
| Normal Lymphocyte | (-) | 1.12 |
| | Galectin-9 | 2.47 |
| | H₂O₂ | 96.8 |

### (2) Cancer cell apoptosis induced by galectin-9

Apoptotic activity was measured by the PI method. When cells are incubated with galectin-9, washed, then subjected to alcohol-fixation, and fragmented DNAs flow out of the cell. Subsequently, incubation with PI leads to a reduction in the fluorescence intensity of apoptotic cells. Apoptosis was examined by the percentage of the cells wherein their fluorescence intensity was reduced. As shown in Fig. 10, apoptosis was strongly induced in malignant melanoma cell line MM-RU, which was incubated with galectin-9 for 72 hr, indicating the induction of cancer cell apoptosis with galectin-9. This induction was at the same level as H₂O₂ (hydrogen peroxide solution) that was used as a positive control. In addition, the same results were obtained by another method for examining apoptosis, TUNEL assay. Next, results of the study on the apoptosis induction with Gal-9 in other malignant tumor cells are shown in Table 3.

**Table 3**

| Cell Lines | Gal9 (-) | Gal9 (+) |
|---|---|---|
| Jurkat | 13.8 | 66.6 |
| MOLT4 | 14.8 | 34.5 |
| BALL-1 | 9.2 | 26.0 |
| THP-1 | 8.3 | 61.2 |
| HL-60 | 38.1 | 64.6 |
| MCF-7 | 7.5 | 14.1 |
| KATO-III | 1.0 | 30.5 |
| Daudi | 59.7 | 58.5 |
| HMC-1 | 27.0 | 26.2 |

In Table 3, Jurkat and MOLT-4 cells are T cell derived malignant cell lines, BALL-1 cells are B cell derived malignant cell lines, THP-1 cells are acute monocytic leukemia derived cell lines, HL60 cells are acute promyelotic leukemia derived cell lines, MM-RU cells are malignant melanoma cell lines, MCF-7 cells are breast cancer cell lines, and KATO-III cells are stomach cancer cell lines. Incubation in the presence of galectin-9 was carried out for at least 48 hr or more. In Table 3, apoptosis induction with galectin-9 can be verified in both non-epithelial and epithelial malignant cells. No apoptosis induction can be observed in Daudi cells (B cells) and HMC-1 (mast cells). These results suggest the existence of binding factors on the cell surface, which are associated with apoptotic signaling, but not with galectin-9 binding factors. It is possible to identify galectin-9 binding molecules that are involved in apoptosis and to develop techniques to inhibit tumor growth using these factors.

Galectin-9 exhibits antitumor activity in all the epithelial malignant tumors (cancer) and non-epithelial malignant tumors (sarcoma and leukemia and others). However, apoptosis induction is scarcely exhibited in non-activated normal T cells. For example, measurements of apoptosis induction in the presence of 1 µM galectin-9 are as shown in Table 4 below (also 1 µM for galectin-3 in Table 4).

**Table 4**

| | CD4 T Cell | CD8 T Cell |
|---|---|---|
| | Resting | Resting |
| (-) | 0.8 | 0.8 |
| Anti-Fas | 15.9 | 8.3 |
| Galectin-9 | 1.8 | 1.4 |
| Galectin-3 | 10.3 | 15.9 |

Based on these results, galectin-9 proteins, galectin-9 agonists, antagonists toward galectin-9 antagonists, anti-galectin-9 binding protein antibodies, anti-galectin-9 binding saccharide chain antibodies, galectin-9 production/release inducers can be used as antitumor agents (anticancer drugs), which exhibit cytotoxicity in tumor cells and induce apoptosis but not in normal cells.

### Example 3

### (1) Antimetastatic activity by galectin-9

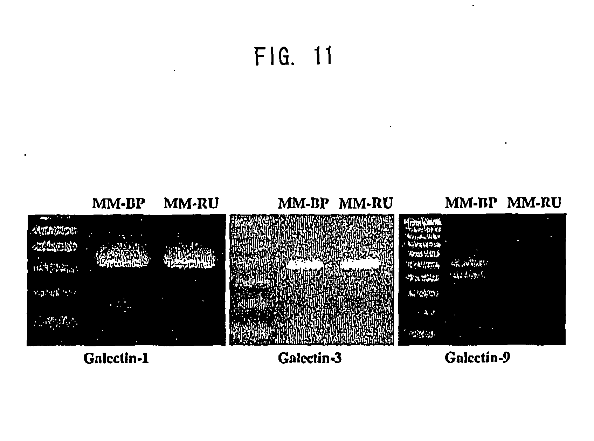

out of human melanoma cell lines, when MM-BP and MM-RU cells are cultured, the former cells will aggregate and proliferate in a colony-forming manner, while the latter cells will proliferate without colony formation. The amount of galectin DNA was analyzed by RT-PCR method using mRNA extracted from human melanoma cell lines, MM-BP and MM-RU. As a result, no difference of galectin-1 and galectin-3 amounts between MM-BP and MM-RU was observed, but galectin-9 was strongly expressed only in colony-forming MM-BP cells and very weakly in MM-RU cells that proliferated without colony formation (Fig. 11).

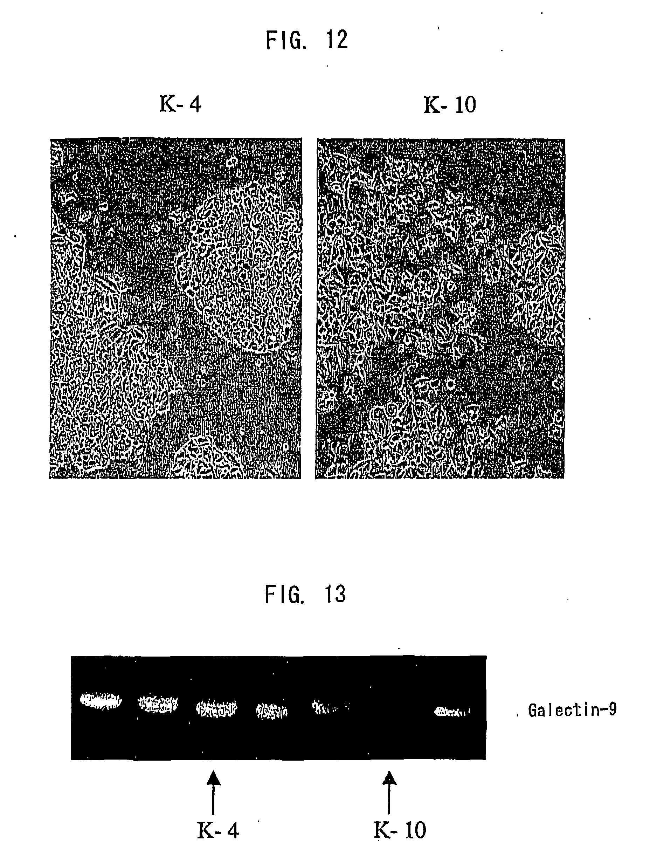

Although breast cancer cell line, MCF-7, is a colony-forming cell, MCF-7 subclones have been prepared by limiting dilution and MCF-7 K-10 cell line has been established, which exhibits no obvious colony formation (Fig. 12). Galectin-9 was detected by Western blotting in MCF-7, but not in MCF-7 K-10 (Fig. 13). Galectin-9 in MCF-7 cells was mainly detected in cytoplasm by immunostaining.

RT-PCR was performed according to the method described by T. Kageshita et al. (Int. J. Cancer, 99: 809-816 (2002)).

MCF-7 cells were cultured in DMEM supplemented with glutamate, 10% fetal bovine serum, penicillin, and streptomycin (ICN Biomedicals, Aurora, OH, USA) in a 5% CO₂/37°C incubator.

Western blot analysis was conducted as follows: approximately 10⁶ cells were prepared, and their cell membranes were destroyed by treating with a lysis buffer containing 150 mM NaCl, 50 mM Tris-HCl, pH7.5, 0.5% Nonidet P-40 (Boehringer-Mannheim, GmbH, Germany), 1 mM PMSF (ICN biomedicals), 50 TIU aprotinin (Wako, Osaka, Japan), and 50 mg/mL Leupeptin (Calbiochem, San Diego, CA, USA). The obtained supernatant was boiled with a 2x sample buffer (125 mM Tris-HCl, pH6.8, 20% glycerol, 2% 2-mercaptoethanol, 4% SDS, 0.02% Bromophenol Blue) for 5 min, and separated through 5-15% gradient gel using SDS PAGE mini-gel system (Bio-Rad, Richmond, CA, USA). The separated proteins were transferred onto a PVDF membrane (Millipore, Bedford, MA, USA), and incubated with 2 µg/mL anti-galectin-9 Ab (α-galectin-9 CT) solution (5 mL) after blocking with 5% skim milk containing 0.05% Tween 20 (Sigma, St. Louis, MO, US) for 1 hr. Then bands were visualized by the ECL system (Amarsham) after treatment with horseradish peroxidase conjugated goat anti-rabbit IgG Ab (Amarsham, Buckinghamshire, UK).

Immunostaining was performed as follows:

Immunohistochemical staining of formalin-fixed, paraffin-embedded tissue sections with polyclonal anti-galectin-9 Ab (α-galectin-9 CT) was performed using DAKO EnVision+™ Peroxidase Rabbit System according to the manufacturer's instructions. Formalin-fixed, paraffin-embedded tissue sections (4 µm thickness) were boiled in a citric acid antigen recovery buffer (10 mM) for 16 min before de-paraffinization and re-hydration. After inactivating endogenous peroxidase by treatment with 0.3% peroxide, the said sections were treated with 5% bovine serum albumin (BSA) for 2 hr at room temperature to block non-specific staining. Said tissue sections were incubated with primary antibodies at room temperature overnight, and incubated with Envision+™ solution containing anti-rabbit IgG antibodies and horseradish peroxidase at room temperature for 1 hr (both were bound with polymer). The coloring agent used herein is 3, 3'-diaminobenzidine tetrahydrochloride. An immunoglobulin fraction (DAKO) obtained from pre-immunized rabbit serum was used as a negative control. All the sections were subjected counterstaining with Giemsa solution, and the percent stained tumor cells for each section were measured by 2 observers independently and those with 50% or more staining were judged positive.

### (2) Induction of cell aggregation by galectin-9

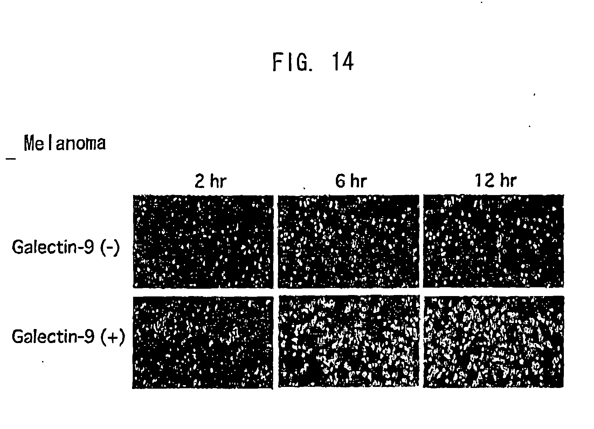

Recombinant galectin-9 prepared via gene transfer into E. coli was added to MM-RU exogenously, and the efficacy of galectin-9 was then examined. As a result, galectin-9 induced aggregation of MM-RU at the concentration of 0.1 µM or higher. In addition, the galectin-9 efficacy was concentration-dependent, and significant between 0.3-1.0µM. In addition, aggregation was observed at 2 hr after addition of galectin-9, and more intensive aggregation at 12 hr (Fig. 14). No obvious aggregation was induced by galectin-1 and galectin-3, which were used as controls, at the dose of at least 1 µM.

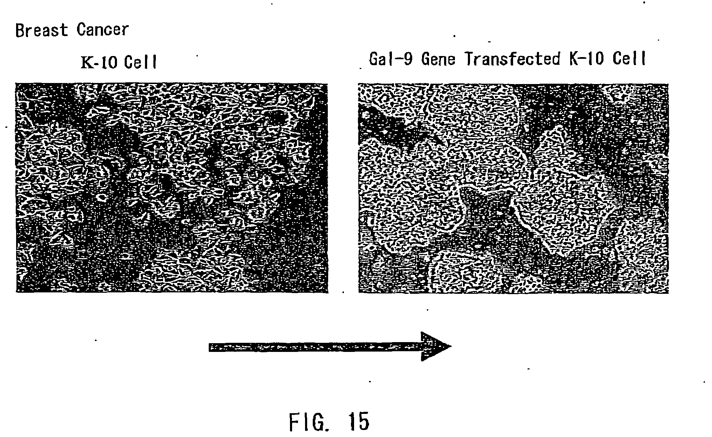

When a galectin-9 gene was introduced into an MCF-7 K-10 cell line established from a breast cancer cell line, the cells had galectin-9 in the cytoplasm, and proliferated with obvious colony formation upon cultivation (Fig. 15).

Construction of expression vectors for three human galectin-9 types, namely galectin-9S, 9M, and 9L, was carried out by inserting cDNA containing each full-length galectin coding region and 7 nucleotides added at the upstream of the initiation codon into the EcoRI-XhoI site of pBK-CMV (Stratagene). Incorporation of galectin-9 genes into the cells was performed using Fugene 6 Transfection Reagent (Roche Molecular Medicals) according to the manufacturer's protocol. Experiments were conducted using the MCF-7 K-10 cell line transfected with pBK-CMV plasmid alone or the plasmid carrying human galectin-9 cDNA. Cells were selectively treated with 800 µg/mL G418 for 2 weeks.

Thus, it is evident that galectin-9 acts on metastatic malignant cells with aggregation induction and is metastasis-inhibitory against malignant tumors such as cancers, so galectin-9 will serve as a cancer metastasis inhibitor. Based on these facts, cancer metastasis inhibitory actions can be attained by incorporation of galectin-9 proteins or derivatives thereof, galectin-9 gene transfers, or induction of galectin-9 production/release, as well as even cancer metastasis inhibitors can be provided.

It is noteworthy that human galectin-9 exhibits cytotoxicity in tumor cells, but not in normal cells. In addition, human galectin-9 induces apoptosis in tumor cells, but not in normal cells. Also, human galectin-9 induces apoptosis in activated T cells, but not in non-activated T cells. Based on these results, it is evident that human galectin-9 is useful as an antitumor agent, antiallergic agent, immunosuppressive agent, anti-autoimmune disease agent, antiinflammatory agent and/or alternate agent for adrenal steroid cortical hormones. In the present invention, the efficacy of human galectin-9 on apoptosis induced by DEX, anti-Fas Ab, or etoposide, was verified, thereby galectin-9 was confirmed as extremely useful for the above applications.

### Example 4

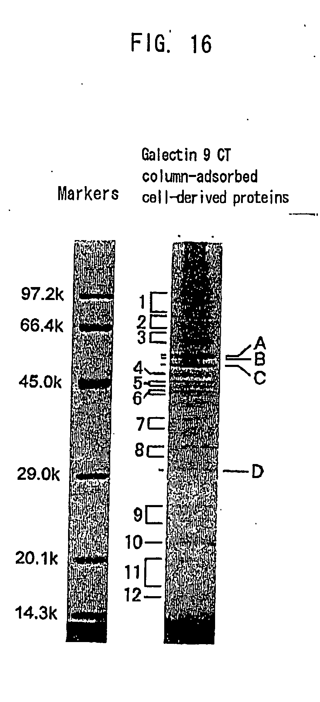

MOLT-4 cells (human T cells) (approx. 7.25×10⁹ cells, wet weight: approx. 18 g) were disrupted by repeated freeze-thaw. The resultant disrupted cells were washed (200 mM lactose (lac) containing solution), and the disrupted cell solution was homogenized. A pellet was obtained by centrifugation. The pellet was solubilized using a detergent. Mainly Triton (product name) was used as the detergent to solubilize the pellet. A supernatant was obtained after centrifugation. Then the obtained supernatant was applied to a GST column (nonspecific adsorption column). Fractions run through the column were collected, and then applied to a GST gal9CT column for specific galectin-9 adsorption. After washing the column, a 200 mM lactose (lac) containing solution was applied to the column to unbind the galectin 9CT and cellular proteins. SDS PAGE results of cell (MOLT-4)-derived proteins adsorbed on the ga19CT column are shown in Fig. 16.

Each of fractions 1 to 12, A, B, C, and D (shown in Fig. 16) was analyzed for ingredient proteins. The analysis was conducted by designated APRO Life Science Institute, Inc., Seto-cho, Naruto-shi, Tokushima, Japan. Candidates were identified by internal protein sequence analysis for the bands A, B, C, and D, and by mass spectrometry (LC-MS/MS) for the bands 1 to 12. First, in the internal protein sequence analysis, samples isolated from the above SDS PAGE bands were subjected to limited degradation (e.g. gel digestion) with protease. When the amount of the resultant samples was less than 1 pmol, amino-acid sequencing was conducted with a sequence analyzer after peptide separation by reverse HPLC (peptide mapping). When less than 0.1 pmol, a database search was conducted after analysis by mass spectrometry (LC-MS/MS). Said proteins were scored by the Mascot search, and those with high scores (the threshold or higher) were selected as candidates. Further information on the Mascot search is available from Matrix Science (http://www.matrixscience.
com/). Mascot is produced based on the Mowse algorithm (D.J.C. Pappin, et al., Curr. Biol., 3, 327-332 (1993)).

The utilizable amino-acid sequence analyzers include Procise 494HT (Applied Biosystems), Hewlett-Packard G1005A, Shimazu PSQ-1 (Gradient System), and Procise 494cLC (Applied Biosystems) and others. The available databases include NCBInr (Protein) and dbEST (DNA) and others. The LC-MS/MS instruments are Q-TOF2 & Capillary HPLC and others. Also, in some cases, MALDI-TOF MS analysis can be conducted, and MS-Fit search is also available. Voyager-DE STR and others can be used for MALDI-TOF MS analysis.

### <Results>

In mass spectrometry (LC-MS/MS), value data for product ions obtained from all precursor ions were retrieved in the database (NCBInr) intended for all entries by Mascot. In the case of no hit with a significant score from the NCBInr database, provided that digestive enzymes and human keratin were excluded, we searched for the dbEST database (intended for human, mouse and others).

The hit proteins with significant scores are listed herein below depending on each hit number in the Mascot Search Results. When there are plural hit proteins with an identical hit number, only representatives are described herein (wherein species identical with sample species are predominantly selected), and when the hits are digestive enzymes or human keratin (derived from contaminants due to operations), their data are eliminated. Protein information data as well as coding gene information data are attained when an entry of the number behind "gi |" is made at the NCBI internet home page (http://www.ncbi.nlm.nih.gov/).

| Band 1 | |
|---|---|
| [NCBInr (all entries)] significant when over score 47 | |
| (1) Protein Name | 4F2 heavy chain antigen |
| Source | human |
| Total score | 153 |
| Peptides matched | 5 |
| GenInfo Identifier or Accession.version number | gi\|177216 |
| Sequence coverage | 12% |

| (2) Protein Name | ATPase, Na⁺/K⁺ transporting, alpha 1 polypeptide |
|---|---|
| Source | human |
| Total score | 150 |
| Peptides matched | 4 |
| GenInfo Identifier or Accession.version number | gi\|21361181 |
| Sequence coverage | 5% |

| (3) Protein Name | sodium-dependent neutral amino acid transporter type 2 truncated isoform |
|---|---|
| Source | human |
| Total score | 134 |
| Peptides matched | 4 |
| GenInfo Identifier or Accession.version number | gi\|15004317 |
| Sequence coverage | 8% |

| (5) Protein Name | stromal cell derived factor receptor 1 isoform a |
|---|---|
| Source | human |
| Total score | 57 |
| Peptides matched | 1 |
| GenInfo Identifier or Accession.version number | gi\|9257240 |
| Sequence coverage | 3% |

| (6) Protein Name | heat shock 90kDa protein 1, beta |
|---|---|
| Source | human |
| Total score | 46 |
| Peptides matched | 1 |
| GenInfo Identifier or Accession.version number | gi\|20149594 |
| Sequence coverage | 3% |

| Band 2 | |
|---|---|
| [NCBInr (all entries)] significant when over score 47 | |
| (1) Protein Name | heat shock 70kDa protein 5 (glucose-regulated protein, 78kDa) |
| Source | human |
| Total score | 227 |
| Peptides matched | 8 |
| GenInfo Identifier or Accession.version number | gi\|16507237 |
| Sequence coverage | 21% |

| (3) Protein Name | heat shock 70kDa protein 8 isoform 2 |
|---|---|
| Source | human |
| Total score | 192 |
| Peptides matched | 5 |
| GenInfo Identifier or Accession.version number | gi\|24234686 |
| Sequence coverage | 20% |

| (4) Protein Name | heat shock 70kDa protein 9B precursor |
|---|---|
| Source | human |
| Total score | 182 |
| Peptides matched | 3 |
| GenInfo Identifier or Accession.version number | gi\|24234688 |
| Sequence coverage | 6% |

| (16) Protein Name | fatty-acid-Coenzyme A ligase, long-chain 3 |
|---|---|
| Source | human |
| Total score | 64 |
| Peptides matched | 4 |
| GenInfo Identifier or Accession.version number | gi\|27469830 |
| Sequence coverage | 6% |

| (21) Protein Name | NADH dehydrogenase (ubiquinone) Fe-S protein 1, 75kDa (NADH-coenzyme Q reductase) |
|---|---|
| Source | human |
| Total score | 55 |
| Peptides matched | 2 |
| GenInfo Identifier or Accession.version number | gi\|4826856 |
| Sequence coverage | 3% |

| Band 3 | |
|---|---|
| [NCBInr (all entries)] significant when over score 47 | |
| (4) Protein Name | S-adenosylhomocysteine hydrolase-like 1 |
| Source (organism) | human |
| Total score | 100 |
| Peptides matched | 3 |
| GenInfo Identifier or Accession.version number | gi\|21361647 |
| Sequence coverage | 11% |

| (5) Protein Name | chaperonin GroEL |
|---|---|
| Source (organism) | Escherichia coli 0157 |
| Total score | 91 |
| Peptides matched | 3 |
| GenInfo Identifier or Accession.version number | gi\|15834378 |
| Sequence coverage | 11% |

| (6) Protein Name | programmed cell death 8 isoform 1 |
|---|---|
| Source (organism) | human |
| Total score | 78 |
| Peptides matched : | 3 |
| GenInfo Identifier or Accession.version number | gi\|4757732 |
| Sequence coverage | 5% |

| (8) Protein Name | 60 kDa heat shock protein, mitochondrial precursor |
|---|---|
| Source (organism) | human |
| Total score | 66 |
| Peptides matched | 1 |
| GenInfo Identifier or Accession.version number | gi\|129379 |
| Sequence coverage | 3% |

| (9) Protein Name | ATP synthase, H⁺ transporting, mitochondrial F1 complex, alpha subunit, isoform 1, cardiac muscle |
|---|---|
| Source (organism) | human |
| Total score | 61 |
| Peptides matched | 1 |
| GenInfo Identifier or Accession.version number | gi\|4757810 |
| Sequence coverage | 2% |

| (10) Protein Name | ribophorin II precursor |
|---|---|
| Source (organism) | human |
| Total score | 55 |
| Peptides matched | 3 |
| GenInfo Identifier or Accession.version number | gi\|88567 |
| Sequence coverage | 10% |

| Band 5 | |
|---|---|
| [NCBInr (all entries)] significant when over score 47 | |
| (1) Protein Name | farnesyl-diphosphate farnesyltransferase 1 |
| Source | human |
| Total score | 345 |
| Peptides matched | 6 |
| GenInfo Identifier or Accession.version number | gi\|4758350 |
| Sequence coverage | 18% |

| (2) Protein Name | Ubiquinol-cytochrome C reductase complex core protein 2, mitochondrial precursor |
|---|---|
| Source | human |
| Total score | 222 |
| Peptides matched | 6 |
| GenInfo Identifier or Accession.version number | gi\|21903482 |
| Sequence coverage | 17% |

| (3) Protein Name | dolichyl-diphosphooligosaccharide-protein glycosyltransferase |
|---|---|
| Source | human |
| Total score | 158 |
| Peptides matched | 4 |
| GenInfo Identifier or Accession.version number | gi\|21104416 |
| Sequence coverage | 19% |

| (4) Protein Name | calcium-binding transporter |
|---|---|
| Source | human |
| Total score | 83 |
| Peptides matched | 2 |
| GenInfo Identifier or Accession.version number | gi\|6841066 |
| Sequence coverage | 6% |

| (5) Protein Name | NADH dehydrogenase-ubiquinone Fe-S protein 2 precursor |
|---|---|
| Source | human |
| Total score | 71 |
| Peptides matched | 2 |
| GenInfo Identifier or Accession.version number | gi\|3540239 |
| Sequence coverage | 8% |

| (6) Protein Name | actin, beta |
|---|---|
| Source | human |
| Total score | 57 |
| Peptides matched | 3 |
| Genlnfo Identifier or Accession.version number | gi\|14250401 |
| Sequence coverage | 18% |

| (10) Protein Name | translation elongation factor EF-Tu-like protein P43 precursor, mitochondrial |
|---|---|
| Source | human |
| Total score | 47 |
| Peptides matched | 1 |
| GenInfo Identifier or Accession.version number | gi\|7443384 |
| Sequence coverage | 3% |

| Band 6 | |
|---|---|
| [NCBInr (all entries)] significant when over score 47 | |
| (1) Protein Name | actin, beta |
| Source (organism) | human |
| Total score | 306 |
| Peptides matched | 12 |
| GenInfo Identifier or Accession.version number | gi\|14250401 |
| Sequence coverage | 45% |

| (4) Protein Name | galectin 9, short isoform |
|---|---|
| Source (organism) | human |
| Total score | 245 |
| Peptides matched | 4 |
| GenInfo Identifier or Accession.version number | gi\|4504987 |
| Sequence coverage | 15% |

| (16) Protein Name | GSTmFra2/2-327 |
|---|---|
| Source (organism) | Expression vector pGH/F2.2-327 |
| Total score | 139 |
| Peptides matched | 4 |
| GenInfo Identifier or Accession.version number | gi\|3002516 |
| Sequence coverage | 13% |

| (24) Protein Name | ATP synthase, H⁺ transporting, mitochondrial F1 complex, alpha subunit, isoform 1, cardiac muscle |
|---|---|
| Source (organism) | human |
| Total score | 103 |
| Peptides matched | 1 |
| GenInfo Identifier or Accession.version number | gi\|4757810 |
| Sequence coverage | 2% |

| Band 7 | |
|---|---|
| [NCBInr (all entries)] significant when over score 47 | |
| (1) Protein Name | metaxin 1 |
| Source | human |
| Total score | 63 |
| Peptides matched | 1 |
| GenInfo Identifier or Accession.version number | gi\|4505281 |
| Sequence coverage | 4% |

| (2) Protein Name | sideroflexin 1 |
|---|---|
| Source | human |
| Total score | 62 |
| Peptides matched | 2 |
| GenInfo Identifier or Accession.version number | gi\|23618867 |
| Sequence coverage | 9% |

| (3) Protein Name | TCR beta chain |
|---|---|
| Source | human |
| Total score | 60 |
| Peptides matched | 1 |
| GenInfo Identifier or Accession.version number | gi\|2982508 |
| Sequence coverage | 5% |

| (4) Protein Name | Hnrnp A1 |
|---|---|
| Source | human |
| Total score | 55 |
| Peptides matched | 1 |
| GenInfo Identifier or Accession.version number | gi\|2194069 |
| Sequence coverage | 8% |

| Band 8 | |
|---|---|
| [NCBInr (all entries)] significant when over score 47 | |
| (1) Protein Name | phosphate carrier precursor isoform 1b |
| Source | human |
| Total score | 222 |
| Peptides matched | 8 |
| GenInfo Identifier or Accession.version number | gi\|4505775 |
| Sequence coverage | 31% |

| (2) Protein Name | sideroflexin 1 |
|---|---|
| Source | human |
| Total score | 204 |
| Peptides matched | 5 |
| GenInfo Identifier or Accession.version number | gi\|23618867 |
| Sequence coverage | 24% |

| (3) Protein Name | ATP synthase, H⁺ transporting, mitochondrial F1 complex, gamma polypeptide 1 |
|---|---|
| Source | human |
| Total score | 112 |
| Peptides matched | 2 |
| GenInfo Identifier or Accession.version number | gi\|4885079 |
| Sequence coverage | 8% |

| (4) Protein Name | voltage-dependent anion channel 1 |
|---|---|
| Source | human |
| Total score | 65 |
| Peptides matched | 2 |
| GenInfo Identifier or Accession.version number | gi\|4507879 |
| Sequence coverage | 10% |

| (5) Protein Name | hyaluronan-binding protein precursor |
|---|---|
| Source | human |
| Total score | 62 |
| Peptides matched | 2 |
| GenInfo Identifier or Accession.version number | gi\|8699626 |
| Sequence coverage | 19% |

| (6) Protein Name | androgen-regulated short-chain dehydrogenase/reductase 1 |
|---|---|
| Source | human |
| Total score | 58 |
| Peptides matched | 1 |
| GenInfo Identifier or Accession.version number | gi\|20070798 |
| Sequence coverage | 4% |

| (7) Protein Name | solute carrier family 25 (mitochondrial carrier; oxoglutarate carrier), member 11 |
|---|---|
| Source | human |
| Total score | 55 |
| Peptides matched | 3 |
| GenInfo Identifier or Accession.version number | gi\|21361114 |
| Sequence coverage | 11% |

| (8) Protein Name | 3-hydroxybutyrate dehydrogenase precursor |
|---|---|
| Source | human |
| Total score | 52 |
| Peptides matched | 1 |
| GenInfo Identifier or Accession.version number | gi\|17738292 |
| Sequence coverage | 4% |

| (9) Protein Name | B-cell receptor associated protein |
|---|---|
| Source | human |
| Total score | 49 |
| Peptides matched | 2 |
| GenInfo Identifier or Accession.version number | gi\|1673514 |
| Sequence coverage | 8% |

| Band 9 | |
|---|---|
| [NCBInr (all entries)] significant when over score 47 | |
| (1) Protein Name | ATP synthase, H⁺ transporting, mitochondrial F1 complex, O subunit |
| Source | human |
| Total score | 255 |
| Peptides matched | 7 |
| GenInfo Identifier or Accession.version number | gi\|4502303 |
| Sequence coverage | 39% |

| (2) Protein Name | ATP synthase, H⁺ transporting, mitochondrial F0 complex, subunit d |
|---|---|
| Source | human |
| Total score | 217 |
| Peptides matched | 8 |
| GenInfo Identifier or Accession.version number | gi\|5453559 |
| Sequence coverage | 55% |

| (3) Protein Name | ATP synthase, H⁺ transporting, mitochondrial F0 complex, subunit b, isoform 1 |
|---|---|
| Source | human |
| Total score | 139 |
| Peptides matched | 4 |
| GenInfo Identifier or Accession.version number | gi\|21361565 |
| Sequence coverage | 24% |

| (4) Protein Name | small GTP-binding protein |
|---|---|
| Source | human |
| Total score | 111 |
| Peptides matched | 2 |
| GenInfo Identifier or Accession.version number | gi\|13569962 |
| Sequence coverage | 15% |

| (6) Protein Name | NADH dehydrogenase (ubiquinone) Fe-S protein 8, 23kDa (NADH-coenzyme Q reductase) |
|---|---|
| Source | human |
| Total score | 79 |
| Peptides matched | 2 |
| GenInfo Identifier or Accession.version number | gi\|4505371 |
| Sequence coverage | 28% |

| (7) Protein Name | vesicle trafficking protein sec22b |
|---|---|
| Source | human |
| Total score | 76 |
| Peptides matched | 1 |
| GenInfo Identifier or Accession.version number | gi\|4759086 |
| Sequence coverage | 6% |

| Band 11 | |
|---|---|
| [NCBInr (all entries)] significant when over score 46 | |
| (1) Protein Name | mitochondrial import receptor Tom22 |
| Source | human |
| Total score | 223 |
| Peptides matched | 7 |
| GenInfo Identifier or Accession.version number | gi\|9910382 |
| Sequence coverage | 51% |

| (2) Protein Name | signal sequence receptor, delta |
|---|---|
| Source | human |
| Total score | 159 |
| Peptides matched | 3 |
| GenInfo Identifier or Accession.version number | gi\|5454090 |
| Sequence coverage | 19% |

| Internal Sequence Analysis Results | | |
|---|---|---|
| Bands | | |
| A+B | Protein Name | ATP synthase, alpha chain |
| | Peptides matched | 5 |
| | GenInfo Identifier | gi\|114517 or P25705 |
| | Sequence coverage | 10.13 % |
| C | Protein Name | ATP synthase, beta chain |
| | Peptides matched | 4 |
| | GenInfo Identifier | gi\|114549 or P06576 |
| | Sequence coverage | 8.13 % |
| | Protein Name : | Sodium/potassium-transporting ATPase |
| | | beta-3 chain |
| | Peptides matched | 1 |
| | GenInfo Identifier | gi\|1703470 or P54709 |
| | Sequence coverage | 3.23 % |
| D | Protein Name : | ADP, ATP carrier protein |
| | Peptides matched | 4 |
| | GenInfo Identifier | gi\|113463 or P12236 |
| | | gi\|113459 or P05141 |
| | | gi\|113455 or P12235 |
| | Sequence coverage | 14.43 % |
| 4 | Protein Name : | ubiquinol-cytochrome C reductase complex core protein 1 |
| | Peptides matched | 1 |
| | GenInfo Identifier | gi\|731047 or P31930 |
| | Sequence coverage | 2.29 % |
| 10 | Protein Name : | Cytochrome c oxidase polypeptide II |
| | | Peptides matched 1 |
| | GenInfo Identifier | gi\|117020\| or P00403 |
| | Sequence coverage | 2.64 % |

The galectin-9-binding molecule candidates identified in the above are further analyzed for their interaction with galectin-9 by the methods listed herein below in order to identify a most suitable galectin-9-binding molecule. Furthermore, the functions of the molecule can be examined and characterized.

### (1) Immunoprecipitation

Galectin-9 is added to cells for interaction, and then the cells are treated for cytolysis to give cell extracts. When a substance that binds to galectin-9 is present in the cells, the substance is present in the cell extracts in the bound state with galectin-9. The resulting cell extract is reacted with anti-galectin-9 Ab labeled with gel or the like to bind to the anti-galectin-9 Ab. Alternately, anti-galectin-9 Ab is conjugated with protein A, and galectin-9 is precipitated together with the anti-galectin-9 Ab by centrifugation. When a protein that binds to galectin-9 is present, the protein is precipitated together with the antibody; thus, the protein can be identified. The precipitate can be detected by western blotting after washing if necessary, denaturing in some cases, and applying to, for example, an SDS-polyacrylamide gel (SDS-PAGE) and transferring to a membrane if necessary.

### (2) West-Western blotting or far-Western method (including ligand blotting analysis)

1. A cell protein extract solution is subjected to electrophoresis, followed by transfer of proteins to a membrane (PVDF, etc.).
2. Label galectin-9, followed by specific binding of the labeled Gal-9 to the proteins on the membrane.
3. Detect the label. Thus, the distribution of proteins that bind to galectin-9 can be disclosed (use of cells derived from a certain tissue will lead to clarification as to which tissue contains the binding protein). The localization (use of fractions from cytoplasm, cell membranes, or the like) and molecular weight are also clarified.

The labeling of galectin-9 can be performed by known methods without any limitation. Examples of the labeling are the following methods:
A. Detect RI such as ¹²⁵I and ³H.
B. Detect a biotin label with streptavidin-conjugated Ab.
C. Detect excitation light or color development of a labeled fluorescence substance.
D. Detect phosphorylated galectin-9 with anti-phosphate Ab.
E. Detect Tag (Flag, His, GST, and so on) fused galectin-9 with anti-Tag Ab.

### (3) Intermolecular cross-linking

1. Reinforcement of the binding between galectin-9 and target proteins by adding chemical cross-linking agents after the addition of galectin-9 to culture cells.
2. Cytolysis of the cells and isolation of galectin-9-binding proteins from the resulting protein extract solution by SDS-PAGE.
3. Detect the target proteins by Western blotting, immunoprecipitation, or the like by using anti-galectin-9 Ab.

### (4) Expression cloning analysis

1. Expression cloning analysis in E. coli
   a. Express target candidate proteins in E. coli.
   b. Prepare a protein extract solution by cytolysis of the in transformed E. coli cells which express the candidate proteins.
   c. Electrophoresis of the E. coli cell extract solution and detection by West-Western blotting or by using anti-galectin-9 Ab. The labeling for the detection can be used as in the above-mentioned West-Western method.
2. Expression cloning analysis in E. coli and phages
   a. E. coli is infected with phages (phage libraries) that express certain proteins.
   b. Transfer the recombinant phage to a nitrocellulose membrane and express the proteins.
   c. Labeled galectin-9 is applied to the nitrocellulose membrane. Identify the recombinant phage that expresses the label-bound protein.
   d. Analysis of galectin-9-binding protein cDNA from the recombinant phage.
3. Expression cloning analysis in culture cells
   a. Transduction of each cDNA of candidate proteins to culture cells for expression of the candidate proteins.
   b. Incubation of the culture cells after the addition of galectin-9, and then washing of the cells for removing non-specifically binding galectin-9.
   c. Incubate the culture cells after addition of anti-galectin-9 Ab labeled with a fluorescent material or chromophoric agent, and detect the galectin-9 bound to the expressed protein by ELISA or FACS (methods for reading the fluorescent and chromophoric materials and readers or devices used for the method).

### (5) Two-hybrid system

In this system, protein-protein interactions are detected in yeast cells wherein the transcription activity of a reporter gene is used as an indicator. This system is characterized in that the protein-protein interaction can be detected in yeast cells (in vivo), and weak transient interactions can be also detected with their high sensitivity. Furthermore, it is advantageous that it is unnecessary to purify proteins and to employ antibodies at the detection stage for the interaction.

The procedures are as follows:
1. Two hybrid fusion proteins, , i.e. one hybrid fusion protein containing a galectin-9 (bait) gene fused to a DNA-binding domain (DNA-BD) and another hybrid fusion protein wherein a gene encoding a candidate protein (prey) which potentially interacts with the bait gene is fused to a gene for a transcription-activating domain (DNA-AD), are expressed in yeast cells.
2. A reporter gene for detecting the transcriptional activity is incorporated into the chromosome of yeast. Various reporter genes which have been developed can be broadly used. They may be used without any limitation. The reporter gene usually used herein includes auxotrophic reporter genes and lacZ genes.
3. The fused transcription-activator gene functions only when the galectin-9 (bait) interacts with the candidate protein (prey) each other in yeast cells, and the transcription at the upstream of the reporter gene is activated to express the target gene.
4. In actual embodiments, the interaction is detected by measuring the restoration of auxotrophy on a medium plate, or β-galactosidase activity. Namely, the medium is prepared so that the yeast cells proliferate only when the interaction occurs or that color development occurs by β-galactosidase; thus, the interaction is detected.

The two-hybrid system using mammalian cells instead of yeast cells can be also used for detecting the protein-protein interaction, without limitations.

### (6) Phage display technique

Phage display is a tool useful in identifying proteins or peptides that interact with other molecules.

The procedures are as follows:
1. DNAs are inserted into bacteriophage genomes so that candidate proteins are displayed on the phage particle surfaces as proteins fused with phage capsid proteins (surface proteins envelopting the phage DNA).
2. The phages are applied to a reaction with galectin-9 coated on the surface of a plate. Non-specifically binding recombinant phages (phages that express proteins) are washed out.
3. Phages that express candidate proteins specifically interact with galectin-9 are selected from the recombinant phages and are concentrated by repeating the above step.
4. Since the cDNAs encoding the expressed proteins can be prepared from the selected phages, both the genes and proteins can be identified.
5. Furthermore, by performing ELISA using the selected phages which interact with a 96-well plate coated with galectin-9, phages that express proteins capable of more strongly binding to galectin-9 can be selected based on the coloring intensity.

### (7) Surface plasmon resonance (technology using BIAcore)

Biacore detection systems utilize the optical phenomenon of Surface Plasmon Resonance (SPR). A micro-change in mass, which is generated on a surface of a sensor chip depending on the binding and dissociation between two molecules, is detected as a SPR signal. The principle of this method is as follows: a sensor chip (gold thin film) is irradiated with light so that the light is totally reflected at a surface where biomolecules are not immobilized. Reduction of the reflected light intensity (generation of SPR signal) is partially observed. The angle (change of refractive index) of the dark light depends on mass on the sensor chip.

Candidate proteins (analytes) are applied to the sensor chip surface on which galectin-9 (ligand) is immobilized. When the binding reaction occurs, a change in mass (an increase of mass) is generated to shift the dark light portion from I to II. It is known that a change of 1 ng/mm² generated by the binding of a material causes a shift of 0.1 degree from I to II. Reversely, when mass is decreased by dissociation, the portion returns from II to I by the degree of the change.

In biacore systems, the amount of shift from I to II, i.e. the amount of change in mass on the sensor chip surface, is displayed on a vertical axis, and a mass change per time is shown as measured data (sensor-gram). The unit of the vertical axis is Resonance Unit (RU), and 1 RU is equivalent to 1 pg/mm² (1 RU = 1 pg/mm²).

It is not required to label the biomolecules, and the interaction can be observed in real time.

The procedures are as follows:
1. Galectin-9 (ligand) is immobilized on a sensor chip.
2. A solution containing candidate proteins (analytes) which potentially bind to galectin-9 is applied on the sensor chip at a constant flow rate.
3. When the binding or dissociation occurs between galectin-9 and the candidate protein, the signal is detected as surface plasmon resonance.
4. Since the binding intensity can be quantitatively measured by the signal intensity, the protein having a strong interaction with galectin-9 can be identified.

### (8) Fluorescence polarization

In fluorescence polarization techniques, saccharide chain-protein, antigen-antibody, or protein-protein interactions in a solution can be directly analyzed without processes of immobilization and separation of tracer molecules.

The principle of the fluorescence polarization is as follows: When fluorescence-conjugated galectin-9 molecules in a solution are excited with plane polarized light, the polarized fluorescent light is emitted in the same plane. However, if the molecule rotates by Brownian motion during the excited state, the fluorescent light is emitted in a different plane from the excitation plane and is depolarized. Namely, the fluorescence polarization indicates the degree of rotation of the fluorescent molecule from the excitation to the emission of light. Generally, since a small molecule labeled with a fluorescent probe very quickly rotates by Brownian motion in a solution, the polarization is low. On the other hand, when a protein molecule is bound to the probe, Brownian motion in the solution decreases and the polarization increases. Therefore, the protein-protein interaction in a solution can be analyzed by the fluorescence polarization using a change of the polarization as an indicator.

The procedures are as follows:
1. Fluorescence polarization of fluorescence-conjugated galectin-9 is measured.
2. A solution containing other proteins is added, and it is detected whether the fluorescence polarization of fluorescence-conjugated galectin-9 is changed or not.
3. When a change in the polarization is large, the candidate protein is bound to the galectin-9. Therefore, the interacted protein is identified.

The galectin-9-binding molecules as identified herein above can be used for screening of a material that controls galectin-9 according to the technology disclosed herein.

### INDUSTRIAL APPLICABILITY

In accordance with the present invention, human galectin-9 has a cytotoxic activity or apoptosis-inducing activity against tumor cells, but does not show the cytotoxic activity against normal cells and further does not induce apoptosis in normal cells. Therefore, according to the present invention, it is possible to develop a technology utilizing galectin-9 proteins, galectin-9 agonists, galectin-9-antagonist antagonists, anti-galectin-9-binding protein antibodies, anti-galectin-9-binding saccharide chain antibodies, and galectin-9 production/release-inducing substances as antineoplastic agents (anti-tumor agents) that induce apoptosis in tumor cells and are cytotoxic to tumor cells, but do not affect normal cells.

Furthermore, human galectin-9 does not induce apoptosis in resting T cells, in particular, in resting CD4-positive T cells (helper T cells), but remarkably induces apoptosis in activated CD4-positive T cells (helper T cells) and induce apoptosis in activated CD8-positive T cells (suppressor T cells and cytotoxic T cells) higher than galectin-1 or galectin-3 does. It is assumed that galectin-9 induces apoptosis through a pathway similar to that in glucocorticoid (dexamethasone), i.e. from a receptor → calcium influx → calpain → caspase-1 → caspase-3 and caspase-7, so galectin-9 can be used as an anti-inflammatory agent, anti-allergy agent, and immunosuppressive agent. Galectin-9 proteins, galectin-9 gene transfers, or induction of galectin-9 can be applied as immunosuppressive agents with low side effects. Furthermore, the co-applications of galectin-9 in combination with glucocorticoids can reduce the dose levels of glucocorticoids, resulting in a decrease of side effects. Thus, by utilizing galectin-9 and its analogues, and controlling the concentration of galectin-9 in vivo or its expression, antineoplastic agents, anti-allergy agents, immunosuppressive agents, anti-autoimmune disease agents, anti-inflammatory agents, and active substitutes for adrenocortical steroid hormones can be provided. Furthermore, the use of galectin-9 can be applied to a field in which a pharmacological effect and a biological activity of glucocorticoid are utilized.

While the present invention has been described specifically in detail with reference to certain embodiments and examples thereof, it would be apparent that it is possible to practice it in other forms. In light of the disclosure, it will be understood that various modifications and variations are within the spirit and scope of the appended claims.

## Claims

1. A pharmaceutical or veterinary drug which comprises
(i) an effective amount of at least one or more members selected from the group consisting of
(a) galectin 9 and analogs thereof;
(b) polynucleotides each coding for galectin 9 or a polypeptide having a biological activity substantially equivalent to that owned by galectin 9;
(c) inducing factors for production and/or release of galectin 9;
(d) anti-galectin 9-receptor antibodies; and
(e) antibodies against a galectin 9-binding saccharide,
(ii) wherein said drug is selected from the group consisting of anti-tumor (antineoplastic) agents, anti-allergic agents, immunosuppressants, drugs for auto-immune diseases, anti-inflammatory agents, and active components for adrenocortical steroid hormone alternatives.

2. An antineoplastic agent comprising an effective amount of at least one or more members selected from the group consisting of galectin 9 and peptide analogs thereof.

3. An anti-allergic agent comprising an effective amount of at least one or more members selected from the group consisting of galectin 9 and peptide analogs thereof.

4. An immunosuppressant comprising an effective amount of at least one or more members selected from the group consisting of galectin 9 and peptide analogs thereof.

5. A drug for auto-immune diseases, comprising an effective amount of at least one or more members selected from the group consisting of galectin 9 and peptide analogs thereof.

6. An anti-inflammatory agent comprising an effective amount of at least one or more members selected from the group consisting of galectin 9 and peptide analogs thereof.

7. An adrenocortical steroid hormone alternative comprising an effective amount of at least one or more members selected from the group consisting of galectin 9 and peptide analogs thereof.

8. A tumor cytotoxic therapeutic agent for malignant cells which comprises an effective amount of at least one or more members selected from the group consisting of (a) galectin 9 and Gal-9 analogs, and (b) polynucleotides each coding for galectin 9 or a polypeptide having a biological activity substantially equivalent to that owned by galectin9.

9. An antimetastatic agent for cancer cells, which comprises an effective amount of at least one or more members selected from the group consisting of (a) galectin 9 and Gal-9 analogs, and (b) polynucleotides each coding for galectin 9 or a polypeptide having a biological activity substantially equivalent to that owned by galectin9.

10. A cytotoxic drug for tumor cells which comprises an effective amount of at least one or more members selected from the group consisting of
(a) galectin 9 and analogs thereof;
(b) polynucleotides each coding for galectin 9 or a polypeptide having a biological activity substantially equivalent to that owned by galectin 9;
(c) inducing factors for production and/or release of galectin 9;
(d) anti-galectin 9-receptor antibodies; and
(e) antibodies against a galectin 9-binding saccharide.

11. An apoptosis-inducing drug for tumor cells, which comprises an effective amount of at least one or more members selected from the group consisting of
(a) galectin 9 and analogs thereof;
(b) polynucleotides each coding for galectin 9 or a polypeptide having a biological activity substantially equivalent to that owned by galectin 9;
(c) inducing factors for production and/or release of galectin 9;
(d) anti-galectin 9-receptor antibodies; and
(e) antibodies against a galectin 9-binding saccharide.

12. An apoptosis-inducing drug for immune cells, including especially activated T cells, which comprises an effective amount of at least one or more members selected from the group consisting of
(a) galectin 9 and analogs thereof;
(b) polynucleotides each coding for galectin 9 or a polypeptide having a biological activity substantially equivalent to that owned by galectin 9;
(c) inducing factors for production and/or release of galectin 9;
(d) anti-galectin 9-receptor antibodies; and
(e) antibodies against a galectin 9-binding saccharide.

13. A prophylactic and/or therapeutic agent for diseases or pathological conditions, caused by activated T cells, which comprises an effective amount of at least one or more members selected from the group consisting of
(a) galectin 9 and analogs thereof;
(b) polynucleotides each coding for galectin 9 or a polypeptide having a biological activity substantially equivalent to that owned by galectin 9;
(c) inducing factors for production and/or release of galectin 9;
(d) anti-galectin 9-receptor antibodies; and
(e) antibodies against a galectin 9-binding saccharide.

14. A galectin 9-binding factor which is selected from the group consisting of
4F2 heavy chain antigen (177216);
ATPase, Na⁺/K⁺ transporting, alpha 1 polypeptide (21361181);
sodium-dependent neutral amino acid transporter type 2 truncated isoform (15004317);
stromal cell derived factor receptor 1 isoform a (9257240);
stromal cell derived factor receptor 1 isoform b;
heat shock 90kDa protein 1, beta (20149594);
heat shock 90kDa protein 1, alpha; heat shock 70kDa protein 5 (glucose-regulated protein, 78kDa) (16507237);
heat shock 70kDa protein 8 isoform 2 (24234686);
heat shock 70kDa protein 9B precursor (24234688);
fatty-acid-Coenzyme A ligase, long-chain 3 (27469830);
NADH dehydrogenase (ubiquinone) Fe-S protein 1, 75kDa (NADH-coenzyme Q reductase) (4826856);
S-adenosylhomocysteine hydrolase-like 1 (21361647); programmed cell death 8 isoform 1 (4757732);
60 kDa heat shock protein, mitochondrial precursor (129379);
ATP synthase, H⁺ transporting, mitochondrial F1 complex, alpha subunit, isoform 1, cardiac muscle (4757810);
ribophorin II precursor (88567);
farnesyl-diphosphate farnesyltransferase 1 (4758350);
Ubiquinol-cytochrome C reductase complex core protein 2, mitochondrial precursor (21903482);
dolichyl-diphosphooligosaccharide-protein glycosyltransferase (21104416);
calcium-binding transporter (6841066);
NADH dehydrogenase-ubiquinone Fe-S protein 2 precursor (3540239);
actin, beta (14250401); translation elongation factor EF-Tu-like protein P43 precursor, mitochondrial (7443384);
metaxin 1 (4505281);
sideroflexin 1 (23618867);
TCR beta chain (2982508);
Hnrnp A1 (2194069);
phosphate carrier precursor isoform 1b (4505775);
ATP synthase, H⁺ transporting, mitochondrial F1 complex, gamma polypeptide 1 (4885079);
voltage-dependent anion channel 1 (4507879);
hyaluronan-binding protein precursor (8699626);
androgen-regulated short-chain dehydrogenase/reductase 1 (20070798);
solute carrier family 25 (mitochondrial carrier;
oxoglutarate carrier), member 11 (21361114);
3-hydroxybutyrate dehydrogenase precursor (17738292);
B-cell receptor associated protein (1673514);
ATP synthase, H⁺ transporting, mitochondrial F1 complex, O subunit (4502303);
ATP synthase, H⁺ transporting, mitochondrial F0 complex, subunit d (5453559);
ATP synthase, H⁺ transporting, mitochondrial F0 complex, subunit b, isoform 1 (21361565);
small GTP-binding protein (13569962);
NADH dehydrogenase (ubiquinone) Fe-S protein 8, 23kDa (NADH-coenzyme Q reductase) (4505371);
vesicle trafficking protein sec22b (4759086);
mitochondrial import receptor Tom22 (9910382);
signal sequence receptor, delta (5454090);
ATP synthase, alpha chain (114517 or P25705);
ATP synthase, beta chain (114549 or P06576);
Sodium/potassium-transporting ATPase beta-3 chain (1703470 or P54709);
ADP, ATP carrier protein (113463, P12236, 113459, P05141, 113455 or P12235);
ubiquinol-cytochrome C reductase complex core protein 1 (731047 or P31930); and
Cytochrome c oxidase polypeptide II (117020 or P00403)
wherein each number in parentheses indicates a "GenInfo Identifier" sequence identification number assigned to each specific protein in databases where protein information and/or nucleotide sequence information (including data of DNA coding for the protein) is acquired by the entry of said number at the NCBI internet home page (http://www.ncbi.nlm. nih.gov/).

15. A technique for controlling the activity of galectin 9 which comprises utilizing any of interactions between the galectin 9-binding factor according to Claim 14 and galectin 9.
